(19) Europäisches Patentamt
European Patent Office
Office européen des brevets

(11) Publication number: **0 152 021**
**A2**

(12) **EUROPEAN PATENT APPLICATION**

(21) Application number: 85101003.3

(22) Date of filing: 31.01.85

(51) Int. Cl.[4]: C 07 D 209/48
C 07 D 413/04, A 01 N 43/38
A 01 N 43/82

(30) Priority: 31.01.84 JP 15854/84

(43) Date of publication of application:
21.08.85 Bulletin 85/34

(84) Designated Contracting States:
AT BE CH DE FR GB IT LI NL

(71) Applicant: SANKYO COMPANY LIMITED
No. 1-6, 3-chome Nihonbashi Honcho Chuo-ku
Tokyo(JP)

(72) Inventor: Aoki, Atsushi c/o Hokkai Sankyo Co., Ltd
2-1, 8-chome, 7-jo Toyohira
Toyohira-ku Sapporo-shi Hokkaido(JP)

(72) Inventor: Endo, Toshimitsu c/o Hokkai Sankyo Co., Ltd
2-1, 8-chome, 7-jo Toyohira
Toyohira-ku Sapporo-shi Hokkaido(JP)

(72) Inventor: Ueda, Takashi c/o Agricultural Chemicals Research
Sankyo Co., Ltd. 2-58, 1-chome
Hiromachi Shinagawa-ku Tokyo(JP)

(72) Inventor: Nakanishi, Toshiro c/o Agricultural Chemicals
Sankyo Co., Ltd. 1041, Yasu
Yasu-cho Yasu-gun Shiga-ken(JP)

(74) Representative: Zumstein, Fritz, Dr. et al,
Dr. F. Zumstein sen Dr. E. Assman Dipl.-Ing. F. Klingseisen Dr. F. Zumstein jun. Bräuhausstrasse 4
D-8000 München 2(DE)

(54) **Phthalimide derivatives, their preparation and their use as agricultural fungicides.**

(57) Compounds of formula (I):

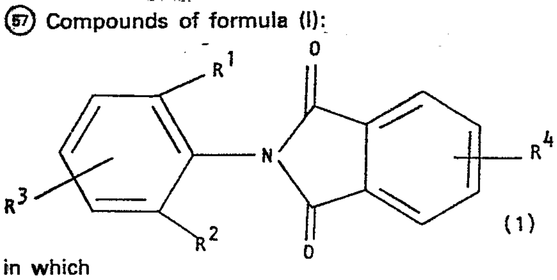

(1)

in which

$R^1$ and $R^2$ are the same or different and each represents a $C_1$-$C_6$ alkyl group or a $C_2$-$C_6$ alkenyl group;

$R^3$ represents hydrogen, a halogen atom or a $C_1$-$C_6$ alkyl group; and

$R^4$ represents a carboxy group; a thiocarboxy group; a cyano group; a nitro group; an amino group; an amino group having 1 or 2 aliphatic carboxylic acyl substituents; a heterocyclic group; an aliphatic carboxylic acyl group; a $C_2$-$C_4$ alkenyl group; or a $C_1$-$C_4$ alkyl or $C_2$-$C_4$ alkenyl group having 1 or 2 substituents selected from the group consisting of groups of formula $-XR^5$ (wherein X represents the oxygen or sulfur atom or the $-NH-$ group, and $R^5$ represents hydrogen, a $C_3$-$C_8$ cycloalkyl group, a $C_2$-$C_4$ alkenyl group, a $C_2$-$C_4$ alkynyl group, an aryl group, a carboxy group, an acyl group, a $C_1$-$C_6$ alkyl group, or a $C_1$-$C_6$ alkyl group having at least one substituent selected from the group consisting of cyano, nitro, $C_1$-$C_4$ alkoxy, aryl, aryloxy and $C_1$-$C_4$ alkylamino substituents), halogen atoms, heterocyclic groups, carboxy groups, thiocarboxy groups, cyano groups, aliphatic carboxylic acyl groups, aromatic carboxylic acyl groups, nitro groups, di($C_1$-$C_3$ alkoxy)phosphoryl groups and hydroxyimino groups; and, where $R^4$ represents a carboxy or thiocarboxy group or a group including said carboxy or thiocarboxy group, the corresponding acid halides, esters, amides and salts thereof;

and, where $R^4$ represents a group including a hydroxy or mercapto group, esters thereof;

and, where $R^4$ represents or includes a substituted or unsubstituted amino group, acid addition salts thereof are useful agricultural fungicides, being especially effective against club root.

EP 0 152 021 A2

# PHTHALIMIDE DERIVATIVES, THEIR PREPARATION AND THEIR USE AS AGRICULTURAL FUNGICIDES

The present invention relates to a series of new phthalimide derivatives which are useful as fungicides, particularly for agricultural purposes.

Agricultural fungicides must meet a number of very specific requirements. They are normally applied under conditions where precise control of the amounts administered is difficult; they must not damage the plants which they are intended to protect, nor must they cause damage to other parts of the environment; where they are used to protect food or fodder crops, they must either not persist in the plant or be innocuous towards humans and other animals or, preferably, both; and finally they must be effective against at least some pathogenic fungi. To compound the problem, resistance to fungicides can develop quite readily and there is, therefore, a continuing need for new fungicides.

Certain phthalimide derivatives similar to those of the present invention have been disclosed for use as

agricultural fungicides in Japanese Patent Applications Kokai No. 25736/75, No. 54924/76 and No. 125625/77. These derivatives may be represented by the formula:

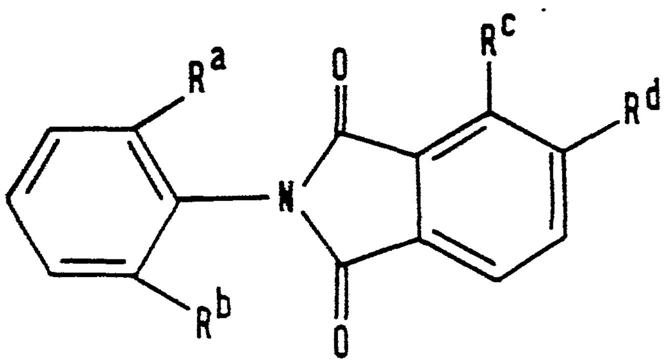

In the above formula:

Japanese Application Kokai No. 25736/75 discloses compounds in which $R^a$ and $R^b$ each represents an ethyl or isopropyl group, whilst $R^c$ and $R^d$ both represent hydrogen atoms;

Japanese Application Kokai No. 54924/76 discloses compounds in which $R^a$ and $R^b$ represent ethyl or isopropyl groups and one of $R^c$ and $R^d$ represents hydrogen whilst the other represents chlorine or methyl; and

Japanese Application Kokai No. 125625/77 discloses compounds in which $R^a$ and $R^b$ represent ethyl or isopropyl groups and one of $R^c$ and $R^d$ represents

hydrogen whilst the other represents methoxy.

We have now discovered a series of novel compounds related to these known phthalimide derivatives which, whilst showing the excellent activity of these known derivatives against many fungi, also show surprisingly enhanced activity against the pathogenic fungus, Plasmodiophora brassicae, responsible for the disease known as "club root", which attacks plants of the genus Brassica, many of which (such as the various varieties of cabbage, rape, swede, mustard and turnips) are of considerable economic importance.

The compounds of the invention may be represented by the formula (I):

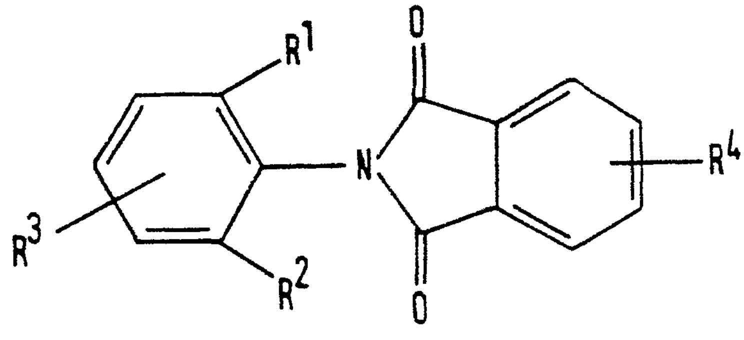

(I)

in which:

$R^1$ and $R^2$ are the same or different and each represents a $C_1$-$C_6$ alkyl group or a $C_2$-$C_6$ alkenyl group;

$R^3$ represents hydrogen, a halogen atom or a $C_1$-$C_6$ alkyl group; and

$R^4$ represents a carboxy group; a thiocarboxy group; a cyano group; a nitro group; an amino group; an amino group having 1 or 2 aliphatic carboxylic acyl substituents; a heterocyclic group; an aliphatic carboxylic acyl group; a $C_2$-$C_4$ alkenyl group; or a $C_1$-$C_4$ alkyl or $C_2$-$C_4$ alkenyl group having 1 or 2 substituents selected from the group consisting of groups of formula $-XR^5$ (wherein X represents the oxygen or sulfur atom or the -NH- group, and $R^5$ represents hydrogen, a $C_3$-$C_8$ cycloalkyl group, a $C_2$-$C_4$ alkenyl group, a $C_2$-$C_4$ alkynyl group, an aryl group, a carboxy group, an acyl group, a $C_1$-$C_6$ alkyl group, or a $C_1$-$C_6$ alkyl group having at least one substituent selected from the group consisting of cyano, nitro, $C_1$-$C_4$ alkoxy, aryl, aryloxy and $C_1$-$C_4$ alkylamino substituents), halogen atoms, heterocyclic groups, carboxy groups, thiocarboxy groups, cyano groups, aliphatic carboxylic acyl groups, aromatic carboxylic acyl groups, nitro groups, di($C_1$-$C_3$ alkoxy)phosphoryl groups and hydroxyimino groups; and, where $R^4$ represents a carboxy or thiocarboxy group or a group including said carboxy or thiocarboxy

group, the corresponding acid halides, esters, amides and salts thereof;

and, where $R^4$ represents a group including a hydroxy or mercapto group, esters thereof;

and, where $R^4$ represents or includes a substituted or unsubstituted amino group, acid addition salts thereof.

The present invention also provides a method of protecting seeds and growing plants from fungal attack, which comprises applying to plant material (which may be seeds, plants, cuttings etc) or to a locus including the same an effective amount of a fungicide, and provides a composition comprising an effective amount of a fungicide in admixture with a carrier, wherein the fungicide is selected from the group consisting of compounds of formula (I) and acid halides, esters, amides and salts thereof.

The invention also provides a process for preparing the compounds of the invention, which process comprises the steps:

(a) reacting an aniline derivative of formula (III):

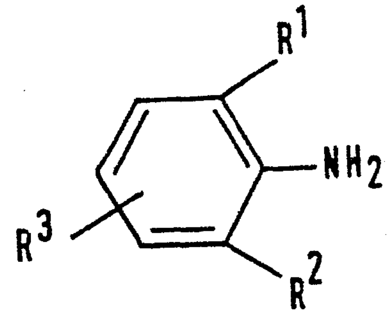

(III)

(wherein $R^1$, $R^2$ and $R^3$ are as defined above) with a phthalic acid derivative of formula (IVa)

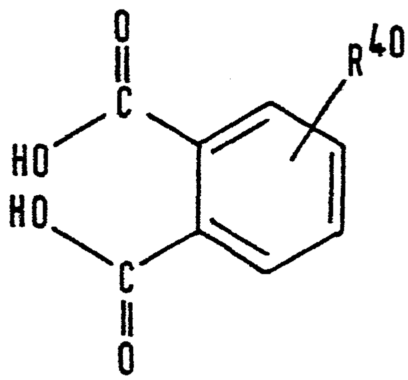

(IVa)

(wherein $R^{40}$ represents any one of the groups defined for $R^4$ or any one of said groups wherein any active group or atom is protected) or with an active derivative of said phthalic acid derivative;

(b) if necessary, removing any protecting group;

(c) if necessary, converting any group represented by $R^4$ to any other group represented by $R^4$; and

(d) if necessary, forming an acid halide, ester, amide or salt of the resulting compound.

The active derivative of said phthalic acid derivative (IVa) is preferably an active ester, active amide, acid halide or anhydride, more preferably the

anhydride of formula (IVb):

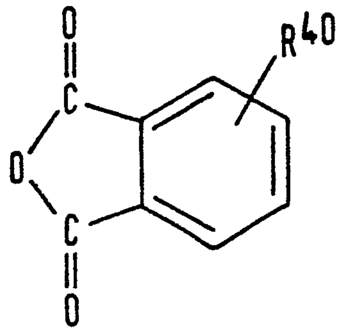

(IVb)

(in which $R^{40}$ is as defined above).

In the case of esters and salts of compounds of formula (I), the acids or alcohols used to form the esters and the acids or bases used to form the salts may be chosen from a wide range of compounds, as is well recognized in the art, provided that the resulting esters or salts are broadly agriculturally acceptable. Within this practical constraint, which is well known and obvious to those skilled in the art, there is no restriction on the nature of such acids, alcohols and

bases.

Where $R^4$ represents a carboxy or thiocarboxy group or an alkyl or alkenyl group having one or two carboxy or thiocarboxy substituents, or $R^5$ represents a carboxy group, the resulting compound of formula (I) will form salts. Examples of such salts include the alkali metal, alkaline earth metal, other innocuous metal, ammonium, quaternary ammonium and organic amine salts. Preferred salts include : alkali metal salts, such as the sodium or potassium salts; alkaline earth metal salts, such as the calcium and magnesium salts; other metal salts, particularly salts of trivalent metals such as aluminum or iron; ammonium salts; quaternary ammonium salts, particularly tetra-($C_1$-$C_6$ alkyl)ammonium salts, such as tetrabutylammonium salts; and organic amine salts, particularly di- or tri-($C_1$-$C_6$ alkyl)amine, especially di- or tri-($C_1$-$C_4$ alkyl)amine, or cyclic amine salts, such as the diethylamine, dipropylamine, trimethylamine, triethylamine, tripropylamine, tributylamine and pyridine salts.

Compounds of formula (I) in which $R^4$ represents a carboxy or thiocarboxy group or an alkyl or alkenyl group having one or two carboxy or thiocarboxy substituents, or $R^5$ represents a carboxy group, being

acids, can also form esters with alcoholic compounds. Apart from the practical constraints outlined above, there is no real limit on the nature of the alcoholic moiety of such esters and a wide range of well known alcoholic moieties may be employed. Such esterified groups represented by $R^4$ may be represented by the formulae $-C(:X')X'R^6$ or $-A[-C(:X')X'R^6]_n$, and those of $R^5$ may be represented by the formula $-COOR^6$, in which: one of X' represents an oxygen atom and the other represents an oxygen or sulfur atom; A represents a $C_1$-$C_5$ alkylene, alkylidene, alkenylene or alkenylidene group or, where n is 1, such a group having one of the substituents specified for alkyl or alkenyl groups of $R^4$; n is 1 or 2; and $R^6$ represents an alkyl group, a substituted alkyl group [wherein the substituents are selected from the group consisting of halogen atoms, cyano groups, hydroxy groups, $C_1$-$C_6$ alkoxy groups, $C_1$-$C_6$ alkylthio groups, phenoxy groups, carboxylic acyloxy groups, epoxy groups, carboxy groups, ($C_1$-$C_6$ alkoxy)carbonyl groups, di($C_1$-$C_6$ alkyl)amino groups and 5- or 6-membered heterocyclic rings containing at least one hetero-atom selected from the group consisting of nitrogen and oxygen atoms], a $C_2$-$C_6$ alkenyl group, a $C_2$-$C_6$ alkynyl group, a $C_3$-$C_8$ cycloalkyl group, a $C_3$-$C_8$ cycloalkyl group having at least one $C_1$-$C_6$ alkyl substituent, an aralkyl group, a substituted aralkyl group (wherein the

substituent is at least one group or atom selected from the group consisting of halogen atoms, $C_1-C_6$ alkyl groups and nitro groups), a phenyl group, a substituted phenyl group [having at least one substituent selected from the group consisting of halogen atoms, nitro groups, $C_1-C_6$ alkyl groups, $C_2-C_6$ alkenyl groups, $C_1-C_6$ alkoxy groups, $C_1-C_6$ alkanoyl groups, ($C_1-C_6$ alkoxy)carbonyl groups, di($C_1-C_6$ alkyl)amino groups, $C_1-C_6$ alkanoylamino groups and cyano groups], a di($C_1-C_6$ alkyl)amino group or a 5- or 6-membered heterocyclic group containing at least one hetero atom selected from the group consisting of nitrogen atoms and oxygen atoms.

Where $R^6$ represents an alkyl group, this may have from 1 to 20 carbon atoms and may be a straight or branched chain group. Examples include the methyl, ethyl, propyl, isopropyl, butyl, isobutyl, sec-butyl, t-butyl, pentyl, hexyl, heptyl, octyl, nonyl, decyl, dodecyl, tetradecyl, pentadecyl and octadecyl groups, of which those alkyl groups having from 1 to 10 carbon atoms are particularly preferred. Where $R^6$ represents a substituted alkyl group, the alkyl group itself may be any one of those mentioned above, but is preferably a $C_1-C_4$ alkyl group. Examples of substituted alkyl groups include the 2-chloroethyl, 2,2-dichloroethyl, 2,2,2-trichloroethyl, 2-cyanoethyl, 2-hydroxyethyl,

4-hydroxybutyl, 2,3-dihydroxypropyl, ethoxymethyl, 2-methoxyethyl, 2-ethoxyethyl, 2-propoxyethyl, 2-ethylthioethyl, 2-phenoxyethyl, 2-benzoyloxyethyl, 2-acetoxyethyl, 2,3-epoxypropyl, carboxymethyl, 2-carboxyethyl, ethoxycarbonylmethyl, 2-diethylamino-ethyl, 2-dimethylamino-1-methylethyl, 3-pyridylmethyl and oxolan-2-ylmethyl groups. Of these, $C_1$-$C_4$ alkyl groups having at least one halogen, $C_1$-$C_4$ alkoxy, $C_1$-$C_4$ alkylthio or ($C_1$-$C_4$ alkoxy)carbonyl substituent are preferred.

Where $R^6$ represents an alkenyl group, this preferably has 3 or 4 carbon atoms, and examples include the allyl, methallyl and 2-butenyl groups.

Where $R^6$ represents an alkynyl group, this likewise preferably has 3 or 4 carbon atoms, the preferred alkynyl group being the 2-propynyl group.

Where $R^6$ represents a cycloalkyl group or substituted cycloalkyl group, the cycloalkyl group preferably has 5 or 6 carbon atoms, the most preferred groups being the cyclohexyl and 2-methylcyclohexyl groups.

Where $R^6$ represents an aralkyl group or a substituted aralkyl group, the aralkyl group is

preferably selected from the group consisting of the benzyl, phenethyl and phenylpropyl groups, and the substituted or unsubstituted aralkyl group is most preferably the benzyl, 4-methylbenzyl, 4-nitrobenzyl, 4-chlorobenzyl, phenethyl or 3-phenylpropyl group.

Where $R^6$ represents a substituted phenyl group, this is preferably a 2-chlorophenyl, 4-chlorophenyl, 2,4-dichlorophenyl, 2,5-dichlorophenyl, 2,6-dichlorophenyl, 3,5-dichlorophenyl, 2,3,4-trichlorophenyl, 2,3,5-trichlorophenyl, 2,4,6-trichlorophenyl, 3,4,5-trichlorophenyl, 2-methylphenyl, 2,4-dimethylphenyl, 2,6-dimethylphenyl, 3,4-dimethylphenyl, 3,5-dimethylphenyl, 2-chloro-6-methylphenyl, 4-chloro-2-methylphenyl, 4-chloro-3-methylphenyl, 2,4-dichloro-6-methylphenyl, 2,6-dichloro-4-methylphenyl, 4-chloro-3,5-dimethylphenyl, 4-chloro-2-nitrophenyl, 2-chloro-4-nitrophenyl, 4-chloro-3,5-dimethyl-6-nitrophenyl, 2-allylphenyl, 2-methoxyphenyl, 3-methoxyphenyl, 4-formylphenyl, 4-acetylphenyl, 4-methoxycarbonylphenyl, 4-butoxycarbonylphenyl, 3-dimethylaminophenyl, 4-acetylaminophenyl and 4-cyanophenyl groups.

Where $R^6$ represent a dialkylamino group, this is preferably a dimethylamino, diethylamino or dipropylamino group.

Where $R^6$ represents a heterocyclic group, this is a heterocyclic group having 5 or 6 ring atoms, at least one of which is a hetero-atom selected from the group consisting of nitrogen atoms and oxygen atoms. The heterocyclic ring itself may be substituted or unsubstituted and, where it is substituted, it preferably has from 1 to 3 $C_1$-$C_4$ alkyl substituents. Examples of such heterocyclic rings represented by $R^6$ include the 5-methylisoxazol-3-yl, 2-pyrimidyl, 2-pyridyl, 3-pyridyl and 1-methyl-3-piperidyl groups.

Also, compounds of formula (I) where $R^4$ represents a carboxy or thiocarboxy group or an alkyl or alkenyl group having one or two carboxy or thiocarboxy substituents can form acid halides, e.g. compounds corresponding to those of formula (I) but in which $R^4$ is replaced by a group of formula -CX".Hal or $-A(-CX".Hal)_n$, in which: A and n are as defined above; X" represents an oxygen or sulfur atom; and Hal represents a halogen atom, particularly a chlorine, fluorine, bromine or iodine atom, preferably a chlorine or bromine atom.

Those compounds where $R^4$ represents a carboxy or thiocarboxy group or an alkyl or alkenyl group having one or two carboxy or thiocarboxy substituents or $R^5$

represents a carboxy group can also form acid amides, e.g. compounds corresponding to those of formula (I) but in which $R^4$ is replaced by a group of formula $-CX''.NR^7R^8$, particularly $-CO.NR^7R^8$, or a group of formula $-A(-CX''.NR^7R^8)_n$, or $R^5$ represents a group of formula $-CO.NR^7R^8$, in which: A, X" and n are as defined above; $R^7$ represents hydrogen, a $C_1$-$C_6$ alkyl group, a $C_1$-$C_6$ hydroxyalkyl group or a $C_2$-$C_6$ alkenyl group; and $R^8$ represents hydrogen, a $C_1$-$C_6$ alkyl group, a substituted $C_1$-$C_6$ alkyl group [having at least one substituent selected from the group consisting of halogen atoms, cyano groups, hydroxy groups, $C_1$-$C_6$ alkoxy groups, phenoxy groups, phenoxy groups having at least one halogen substituent, carboxy groups, ($C_1$-$C_6$ alkoxy)carbonyl groups, di($C_1$-$C_6$ alkyl)amino groups and 5- or 6-membered heterocyclic rings containing at least one hetero-atom selected from the group consisting of nitrogen atoms and oxygen atoms], a $C_2$-$C_6$ alkenyl group, a $C_2$-$C_6$ alkynyl group, a $C_3$-$C_8$ cycloalkyl group, a substituted $C_3$-$C_8$ cycloalkyl group having at least one $C_1$-$C_6$ alkyl substituent, an aralkyl group, a substituted aralkyl group (having at least one substituent selected from the group consisting of halogen atoms, $C_1$-$C_6$ alkyl groups and nitro groups), a phenyl group, a substituted phenyl group [having at least one substituent selected from the group consisting of

halogen atoms, hydroxy groups, nitro groups, cyano groups, $C_1$-$C_6$ alkyl groups, trifluoromethyl groups, $C_1$-$C_6$ alkoxy groups and ($C_1$-$C_6$ alkoxy)carbonyl groups], a hydroxy group, a $C_1$-$C_6$ alkoxy group, a $C_1$-$C_6$ alkanoyloxy group, a $C_1$-$C_6$ alkanoyl group, a substituted $C_1$-$C_6$ alkanoyl group having at least one $C_1$-$C_6$ alkoxy substituent, a ($C_1$-$C_6$ alkoxy)-thiocarbonyl group, a ($C_1$-$C_6$ alkyl)thiocarbamoyl group, a phenylthiocarbamoyl group, a phenylthiocarbamoyl group having at least one halogen substituent on the phenyl moiety, an amino group, a di($C_1$-$C_6$ alkyl)amino group, an anilino group or a 5- or a 6-membered heterocyclic group containing at least one hetero-atom selected from the group consisting of nitrogen, sulfur and oxygen atoms; or $R^7$ and $R^8$, together with the nitrogen atom to which they are attached, represent a piperidino group or a morpholino group.

In these groups of formulae $-CX''.NR^7R^8$ and $-CO.NR^7R^8$, the portion of formula $-NR^7R^8$ is preferably an amino, methylamino, ethylamino, propylamino, isopropylamino, butylamino, isobutylamino, sec-butylamino, t-butylamino, pentylamino, hexylamino, 3-chloropropylamino, dimethylamino, diethylamino, dipropylamino, diisopropylamino, ethylbutylamino, 2-chloroethylamino, cyanomethylamino, 2-cyanoethylamino,

hydroxymethylamino, 2-hydroxyethylamino, 2-hydroxyethyl(methyl)amino, 2-hydroxyethyl(ethyl)amino, di(2-hydroxyethyl)amino, 2,3,4,5,6-pentahydroxyhexyl(methyl)amino, 2-methoxyethylamino, 2-(2,6-dichlorophenoxy)ethylamino, carboxymethylamino, methoxycarbonylmethylamino, 2-(dimethylamino)ethylamino, 2-(diisopropylamino)ethylamino, 3-(dimethylamino)propylamino, 2-morpholinoethylamino, allylamino, diallylamino, 2-propynylamino, cyclopentylamino, cyclohexylamino, 2,3-dimethylcyclohexylamino, benzylamino, 4-chlorobenzylamino, 4-nitrobenzylamino, 4-methylbenzylamino, phenethylamino, α,α-dimethylbenzylamino, anilino, 4-chloroanilino, 3,4-dichloroanilino, 3-chloro-2-hydroxyanilino, 2-hydroxyanilino, 3-hydroxyanilino, 4-hydroxyanilino, 4-nitroanilino, 4-methylanilino, 2,6-dimethylanilino, 2,6-diethylanilino, 2,6-diisopropylanilino, 3-trifluoromethylanilino, 4-cyanoanilino, 2-methoxyanilino, 3-methoxyanilino, 4-methoxyanilino, 2-ethoxyanilino, 3-ethoxyanilino, 4-ethoxyanilino, 2-methoxycarbonylanilino, hydroxyamino, methoxyamino, acetoxyamino, 3-pyridylmethylamino, 4-pyridylmethylamino, formyl(methyl)amino, methoxyacetyl(methyl)amino, methoxythiocarbonylamino, 3-methylthioureido, 3-(4-chlorophenyl)thioureido, hydrazino, 1-methylhydrazino, 2-dimethylhydrazino, 2-phenylhydrazino, acetohydrazino, piperidinoamino, morpholinoamino,

2-pyrimidylamino, 2-pyridylamino, 3-pyridylamino, 4-pyridylamino, 2-thiazolylamino, piperidino or morpholino group, preferably an amino, ($C_1$-$C_6$ alkyl)amino, ($C_1$-$C_6$ alkoxy)amino or morpholinoamino group.

Compounds of formula (I) in which $R^4$ represents an alkyl or alkenyl group having one or two hydroxy or mercapto substituents are alcohols or thiols and can thus form esters with acids, as can those compounds where $R^4$ represents an alkyl group having a hydroxyimino (=N-OH) substituent. Such compounds correspond to those of formula (I), but in which $R^4$ is replaced by a group of formula $-A(X''R^9)_n$ or $-A=N-OR^9$, in which A, X" and <u>n</u> are as defined above and $R^9$ represents an acyl group derived from a carboxylic, sulfonic or phosphorus acid.

Where $R^9$ represents an acyl group derived from a carboxylic acid, this may be an aliphatic or aromatic acyl group. Aliphatic acyl groups preferably have from 1 to 7, more preferably from 2 to 4, carbon atoms and may be alkanoyl, haloalkanoyl, alkoxyalkanoyl or alkoxycarbonyl groups; preferred such groups include formyl, acetyl, propionyl, butyryl, isobutyryl, valeryl, isovaleryl, pivaloyl, hexanoyl, trichloroacetyl, trifluoroacetyl, methoxyacetyl, methoxycarbonyl and

ethoxycarbonyl groups. The aromatic acyl groups are preferably arylcarbonyl groups, of which the aryl moiety is preferably a phenyl group optionally having from 1 to 3 substituents selected from the group consisting of $C_1$-$C_4$ alkyl groups (e.g. methyl, ethyl, or t-butyl) and halogen atoms (e.g. chlorine). Preferred aromatic acyl groups are the benzoyl, toluoyl, and o-, m- or p-chlorobenzoyl groups.

Where $R^9$ represents an acyl group derived from a sulfonic acid, this is preferably an alkanesulfonyl group, for example the methanesulfonyl or ethanesulfonyl group.

Where $R^9$ represents an acyl group derived from a phosphorus acid, this is preferably a di($C_1$-$C_3$ alkoxy)phosphoryl group, e.g. a dimethoxyphosphoryl or diethoxyphosphoryl group.

Where $R^4$ represents an amino group having 1 or 2 aliphatic carboxylic acyl substituents, an aliphatic carboxylic acyl group or a $C_1$-$C_4$ alkyl or $C_2$-$C_4$ alkenyl group having 1 or 2 aliphatic carboxylic acyl substituents, or $R^5$ represents an acyl group, the acyl groups are preferably chosen from the aliphatic acyl groups listed above, amongst the examples of groups which may be represented by $R^9$. Other preferred

carboxylic acyl groups which may be represented by $R^4$ include $C_1$-$C_6$ alkanoyl and $C_3$-$C_6$ alkenoyl groups having 1 or 2 ($C_1$-$C_4$ alkoxy)carbonyl, e.g. methoxycarbonyl or ethoxycarbonyl, substituents, for example the 2,2-di(ethoxycarbonyl)acetyl and 2,2-di(ethoxycarbonyl)pent-4-enoyl groups.

Where $R^4$ represents a heterocyclic group, this is preferably a heterocyclic group having from 3 to 6, more preferably 3, 5 or 6, ring atoms, at least one of which is a hetero-atom selected from the group consisting of nitrogen, oxygen and sulfur atoms. Preferably 1, 2, 3 or 4 of the ring atoms are selected from said hetero-atoms. The heterocyclic ring itself may be substituted or unsubstituted and, where it is substituted, it preferably has from 1 to 3 $C_1$-$C_4$ alkyl substituents (e.g. methyl, ethyl, propyl or butyl substituents, of which the methyl substituents are preferred). Examples of such heterocyclic groups which may be represented by $R^4$ include the dioxolanyl groups (preferably the 1,3-dioxolanyl and more preferably 1,3-dioxolan-2-yl groups), oxiranyl groups, oxazolinyl groups (preferably the 2-oxazolinyl group), oxazinyl groups and their dihydro analogues (preferably the 1,3-oxazinyl and more preferably the 1,3-oxazin-2-yl groups and their analogues, such as 4,5-dihydro-1,3-oxazin-2-yl), the oxadiazolyl groups and

alkyl- and oxo- substituted oxadiazolyl groups (particularly the 1,3,4- and 1,2,4-oxadiazolyl groups and substituted groups, such as the 1,3,4-oxadiazol-2-yl, 2-methyl-1,3,4-oxadiazol-5-yl and 5-oxo-1,2,4-oxadiazol-3-yl groups), and tetrazolyl groups and alkyl-substituted tetrazolyl groups (particularly the 1*H*-tetrazol-5-yl, 1-methyl-1*H*-tetrazol-5-yl and 2-methyl-2*H*-tetrazol-5-yl groups).

Where $R^4$ represents a $C_2$-$C_4$ alkenyl group or a substituted $C_2$-$C_4$ alkenyl group, or $R^5$ represents a $C_2$-$C_4$ alkenyl group, the alkenyl group is preferably a vinyl, allyl, 1-propenyl, isopropenyl, 1-butenyl, 2-butenyl or 3-butenyl group, more preferably a vinyl, 1-propenyl or 1-butenyl group.

Where $R^4$ represents a substituted $C_1$-$C_4$ alkyl group, the alkyl group is preferably a methyl, ethyl, propyl, isopropyl, butyl, isobutyl, sec-butyl or t-butyl group, more preferably a methyl, ethyl or propyl group.

Where $R^4$ or $R^5$ represents an alkyl group having a halogen substituent, the halogen atom is preferably a fluorine, chlorine, bromine or iodine atom, more preferably a chlorine or bromine atom.

Where $R^4$ represents an alkyl or alkenyl group having a di($C_1$-$C_3$ alkyl)phosphono substituent, the substituent is preferably selected from those listed above in relation to $R^9$.

Where $R^4$ represents an alkyl group having a heterocyclic substituent, the heterocyclic group is preferably as defined for $R^6$ and, in this case, is more preferably an azolyl group (i.e. a 5-membered heterocyclic group of which at least 1 hetero-atom is nitrogen), most preferably an imidazolyl or 1,2,4-triazolyl group.

Where $R^5$ represents an alkyl group having at least one $C_1$-$C_4$ alkoxy substituent, the alkoxy group is preferably a methoxy, ethoxy, propoxy, isopropoxy, butoxy or t-butoxy group.

Where $R^5$ represents an alkyl group having at least one aryl or aryloxy substituent, or $R^5$ represents an aryl group, the aryl group is an aromatic carbocyclic group (more preferably phenyl or naphthyl and most preferably phenyl), which may be unsubstituted or may have from 1 to 3 substituents selected from halogen atoms (particularly chlorine or bromine) and $C_1$-$C_4$ alkyl groups (particularly methyl or ethyl).

Where $R^5$ represents an alkyl group having at least one $C_1$-$C_4$ alkylamino substituent, this is preferably a methylamino, ethylamino, propylamino, butylamino or t-butylamino group.

Where $R^5$ represents a $C_3$-$C_8$ cycloalkyl group or a $C_2$-$C_4$ alkynyl group, preferred examples of these groups are as given in relation to the corresponding groups represented by $R^6$.

Where $R^5$ represents an acyl group, this may be derived from a carboxylic, sulfonic or phosphorus acid, and preferred examples are given in relation to $R^9$.

Where $R^1$, $R^2$, $R^3$ or $R^5$ represents a $C_1$-$C_6$ alkyl group, this may be a straight or branched chain group and preferably has from 1 to 4 carbon atoms. Examples include the methyl, ethyl, propyl, isopropyl, butyl, sec-butyl, t-butyl, pentyl, isopentyl, hexyl and 2,2-dimethylbutyl groups, of which the methyl, ethyl, isopropyl and sec-butyl groups are more preferred. In the case of $R^1$ and $R^2$, the most preferred groups represented by these symbols are the isopropyl groups, or $R^1$ is the ethyl group and $R^2$ is the sec-butyl group.

Where $R^1$ or $R^2$ represents a $C_2$-$C_6$ alkenyl

group, this may be a straight or branched chain group and is preferably a group having 3 or 4 carbon atoms, such as the allyl, methallyl, 1-methylpropenyl and 2-butenyl groups.

Where $R^3$ represents a halogen atom, this may be a chlorine, bromine, fluorine or iodine atom, preferably a chlorine or bromine atom. It is, however, most preferred that $R^3$ should represent a hydrogen atom.

A preferred class of compounds of the present invention may be represented by the formula (II), in which, for the avoidance of doubt, there is also indicated the numbering system applied to the benzene ring of the phthalimide portion of the molecule:

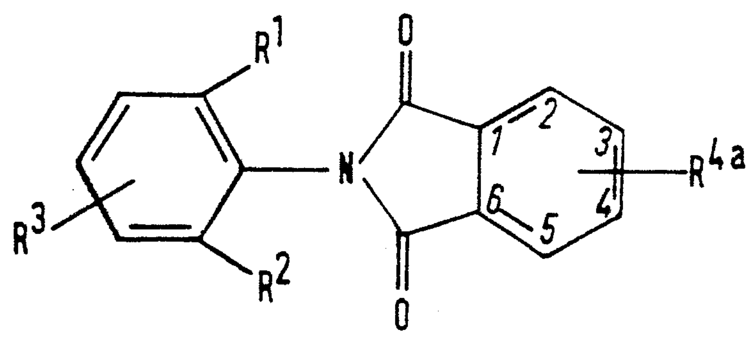

(II)

In the above formula, $R^1$, $R^2$ and $R^3$ are as defined above and $R^{4a}$ represents a group of formula $-CO.R^{30}$, $-CO.X"R^{11}$, $-A-COX"R^{31}$, $-CONR^7R^8$, $-A-CONR^7R^8$, $-A-NHR^{32}$, $-A-X"R^{33}$ or $-A-R^{34}$ or a cyano or heterocyclic group.

Where $R^{4a}$ represents a group of formula $-CO.R^{30}$, $R^{30}$ represents a hydrogen or halogen atom, preferably a fluorine, chlorine, bromine or iodine atom, more preferably a chlorine or bromine atom.

In the group of formula $-CO.X"R^{11}$: X" represents an oxygen or sulfur atom; and $R^{11}$ represents hydrogen, an alkali metal atom, an alkaline earth metal atom, an atom of another innocuous metal, an ammonium group, an organic amine residue, or any one of the groups represented by $R^6$.

Where $R^{11}$ represents a metal (such as an alkali metal, an alkaline earth metal or a trivalent metal), an ammonium group or an organic amine residue, these are salts of the compounds of the invention, the preferred salts being: alkali metal salts, such as sodium or potassium salts; alkaline earth metal salts, such as calcium or magnesium salts; the ammonium salts; and organic amine salts, such as the trimethylamine, triethylamine, tripropylamine and tributylamine salts. Preferred examples of other groups which may be

represented by $R^{11}$ are given above in relation to $R^6$.

When $R^{4a}$ represents a group of formula $-A-COX''R^{31}$, A and X" are as defined above and $R^{31}$ represents hydrogen or a $C_1$-$C_6$ alkyl group.

In the groups of formulae $-CO.NR^7R^8$ and $-A-CONR^7R^8$, A, $R^7$ and $R^8$ are as defined above and examples are also given above.

Where $R^{4a}$ represents a group of formula $-A-NHR^{32}$, A is as defined above, preferably a $C_1$-$C_4$ alkylene group, and $R^{32}$ represents a phenyl group or a phenyl group having a $C_1$-$C_6$ alkyl or ($C_1$ - $C_6$ alkyl)carbamoyl substituent.

Where $R^{4a}$ represents a group of formula $-A-X''R^{33}$, X" and A are as defined above, A preferably being a $C_1$-$C_4$ alkylene group, and $R^{33}$ represents hydrogen or a $C_1$-$C_6$ alkanoyl, $C_1$-$C_6$ haloalkanoyl, benzoyl or $C_1$-$C_6$ alkyl group.

Where $R^{4a}$ represents a group of formula $-A-R^{34}$, A is as defined above, preferably a $C_1$-$C_4$ alkylene group, and $R^{34}$ represents a halogen atom, the cyano group or a $C_1$-$C_6$ alkanoyl group.

Where $R^{4a}$ represents a heterocyclic group, it is preferably an oxiranyl, 1,3,4-oxadiazol-2-yl or 5-oxo-1,2,4-oxadiazol-3-yl group.

We particularly prefer compounds of formula (I) or (II) in which $R^4$ or $R^{4a}$, respectively, is present at the 3- position.

Particularly preferred compounds are compounds of formula (II) in which $R^{4a}$ (which is preferably at the 3- position) represents a group of formula $-COR^{30}$, $-CO.X"R^{11}$, $-A-COX"R^{31}$, $-CO.NHR^8$, $-A-CONHR^8$, $-A-X"R^{33}$ or $-A-R^{34}$, or a cyano or 1,3,4-oxadiazol-2-yl group (in which X" represents an oxygen or sulphur atom; $R^{11}$ represents hydrogen, a $C_1-C_{10}$ alkyl group or a phenyl group; $R^8$ represents hydrogen or a $C_1-C_6$ alkyl group; A represents a $C_1-C_4$ alkylene or alkenylene group; $R^{30}$ represents hydrogen; and $R^{33}$ and $R^{34}$ are as defined above).

Particularly preferred compounds of formulae (I) and (II) are compounds in which $R^3$ represents a hydrogen atom, and more preferably $R^1$ and $R^2$ are the same and both represent ethyl groups or isopropyl groups, or $R^1$ is the ethyl group and $R^2$ is the sec-butyl group and especially preferred are these compounds of formula (II) in which $R^{4a}$ is also as defined in the preceding paragraph.

Examples of compounds of the present invention are given in the following list; the numbers appended to the compounds in this list are used hereinafter, where appropriate, to refer to these compounds:

1. 2-carboxy-N-(2,6-diethylphenyl)phthalimide, melting at 194-195°C.

2. 2-carboxy-N-(2,6-diisopropylphenyl)phthalimide, melting at 187-188°C.

3. 3-carboxy-N-(2-methyl-6-propylphenyl)phthalimide, melting at 179-180°C.

4. 3-carboxy-N-(6-isopropyl-2-methylphenyl)phthalimide, melting at 214-215°C.

5. N-(6-t-butyl-2-methylphenyl)-3-carboxyphthalimide, melting at 200-201°C.

6. 3-carboxy-N-(2,6-diethylphenyl)phthalimide, melting at 224-225°C.

7. 3-carboxy-N-(2-ethyl-6-propylphenyl)phthalimide, melting at 271-272°C.

8. 3-carboxy-N-(2-ethyl-6-isopropylphenyl)phthalimide, melting at 228-230°C.

9. N-(2-sec-butyl-6-ethylphenyl)-3-carboxyphthalimide, melting at 210-211°C.

10. 3-carboxy-N-(2-isopropyl-6-propylphenyl)-phthalimide, melting at 211-212°C.

11. 3-carboxy-N-(2,6-diisopropylphenyl)phthalimide, melting at 274-275°C.

12. N-(2-allyl-6-methylphenyl)-3-carboxyphthalimide, melting at 187-188°C.

13. N-(2-allyl-6-ethylphenyl)-3-carboxyphthalimide, melting at 245-246°C.

14. N-(2-allyl-6-isopropylphenyl)-3-carboxyphthalimide, melting at 135-136°C.

15. N-(2-allyl-6-propylphenyl)-3-carboxyphthalimide

16. 3-carboxy-N-(2,6-dipropylphenyl)phthalimide

17. 3-carboxy-N-(2,6-di-sec-butylphenyl)phthalimide

18. N-(2-sec-butyl-6-methallylphenyl)-3-carboxyphthalimide

19. 3-carboxy-N-(2,6-di-t-butylphenyl)phthalimide

20. 3-carboxy-N-(2,5-dimethyl-6-propylphenyl)-phthalimide, melting at 206-207°C.

21. 3-carboxy-N-(4-chloro-2,6-diethylphenyl)-phthalimide, melting at 201-202°C.

22. 3-carboxy-N-(2,4,6-triethylphenyl)phthalimide, melting at 188-189°C.

23. 3-carboxy-N-(2,5-diethyl-6-propylphenyl)-phthalimide, melting at 152-153°C.

24. 3-carboxy-N-(2,5-diisopropyl-6-propylphenyl)-phthalimide, melting at 257-258°C.

25. 3-carboxy-N-(4-chloro-2,6-diisopropylphenyl)-phthalimide, melting at 252-253°C.

26. 3-carboxy-N-(2,4,6-triisopropylphenyl)phthalimide, melting at 297.5-298.5°C.

27. N-(2-allyl-3,6-dimethylphenyl)-3-carboxy-phthalimide, melting at 202-203°C.

28. N-(2-allyl-5,6-dimethylphenyl)-3-carboxy-phthalimide, melting at 198-200°C.

29. N-(2-allyl-3,6-diethylphenyl)-3-carboxyphthalimide, melting at 138-140°C.

30. N-(2-allyl-3,6-diisopropylphenyl)-3-carboxyphthalimide, melting at 248-249°C.

31. N-(2,6-diisopropylphenyl)-3-thiocarboxyphthalimide.

32. sodium 3-carboxylato-N-(2,6-diisopropylphenyl)-phthalimide, melting point >345°C.

33. ammonium 3-carboxylato-N-(2,6-diisopropylphenyl)-phthalimide, melting at 155-157°C.

34. triethylammonium 3-carboxylato-N-(2,6-diisopropylphenyl)phthalimide, melting at 115-119°C.

35. 2-chloroformyl-N-(2,6-diisopropylphenyl)-phthalimide, melting at 98-100°C.

36. 3-chloroformyl-N-(2,6-dimethylphenyl)phthalimide, melting at 132-133°C.

37. 3-chloroformyl-N-(2-ethyl-6-methylphenyl)-phthalimide, melting at 108-109°C.

38. 3-chloroformyl-N-(2-isopropyl-6-methylphenyl)-phthalimide, melting at 133-135°C.

39. N-(2-t-butyl-6-methylphenyl)-3-chloroformylphthalimide, melting at 139-140°C.

40. 3-chloroformyl-N-(2,6-diethylphenyl)phthalimide, melting at 114-115°C.

41. 3-chloroformyl-N-(2-ethyl-6-isopropylphenyl)-phthalimide, melting at 117-120°C.

42. N-(2-sec-butyl-6-ethylphenyl)-3-chloroformylphthalimide, melting at 145-146°C.

43. N-(2-allyl-6-ethylphenyl)-3-chloroformylphthalimide, $n_D^{25}$ = 1.6003.

44. 3-chloroformyl-N-(2,6-diisopropylphenyl)-phthalimide, melting at 148-150°C.

45. N-(4-bromo-2,6-dimethylphenyl)-3-chloroformylphthalimide, melting at 155-157°C.

46. N-(4-chloro-2,6-dimethylphenyl)-3-chloroformylphthalimide, melting at 147-148°C.

47. 3-chloroformyl-N-(2,4,6-trimethylphenyl)-phthalimide, melting at 150-153°C.

48. N-(4-chloro-2,6-diethylphenyl)-3-chloroformylphthalimide, melting at 162-163°C.

49. 3-chloroformyl-N-(2,4,6-triethylphenyl)-phthalimide, melting at 152-153°C.

50. 3-chloroformyl-N-(3,6-dimethyl-2-propylphenyl)-phthalimide, melting at 126-127°C.

51. 3-chloroformyl-N-(3,6-diisopropyl-2-propylphenyl)-phthalimide, melting at 148-149°C.

52. N-(4-chloro-2,6-diisopropylphenyl)-3-chloroformyl-phthalimide, melting at 178-179°C.

53. 3-chloroformyl-N-(2,4,6-triisopropylphenyl)-phthalimide, melting at 214-215°C.

54. N-(2-allyl-3,6-dimethylphenyl)-3-chloroformyl-phthalimide, melting at 102-103°C.

55. N-(2-allyl-5,6-dimethylphenyl)-3-chloroformyl-phthalimide, melting at 116-117°C.

56. N-(2-allyl-3,6-diisopropylphenyl)-3-chloroformyl-phthalimide, melting at 132-135°C.

57. N-(2,6-diethylphenyl)-2-methoxycarbonylphthalimide, melting at 92.5-93.5°C.

58. N-(2,6-diisopropylphenyl)-2-methoxycarbonylphthalimide, melting at 134-135°C.

59. N-(2-isopropyl-6-methylphenyl)-3-methoxycarbonylphthalimide, melting at 40-45°C.

60. N-(2-t-butyl-6-methylphenyl)-3-methoxycarbonylphthalimide, melting at 132-133°C.

61. N-(2,6-diethylphenyl)-3-methoxycarbonylphthalimide, melting at 95-96.5°C.

62. N-(2-ethyl-6-isopropylphenyl)-3-methoxycarbonylphthalimide, melting at 112-114°C.

63. N-(2-sec-butyl-6-ethylphenyl)-3-methoxycarbonylphthalimide, melting at 115-116°C.

64. N-(2-allyl-6-ethylphenyl)-3-methoxycarbonylphthalimide, melting at 101-102°C.

65. N-(2,6-diisopropylphenyl)-3-methoxycarbonylphthalimide, melting at 173-173.5°C.

66. N-(4-bromo-2,6-dimethylphenyl)-3-methoxycarbonylphthalimide, melting at 154-155°C.

67. N-(3,6-dimethyl-2-propylphenyl)-3-methoxycarbonyl-phthalimide, $n_D^{22}$ = 1.5746.

68. 3-methoxycarbonyl-N-(2,4,6-triethylphenyl)-phthalimide, melting at 111-112°C.

69. N-(3,6-diisopropyl-2-propylphenyl)-3-methoxy-carbonylphthalimide, melting at 161-162°C.

70. N-(4-chloro-2,6-diisopropylphenyl)-3-methoxy-carbonylphthalimide, melting at 165-166°C.

71. 3-methoxycarbonyl-N-(2,4,6-triisopropylphenyl)-phthalimide, melting at 184-185°C.

72. N-(2-allyl-5,6-dimethylphenyl)-3-methoxycarbonyl-phthalimide, melting at 144-145°C.

73. N-(2-allyl-3,6-diisopropylphenyl)-3-methoxy-carbonylphthalimide, melting at 155-156°C.

74. N-(2,6-diethylphenyl)-3-ethoxycarbonylphthalimide, melting at 86-87°C.

75. N-(2,6-diisopropylphenyl)-3-ethoxycarbonyl-phthalimide, melting at 133-135°C.

76. N-(2,6-diethylphenyl)-2-isopropoxycarbonyl-phthalimide, melting at 101-102°C.

77. N-(2,6-diisopropylphenyl)-3-propoxycarbonyl-phthalimide, melting at 122-125°C.

78. 3-butoxycarbonyl-N-(2,6-diisopropylphenyl)-phthalimide, melting at 110-111°C.

79. N-(2,6-diisopropylphenyl)-3-isobutoxycarbonyl-phthalimide, melting at 52-54°C.

80. 3-t-butoxycarbonyl-N-(2,6-diisopropylphenyl)-phthalimide, melting at 176-178°C.

81. N-(2,6-diisopropylphenyl)-3-pentyloxycarbonyl-phthalimide, melting at 78-79°C.

82. N-(2,6-diisopropylphenyl)-3-hexyloxycarbonyl-phthalimide, melting at 65-67°C.

83. N-(2,6-diisopropylphenyl)-3-heptyloxycarbonyl-phthalimide, melting at 62-64°C.

84. N-(2,6-diisopropylphenyl)-3-octyloxycarbonyl-phthalimide, melting at 58.6°C.

85. N-(2,6-diisopropylphenyl)-3-nonyloxycarbonyl-phthalimide, melting at 53.7°C.

86. 3-decyloxycarbonyl-N-(2,6-diisopropylphenyl)-phthalimide, melting at 63.0°C.

87. N-(2,6-diisopropylphenyl)-3-dodecyloxycarbonyl-phthalimide, melting at 56.2°C.

88. N-(2,6-diisopropylphenyl)-3-tetradecyloxycarbonyl-phthalimide, melting at 60.6°C.

89. N-(2,6-diisopropylphenyl)-3-hexadecyloxycarbonyl-phthalimide, melting at 67.6°C.

90. N-(2,6-diisopropylphenyl)-3-octadecyloxycarbonyl-phthalimide, melting at 72.1°C.

91. N-(2,6-diisopropylphenyl)-3-(methylthio)carbonyl-phthalimide, melting at 166-168°C.

92. N-(2,6-diethylphenyl)-3-(ethylthio)carbonyl-phthalimide, melting at 124.5-126°C.

93. N-(2,6-diisopropylphenyl)-3-(ethylthio)carbonyl-phthalimide, melting at 168-169°C.

94. N-(2,6-diethylphenyl)-3-(propylthio)carbonylphthalimide, melting at 114.5-116°C.

95. 3-(2-chloroethoxycarbonyl)-N-(2,6-diisopropylphenyl)phthalimide, melting at 57-60°C.

96. N-(2,6-diisopropylphenyl)-3-(2,2,2-trichloroethoxycarbonyl)phthalimide, melting at 64-66°C.

97. N-(2,6-diisopropylphenyl)-3-(2,2,2-trifluoroethoxycarbonyl)phthalimide, melting at 142-145°C.

98. 3-(2-cyanoethoxycarbonyl)-N-(2,6-diisopropylphenyl)phthalimide, melting at 56-59°C.

99. N-(2,6-diisopropylphenyl)-3-(2-hydroxyethoxycarbonyl)phthalimide, melting at 149-150°C.

100. N-(2,6-diisopropylphenyl)-3-(2,3-dihydroxypropoxycarbonyl)phthalimide, melting at 59-61°C.

101. N-(2,6-diisopropylphenyl)-3-(2-methoxyethoxycarbonyl)phthalimide, melting at 45-48°C.

102. N-(2,6-diisopropylphenyl)-3-(2-ethoxyethoxycarbonyl)phthalimide, melting at 85-87°C.

103. N-(2,6-diisopropylphenyl)-3-(2-propoxyethoxycarbonyl)phthalimide, melting at 100-102°C.

104. N-(2,6-diisopropylphenyl)-3-(2-ethylthioethoxycarbonyl)phthalimide, melting at 97-99°C.

105. N-(2,6-diisopropylphenyl)-3-(2-phenoxyethoxycarbonyl)phthalimide, melting at 35-38°C.

106. 3-(2-acetoxyethoxycarbonyl)-N-(2,6-diisopropylphenyl)phthalimide, melting at 126.9°C.

107. 3-(2-benzoyloxyethoxycarbonyl)-N-(2,6-diisopropylphenyl)phthalimide.

108. N-(2,6-diisopropylphenyl)-3-(2,3-epoxypropoxycarbonyl)phthalimide, melting at 148.8°C.

109. N-(2,6-diisopropylphenyl)-3-ethoxycarbonylmethylthiocarbonylphthalimide, melting at 128-130°C.

110. N-(2,6-diisopropylphenyl)-3-(2-dimethylaminoethoxycarbonyl)phthalimide, melting at 95-97°C.

111. N-(2,6-diisopropylphenyl)-3-(2-dimethylamino-1-methylethoxycarbonyl)phthalimide, melting at 120-121°C.

112. N-(2,6-diisopropylphenyl)-3-(3-pyridylmethoxycarbonyl)phthalimide, melting at 53.3°C.

113. N-(2,6-diisopropylphenyl)-3-(2-tetrahydrofuryl-methoxycarbonyl)phthalimide, melting at 102.5°C.

114. 3-allyloxycarbonyl-N-(2,6-diisopropylphenyl)-phthalimide, melting at 122-125°C.

115. N-(2,6-diisopropylphenyl)-3-(2-propynyloxy-carbonyl)phthalimide, melting at 150-153°C.

116. 3-cyclohexyloxycarbonyl-N-(2,6-diisopropylphenyl)-phthalimide, melting at 126-128°C.

117. N-(2,6-diisopropylphenyl)-3-(2-methylcyclohexyloxycarbonyl)phthalimide, melting at 77-78°C.

118. N-(2,6-diisopropylphenyl)-3-(4-methylbenzyloxycarbonyl)phthalimide, melting at 163-164°C.

119. N-(2,6-diisopropylphenyl)-3-(4-nitrobenzyloxycarbonyl)phthalimide, melting at 172-174°C.

120. N-(2,6-diethylphenyl)-3-(3-phenylpropoxy-carbonyl)phthalimide, $n_D^{23}$ = 1.5805.

121. 3-(benzylthio)carbonyl-N-(2,6-diisopropylphenyl)-phthalimide, melting at 129-131°C.

122. 3-[(4-chlorobenzylthio)carbonyl]-N-(2,6-diethylphenyl)phthalimide, melting at 177-179°C.

123. N-(2,6-diisopropylphenyl)-3-phenoxycarbonyl-phthalimide, melting at 175-177°C.

124. 3-(2-chlorophenoxycarbonyl)-N-(2,6-diisopropylphenyl)phthalimide, melting at 148-150°C.

125. N-(2,6-diisopropylphenyl)-3-(2,3,5-trichloro-phenoxycarbonyl)phthalimide, melting at 212-213°C.

126. N-(2,6-diisopropylphenyl)-3-(2-methylphenoxy-carbonyl)phthalimide, melting at 144-146°C.

127. N-(2,6-diisopropylphenyl)-3-(2,4-dimethylphenoxy-carbonyl)phthalimide, melting at 158-160°C.

128. N-(2,6-diisopropylphenyl)-3-(3,4-dimethylphenoxy-carbonyl)phthalimide, melting at 149-151°C.

129. N-(2,6-diisopropylphenyl)-3-(3,5-dimethylphenoxy-carbonyl)phthalimide, melting at 150-152°C.

130. 3-(4-chloro-3-methylphenoxycarbonyl)-N-(2,6-diisopropylphenyl)phthalimide, melting at 175-178°C.

131. 3-(2-chloro-4-nitrophenoxycarbonyl)-N-(2,6-diisopropylphenyl)phthalimide, melting at 232-235°C.

132. 3-(2-allylphenoxycarbonyl)-N-(2,6-diisopropylphenyl)phthalimide, melting at 154-155°C.

133. N-(2,6-diisopropylphenyl)-3-(2-methoxyphenoxycarbonyl)phthalimide, melting at 134.1°C.

134. N-(2,6-diisopropylphenyl)-3-(3-methoxyphenoxycarbonyl)phthalimide, melting at 123-124°C.

135. N-(2,6-diisopropylphenyl)-3-(4-formylphenoxycarbonyl)phthalimide, melting at 201-203°C.

136. 3-(4-acetylphenoxycarbonyl)-N-(2,6-diisopropylphenyl)phthalimide, melting at 202-204°C.

137. 3-(4-butoxycarbonylphenoxycarbonyl)-N-(2,6-diisopropylphenyl)phthalimide, melting at 127-132.5°C.

138. N-(2,6-diisopropylphenyl)-3-(3-dimethylaminophenoxycarbonyl)phthalimide, melting at 129-130°C.

139. 3-(4-acetamidophenoxycarbonyl)-N-(2,6-diisopropylphenyl)phthalimide, melting at 225-228°C.

140. 3-(4-cyanophenoxycarbonyl)-N-(2,6-diisopropylphenyl)phthalimide, melting at 209.5-210.5°C.

141. N-(2,6-diisopropylphenyl)-3-(phenylthio)carbonylphthalimide, melting at 158-161°C.

142. 3-diethylaminooxycarbonyl-N-(2,6-diisopropylphenyl)phthalimide, melting at 127-129°C.

143. N-(2,6-diisopropylphenyl)-3-(5-methylisoxazol-3-yloxycarbonyl)phthalimide, melting at 140-142°C.

144. N-(2,6-diisopropylphenyl)-3-(2-pyrimidinyloxycarbonyl)phthalimide, melting at 178-180°C.

145. N-(2,6-diisopropylphenyl)-3-(3-pyridyloxycarbonyl)phthalimide, melting at 148-150°C.

146. N-(2,6-diisopropylphenyl)-3-(1-methylpiperid-3-yloxycarbonyl)phthalimide, melting at 154-155°C.

147. 2-carbamoyl-N-(2,6-diethylphenyl)phthalimide, melting at 175-176°C.

148. 2-carbamoyl-N-(2,6-diisopropylphenyl)phthalimide, melting at 189-190°C.

149. 3-carbamoyl-N-(2-isopropyl-6-methylphenyl)-phthalimide, melting at 288-290°C.

150. 3-carbamoyl-N-(2,6-diethylphenyl)phthalimide, melting at 247-248°C.

151. 3-carbamoyl-N-(2-ethyl-6-propylphenyl)phthalimide, melting at 242-243°C.

152. 3-carbamoyl-N-(2-ethyl-6-isopropylphenyl)-phthalimide, melting at 242-244°C.

153. 3-carbamoyl-N-(2,6-diisopropylphenyl)phthalimide, melting at 251-252°C.

154. 3-carbamoyl-N-(2,4,6-triethylphenyl)phthalimide, melting at 181-182°C.

155. 3-carbamoyl-N-(2,5-dimethyl-6-propylphenyl)-phthalimide, melting at 215-216°C.

156. 3-carbamoyl-N-(2,5-diisopropyl-6-propylphenyl)-phthalimide, melting at 190-191°C.

157. 3-carbamoyl-N-(2,4,6-triisopropylphenyl)-phthalimide, melting at 250-252°C.

158. N-(2-allyl-5,6-dimethylphenyl)-3-carbamoyl-phthalimide, melting at 209-210°C.

159. N-(2-allyl-3,6-dimethylphenyl)-3-carbamoyl-phthalimide, melting at 195-196°C.

160. N-(2-isopropyl-6-methylphenyl)-3-methylcarbamoyl-phthalimide, melting at 202.5-205°C.

161. N-(2-t-butyl-6-methylphenyl)-3-methylcarbamoyl-phthalimide, melting at 241-242°C.

162. N-(2,6-diethylphenyl)-3-methylcarbamoyl-phthalimide, melting at 172-174°C.

163. N-(2-ethyl-6-isopropylphenyl)-3-methylcarbamoyl-phthalimide, melting at 187-189°C.

164. N-(2-sec-butyl-6-ethylphenyl)-3-methylcarbamoyl-phthalimide, melting at 190-191°C.

165. N-(2-allyl-6-ethylphenyl)-3-methylcarbamoyl-phthalimide, melting at 175-176°C.

166. N-(2,6-diisopropylphenyl)-3-methylcarbamoyl-phthalimide, melting at 217-219°C.

167. N-(2,5-dimethyl-6-propylphenyl)-3-methylcarbamoyl-phthalimide, melting at 182-184°C.

168. N-(2,5-diisopropyl-6-propylphenyl)-3-methylcarbamoylphthalimide, melting at 228-229°C.

169. 3-methylcarbamoyl-N-(2,4,6-triisopropylphenyl)-phthalimide, melting at 274-275°C.

170. N-(2-allyl-5,6-dimethylphenyl)-3-methylcarbamoyl-phthalimide, melting at 196-197°C.

171. N-(2-allyl-3,6-dimethylphenyl)-3-methylcarbamoyl-phthalimide, melting at 125-128°C.

172. N-(2-allyl-3,6-diisopropylphenyl)-3-methyl-carbamoylphthalimide, melting at 224-225°C.

173. N-(2,6-diethylphenyl)-3-ethylcarbamoyl-phthalimide, melting at 141-142.5°C.

174. N-(2,6-diisopropylphenyl)-3-ethylcarbamoyl-phthalimide, melting at 192-194°C.

175. N-(2,6-diethylphenyl)-3-propylcarbamoylphthalimide, melting at 146-147°C.

176. N-(2,6-diisopropylphenyl)-3-propylcarbamoylphthalimide, melting at 168-170°C.

177. 3-isopropylcarbamoyl-N-(6-isopropyl-2-methylphenyl)phthalimide, melting at 189.5-191°C.

178. 3-isopropylcarbamoyl-N-(2,6-diisopropylphenyl)-phthalimide, melting at 201-202°C.

179. 2-butylcarbamoyl-N-(2,6-diethylphenyl)-phthalimide, melting at 100.5-101°C.

180. 3-butylcarbamoyl-N-(2,6-diethylphenyl)-phthalimide, melting at 133.5-135°C.

181. N-(2,6-diethylphenyl)-3-isobutylcarbamoylphthalimide, melting at 159.5-161°C.

182. N-(2,6-diisopropylphenyl)-3-pentylcarbamoylphthalimide, melting at 135-137°C.

183. N-(2,6-diethylphenyl)-3-dimethylcarbamoylphthalimide, melting at 153.5-154.5°C.

184. 3-(2-chloroethylcarbamoyl)-N-(2-isopropyl-6-methylphenyl)phthalimide, melting at 189-190°C.

185. N-(2-t-butyl-6-methylphenyl)-3-(2-chloroethylcarbamoyl)phthalimide, melting at 207-209°C.

186. 3-(2-chloroethylcarbamoyl)-N-(2,6-diethylphenyl)-phthalimide, melting at 129-130°C.

187. N-(2-sec-butyl-6-ethylphenyl)-3-(2-chloroethylcarbamoyl)phthalimide, melting at 151-152°C.

188. 3-(2-chloroethylcarbamoyl)-N-(2,6-diisopropylphenyl)phthalimide, melting at 208-210°C.

189. 3-cyanomethylcarbamoyl-N-(2,6-diisopropylphenyl)-phthalimide, melting at 245-248°C.

190. N-(2,6-diisopropylphenyl)-3-hydroxymethylcarbamoylphthalimide, melting at 172-174°C.

191. 3-(2-hydroxyethylcarbamoyl)-N-(2-isopropyl-6-methylphenyl)phthalimide, melting at 134-135°C.

192. N-(2,6-diethylphenyl)-3-(2-hydroxyethylcarbamoyl)-phthalimide, melting at 144-145°C.

193. N-(2-t-butyl-6-methylphenyl)-3-(2-hydroxyethylcarbamoyl)phthalimide, melting at 195-196°C.

194. N-(2-sec-butyl-6-ethylphenyl)-3-(2-hydroxyethylcarbamoyl)phthalimide, melting at 115-116°C.

195. N-(2,6-diisopropylphenyl)-3-(2-hydroxyethylcarbamoyl)phthalimide, melting at 193-194°C.

196. N-(2,6-diisopropylphenyl)-3-(N-2-hydroxyethyl-N-methylcarbamoyl)phthalimide, melting at 140-142°C.

197. N-(2,6-diisopropylphenyl)-3-(N-ethyl-N-2-hydroxyethylcarbamoyl)phthalimide, melting at 151-153°C.

198. 3-[N,N-bis(2-hydroxyethyl)carbamoyl]-N-(2,6-diisopropylphenyl)phthalimide, melting at 165-167°C.

199. N-(2,6-diisopropylphenyl)-3-(N-2,3,4,5,6-pentahydroxyhexyl-N-methylcarbamoyl)phthalimide, melting at 98-100°C.

200. N-(2,6-diisopropylphenyl)-3-(2-methoxyethylcarbamoyl)phthalimide, melting at 116-119°C.

201. 3-[2-(2,6-dichlorophenoxy)ethylcarbamoyl]-N-(2,6-diisopropylphenyl)phthalimide, melting at 216.5-217.5°C.

202. 3-carboxymethylcarbamoyl-N-(2,6-diisopropylphenyl)phthalimide.

203. N-(2,6-diisopropylphenyl)-3-methoxycarbonylmethylcarbamoylphthalimide, melting at 210-212°C.

204. 3-(2-diisopropylaminoethylcarbamoyl)-N-(2,6-diisopropylphenyl)phthalimide, melting at 182-184°C.

205. N-(2,6-diisopropylphenyl)-3-(3-dimethylaminopropylcarbamoyl)phthalimide, melting at 117-120°C.

206. N-(2,6-diisopropylphenyl)-3-(2-morpholinoethylcarbamoyl)phthalimide, melting at 206-207°C.

207. 3-allylcarbamoyl-N-(2,6-diisopropylphenyl)-phthalimide, melting at 169-171°C.

208. 3-diallylcarbamoyl-N-(2,6-diisopropylphenyl)-phthalimide, melting at 145-147°C.

209. N-(2,6-diisopropylphenyl)-3-propyn-2-ylcarbamoyl-phthalimide, melting at 196-197°C.

210. 3-cyclopentylcarbamoyl-N-(2,6-diisopropylphenyl)-phthalimide, melting at 225-227°C.

211. N-(2,6-diisopropylphenyl)-3-(2,3-dimethyl-cyclohexylcarbamoyl)phthalimide, melting at 258-259°C.

212. 3-benzylcarbamoyl-N-(2,6-diethylphenyl)-phthalimide, melting at 192.5-193.5°C.

213. 3-(4-chlorobenzylcarbamoyl)-N-(2,6-diethylphenyl)phthalimide, melting at 196-197°C.

214. N-(2,6-diisopropylphenyl)-3-(4-nitrobenzyl-carbamoyl)phthalimide, melting at 258-261°C.

215. N-(2,6-diisopropylphenyl)-3-(4-methylbenzyl-carbamoyl)phthalimide, melting at 235-236°C.

216. N-(2,6-diisopropylphenyl)-3-phenethylcarbamoyl-phthalimide, melting at 51-53°C.

217. N-(2,6-diethylphenyl)-3-(α,α-dimethylbenzyl-carbamoyl)phthalimide, melting at 196-197°C.

218. N-(2,6-diisopropylphenyl)-3-phenylcarbamoyl-phthalimide, melting at 253-255°C.

219. 3-(4-chlorophenylcarbamoyl)-N-(2,6-diethylphenyl)phthalimide, melting at 269-270°C.

220. N-(2,6-diisopropylphenyl)-3-(2-hydroxyphenylcarbamoyl)phthalimide, melting at 265.5-266.5°C (with decomposition).

221. N-(2,6-diisopropylphenyl)-3-(4-nitrophenylcarbamoyl)phthalimide, melting at 328-329°C.

222. N-(2,6-diisopropylphenyl)-3-(4-methylphenylcarbamoyl)phthalimide, melting at 263.5-266°C.

223. N-(2,6-diisopropylphenyl)-3-(2,6-dimethylphenylcarbamoyl)phthalimide, melting at 255-258°C.

224. N-(2,6-diisopropylphenyl)-3-(3-trifluoromethylphenylcarbamoyl)phthalimide, melting at 256-260°C.

225. 3-(4-cyanophenylcarbamoyl)-N-(2,6-diisopropylphenyl)phthalimide, melting at 300-304°C.

226. N-(2,6-diisopropylphenyl)-3-(2-methoxyphenylcarbamoyl)phthalimide, melting at 212.5-214°C.

227. N-(2,6-diisopropylphenyl)-3-(4-ethoxyphenylcarbamoyl)phthalimide, melting at 219-220°C.

228. N-(2,6-diisopropylphenyl)-3-(2-methoxycarbonylphenylcarbamoyl)phthalimide, melting at 265-266°C.

229. N-(2,6-diisopropylphenyl)-3-hydroxycarbamoylphthalimide, melting at 93-95°C.

230. N-(2-isopropyl-6-methylphenyl)-3-methoxycarbamoylphthalimide, melting at 190-191°C.

231. N-(2,6-diethylphenyl)-3-methoxycarbamoylphthalimide, melting at 155-156°C.

232. N-(2,6-diisopropylphenyl)-3-methoxycarbamoylphthalimide, melting at 165-167°C.

233. 3-acetoxycarbamoyl-N-(2,6-diisopropylphenyl)phthalimide, melting at 169-170°C.

234. N-(2,6-diisopropylphenyl)-3-(3-pyridylmethylcarbamoyl)phthalimide, melting at 183.2°C.

235. N-(2,6-diisopropylphenyl)-3-(4-pyridylmethylcarbamoyl)phthalimide, melting at 205.3°C.

236. N-(2,6-diisopropylphenyl)-3-(N-methylformamidocarbonyl)phthalimide, melting at 137-140°C.

237. N-(2,6-diethylphenyl)-3-(2-methoxy-N-methylacetamido)carbonylphthalimide, melting at 96-97°C.

238. N-(2,6-diisopropylphenyl)-3-(2-methoxy-N-methylacetamido)carbonylphthalimide, melting at 134-135°C.

239. N-(2,6-diisopropylphenyl)-3-methoxy(thiocarbonyl)-carbamoylphthalimide, melting at 186-187°C.

240. N-(2,6-diisopropylphenyl)-3-[N'-methyl-(thioureido)carbonyl]phthalimide, melting at 193-194°C.

241. 3-[N'-p-chlorophenyl(thioureido)carbonyl]-N-(2,6-diisopropylphenyl)phthalimide, melting at 231-233°C.

242. N-(2,6-diisopropylphenyl)-3-(N-methylhydrazinocarbonyl)phthalimide, melting at 105-108°C.

243. N-(2,6-diisopropylphenyl)-3-(N',N'-dimethylhydrazinocarbonyl)phthalimide, melting at 189-191°C.

244. N-(2,6-diisopropylphenyl)-3-(N'-phenylhydrazinocarbonyl)phthalimide, melting at 180-182°C.

245. N-(2,6-diisopropylphenyl)-3-piperidinocarbamoylphthalimide, melting at 249-251°C (with decomposition).

246. N-(2,6-diisopropylphenyl)-3-morpholinocarbamoylphthalimide, melting at 222-224°C.

247. N-(2,6-diisopropylphenyl)-3-(2-pyrimidinylcarbamoyl)phthalimide, melting at 260-262°C.

248. N-(2,6-diisopropylphenyl)-3-(2-pyridylcarbamoyl)phthalimide, melting at 216-219°C.

249. N-(2,6-diisopropylphenyl)-3-(3-pyridylcarbamoyl)phthalimide, melting at 237°C.

250. N-(2,6-diisopropylphenyl)-3-(4-pyridylcarbamoyl)phthalimide, melting at 171.5°C.

251. N-(2,6-diisopropylphenyl)-3-(2-thiazolylcarbamoyl)phthalimide, melting at 291.5-292.5°C.

252. N-(2,6-diethylphenyl)-3-piperidinocarbonylphthalimide, melting at 190-191°C.

253. N-(2,6-diisopropylphenyl)-3-morpholinocarbonylphthalimide, melting at 230-232°C.

254. 3-cyano-N-(2,6-dimethylphenyl)phthalimide, melting at 153-154°C.

255. 3-cyano-N-(2-ethyl-6-methylphenyl)phthalimide, melting at 97-99°C.

256. 3-cyano-N-(2-isopropyl-6-methylphenyl)-phthalimide, melting at 150-151.5°C.

257. 3-cyano-N-(2,6-diethylphenyl)phthalimide, melting at 115-116°C.

258. 3-cyano-N-(2,6-diisopropylphenyl)phthalimide, melting at 162-163°C.

259. 3-cyano-N-(3,6-dimethyl-2-propylphenyl)-phthalimide, melting at 122-123.5°C.

260. 3-cyano-N-(2,4,6-triethylphenyl)phthalimide, melting at 178-179°C.

261. N-(2-allyl-3,6-dimethylphenyl)-3-cyano-phthalimide, melting at 121-122.5°C.

262. 3-cyano-N-(2,4,6-triisopropylphenyl)phthalimide, melting at 127-128°C.

263. N-(2-allyl-5,6-dimethylphenyl)-3-cyano-phthalimide, melting at 132-134°C.

264. N-(2,6-diisopropylphenyl)-3-(3-hydroxypropylcarbamoyl)phthalimide, melting at 226.5-227.5°C.

265. N-(2,6-diisopropylphenyl)-3-(2-pyridyloxycarbonyl)phthalimide, melting at 153.7°C.

266. aluminum tris[3-carboxylato-N-(2,6-diisopropylphenyl)phthalimide], melting point > 300°C.

267. ferric tris[3-carboxylato-N-(2,6-diisopropylphenyl)phthalimide], gradually started to decompose above 200°C, with a color change.

268. N-(2,6-diisopropylphenyl)-3-hydrazinocarbonylphthalimide, melting at 168 - 169°C.

269. N-(2,6-diisopropylphenyl)-3-(1-methyl-1H-tetrazol-5-yl)phthalimide, melting at 203-204°C.

270. tetrabutylammonium 3-carboxylato-N-(2,6-diisopropylphenyl)phthalimide.

271. calcium bis[3-carboxylato-N-(2,6-diisopropylphenyl)phthalimide].

272. 3-(N'-acetylhydrazinocarbonyl)-N-(2,6-diisopropylphenyl)phthalimide, melting at 165 - 166°C.

273. N-(2,6-diisopropylphenyl)-3-(2-methyl-2H-tetrazol-5-yl)phthalimide, melting at 196-197°C.

274. 3-(3-chloropropylcarbamoyl)-N-(2,6-diisopropylphenyl)phthalimide, melting at 195 - 197°C.

275. 3-hydroxymethyl-N-(2-isopropyl-6-methylphenyl)-phthalimide, a vitreous solid.

276. N-(2-t-butyl-6-methylphenyl)-3-hydroxymethylphthalimide, melting at 152-153°C.

277. N-(2,6-diethylphenyl)-3-hydroxymethylphthalimide, melting at 102-103°C.

278. N-(2-ethyl-6-isopropylphenyl)-3-hydroxymethylphthalimide, melting at 139-141°C.

279. N-(2-sec-butyl-6-ethylphenyl)-3-hydroxymethylphthalimide, a vitreous solid.

280. N-(2,6-diisopropylphenyl)-3-hydroxymethylphthalimide, melting at 187-189°C.

281. N-[2-ethyl-6-(1-methyl-2-propenyl)phenyl)-3-hydroxymethylphthalimide.

282. N-(2-allyl-6-methallylphenyl)-3-hydroxymethyl-phthalimide.

283. 3-acetoxymethyl-N-(2,6-diethylphenyl)phthalimide, melting at 97-98°C.

284. 3-acetoxymethyl-N-(2,6-diisopropylphenyl)-phthalimide, melting at 166-167°C.

285. N-(2,6-diisopropylphenyl)-3-(trifluoroacetoxy)-methylphthalimide, melting at 223-224°C.

286. N-(2,6-diisopropylphenyl)-3-isobutyryloxymethyl-phthalimide, melting at 158-159°C.

287. 3-benzoyloxymethyl-N-(2,6-diisopropylphenyl)-phthalimide, melting at 180-181°C.

288. 3-(4-chlorobenzoyloxymethyl)-N-(2,6-diethylphenyl)phthalimide, melting at 106-107°C.

289. 3-(4-chlorobenzoyloxymethyl)-N-(2,6-diisopropylphenyl)phthalimide, melting at 149-150°C.

290. N-(2,6-diisopropylphenyl)-N-methanesulfonyloxy-methylphthalimide, melting at 136-137°C.

291. N-(2,6-diethylphenyl)-3-methoxymethylphthalimide, melting at 85-88°C.

292. N-(2,6-diisopropylphenyl)-3-methoxymethylphthalimide, melting at 128-129°C.

293. N-(2,6-diisopropylphenyl)-3-ethoxymethylphthalimide, melting at 122-123°C.

294. N-(2,6-diisopropylphenyl)-3-propoxymethylphthalimide, melting at 141-142°C.

295. 3-butoxymethyl-N-(2,6-diisopropylphenyl)-phthalimide, melting at 117-118°C.

296. 3-butoxymethyl-N-(2-ethyl-6-methallylphenyl)-phthalimide.

297. N-(2,6-diisopropylphenyl)-3-(2-ethoxyethoxymethyl)phthalimide, melting at 101-102°C.

298. N-(2,6-diisopropylphenyl)-3-(2-ethylaminoethoxymethyl)phthalimide, melting at 127-128°C.

299. 3-benzyloxymethyl-N-(2,6-diisopropylphenyl)-phthalimide, melting at 104-105°C.

300. N-(2,6-diisopropylphenyl)-3-(2-phenoxyethoxymethyl)phthalimide, melting at 126-127°C.

301. 3-bromomethyl-N-(2-isopropyl-6-methylphenyl)-phthalimide, melting at 124-125°C.

302. 3-chloromethyl-N-(2,6-diethylphenyl)phthalimide, melting at 107°C.

303. 3-bromomethyl-N-(2,6-diethylphenyl)phthalimide, melting at 132-133°C.

304. 3-bromomethyl-N-(2-ethyl-6-isopropylphenyl)-phthalimide, melting at 146-147°C.

305. 3-bromomethyl-N-(2-sec-butyl-6-ethylphenyl)-phthalimide, melting at 175-176°C.

306. 3-chloromethyl-N-(2,6-diisopropylphenyl)-phthalimide, melting at 175-176°C.

307. 3-bromomethyl-N-(2,6-diisopropylphenyl)-phthalimide, melting at 201-202°C.

308. 3-aminomethyl-N-(2,6-diisopropylphenyl)-phthalimide, melting at 134-137.5°C.

309. N-(2,6-diisopropylphenyl)-3-ethylaminomethylphthalimide, melting at 140-142°C.

310. 3-(2-cyanoethylaminomethyl)-N-(2,6-diisopropylphenyl)phthalimide, melting at 112-113°C.

311. N-(2,6-diisopropylphenyl)-3-(2,2-dimethoxyethylaminomethyl)phthalimide, melting at 111-112°C.

312. 3-cyclohexylaminomethyl-N-(2,6-diisopropylphenyl)-phthalimide, melting at 148-149°C.

313. 3-anilinomethyl-N-(2,6-diisopropylphenyl)-phthalimide, melting at 204-208°C.

314. N-(2,6-diisopropylphenyl)-3-(3-methylanilinomethyl)phthalimide, melting at 135-137°C.

315. N-(2,6-diisopropylphenyl)-3-ethoxycarbonylaminomethylphthalimide, melting at 121-123°C.

316. N-(2,6-diisopropylphenyl)-3-methoxyacetamidomethylphthalimide, melting at 167-168°C.

317. N-(2,6-diisopropylphenyl)-3-methanesulfonylaminomethylphthalimide, melting at 150-152°C.

318. N-(2,6-diethylphenyl)-3-(N'-methylureidomethyl)-phthalimide, melting at 135 - 136°C.

319. N-(2,6-diisopropylphenyl)-3-(1-imidazolylmethyl)-phthalimide, melting at 216-218°C.

320. N-(2,6-diisopropylphenyl)-3-(1H-1,2,4-triazolyl-methyl)phthalimide, melting at 221-223°C.

321. N-(2-ethyl-6-methylphenyl)-3-formylphthalimide, melting at 88-90°C.

322. N-(2,6-diethylphenyl)-3-formylphthalimide, a vitreous solid.

323. N-(2,6-diisopropylphenyl)-3-formylphthalimide, melting at 158.5 - 159.5°C.

324. N-(2-ethyl-6-methallylphenyl)-3-formylphthalimide.

325. N-(2,6-diethylphenyl)-3-hydroxyiminomethyl-phthalimide, melting at 174-175°C.

326. N-(2,6-diisopropylphenyl)-3-hydroxyiminomethyl-phthalimide, melting at 232-234°C.

327. N-(2,6-diisopropylphenyl)-3-dimethoxymethyl-phthalimide, melting at 154-157°C.

328. N-(2,6-diisopropylphenyl)-3-(1,3-dioxolan-2-yl)-phthalimide, melting at 189.5 - 190.5°C.

329. 3-carboxymethyl-N-(2,6-diisopropylphenyl)-phthalimide, melting at 231-233°C.

330. 3-(2-carboxyethyl)-N-(2,6-diisopropylphenyl)-phthalimide, melting at 183-185°C.

331. N-(2,6-diisopropylphenyl)-3-methoxycarbonyl-methylphthalimide.

332. N-(2,6-diisopropylphenyl)-3-ethoxycarbonylmethyl-phthalimide melting at 160-162°C.

333. N-(2,6-diisopropylphenyl)-3-(ethylthio)carbonyl-methylphthalimide.

334. N-(2,6-diisopropylphenyl)-3-propoxycarbonyl-methylphthalimide.

335. N-(2,6-diisopropylphenyl)-3-isobutoxycarbonyl-methylphthalimide.

336. N-(2,6-diisopropylphenyl)-3-heptyloxycarbonyl-methylphthalimide.

337. N-(2,6-diisopropylphenyl)-3-(2-methoxycarbonylethyl)phthalimide.

338. N-(2,6-diisopropylphenyl)-3-(2-ethoxycarbonylethyl)phthalimide, melting at 133-134.5.

339. 3-(2-ethoxycarbonylethyl)-N-(2-ethyl-6-methallylphenyl)phthalimide.

340. N-(2,6-diisopropylphenyl)-3-[(2-ethylthio)-carbonylethyl]phthalimide.

341. N-(2,6-diisopropylphenyl)-3-(2-propoxycarbonylethyl)phthalimide.

342. 3-(2-butoxycarbonylethyl)-N-(2,6-diisopropylphenyl)phthalimide.

343. N-(2,6-diisopropylphenyl)-3-(2-heptyloxycarbonylethyl)phthalimide.

344. 3-cyanomethyl-N-(2-ethyl-6-isopropylphenyl)phthalimide.

345. N-(2-sec-butyl-6-ethylphenyl)-3-cyanomethylphthalimide, melting at 153-154°C.

346. 3-cyanomethyl-N-(2,6-diisopropylphenyl)-phthalimide, melting at 206-209°C.

347. N-(2-allyl-6-methallylphenyl)-3-cyanomethylphthalimide.

348. 3-carbamoylmethyl-N-(2,6-diisopropylphenyl)-phthalimide, melting at 212-213.5°C.

349. 3-(2-carbamoylethyl)-N-(2,6-diisopropylphenyl)-phthalimide.

350. 3-(2,2-diacetylethyl)-N-(2,6-diisopropylphenyl)-phthalimide, melting at 146-149°C.

351. N-(2,6-diisopropylphenyl)-3-(1-hydroxyethyl)-phthalimide, melting at 166-167°C.

352. N-(2,6-diisopropylphenyl)-3-(1-hydroxypropyl)-phthalimide, melting at 97-99°C.

353. 3-(2-acetyl-1-hydroxyethyl)-N-(2,6-diisopropylphenyl)phthalimide, melting at 154.5-158.5°C.

354. N-(2,6-diisopropylphenyl)-3-(1-hydroxy-2-nitroethyl)phthalimide, melting at 172-176°C.

355. 3-acetyl-N-(2,6-diisopropylphenyl)phthalimide, melting at 149-149.5°C.

356. 3-butyryl-N-(2,6-diisopropylphenyl)phthalimide, melting at 124-125°C.

357. N-(2,6-diisopropylphenyl)-3-trifluoroacetylphthalimide.

358. 3-[2,2-bis(ethoxycarbonyl)acetyl]-N-(2,6-diisopropylphenyl)phthalimide, melting at 114-122.5°C.

359 3-[2,2-bis(ethoxycarbonyl)pent-4-enoyl]-N-(2,6-diisopropylphenyl)phthalimide, melting at 106.5-107.5°C.

360. N-(2,6-diisopropylphenyl)-3-vinylphthalimide, melting at 60-61°C.

361. N-(2,6-diisopropylphenyl)-3-(1-propenyl)phthalimide, melting at 166-168°C.

362. 3-(1-butenyl)-N-(2,6-diisopropylphenyl)phthalimide, melting at 159-162°C.

363. 3-(2-acetylvinyl)-N-(2,6-diisopropylphenyl)phthalimide, melting at 187-188.5°C.

364. N-(2-sec-butyl-6-ethylphenyl)-3-(2-carboxyvinyl)-phthalimide.

365. 3-(2-carboxyvinyl)-N-(2,6-diisopropylphenyl)-phthalimide, melting at 255.5-256.5°C.

366. N-(2,6-diisopropylphenyl)-3-(2-ethoxycarbonylvinyl)phthalimide, melting at 131.5-132.5°C.

367. 3-(2-carboxyprop-1-enyl)-N-(2,6-diisopropylphenyl)phthalimide, melting at 259-260°C.

368. N-(2,6-diisopropylphenyl)-3-oxiranylphthalimide, melting at 84-86°C.

369. N-(2,6-diisopropylphenyl)-3-dimethoxyphosphoryl-methylphthalimide, melting at 133-134°C.

370. 3-diethoxyphosphorylthiomethyl-N-(2,6-diisopropylphenyl)phthalimide, melting at 80-82°C.

371. N-(2,6-diisopropylphenyl)-3-mercaptomethylphthal-imide.

372. N-(2,6-diisopropylphenyl)-3-ethylthiomethyl-phthalimide, melting at 154-155°C.

373. 3-allylthiomethyl-N-(2,6-diisopropylphenyl)phthalimide, melting at 142-143°C.

374. N-(2,6-diisopropylphenyl)-3-(2-propynylthiomethyl)phthalimide, melting at 124-127°C.

375. N-(2,6-diisopropylphenyl)-3-(N-hydroxyamidino)-phthalimide, melting at 233-234°C.

376. N-(2,6-diisopropylphenyl)-3-(N-ethoxycarbonyloxy-amidino)phthalimide, melting at 111-112°C.

377. N-(2,6-diethylphenyl)-3-nitrophthalimide, melting at 133-134°C.

378. N-(2-sec-butyl-6-ethylphenyl)-3-nitrophthalimide, melting at 173-174°C.

379. N-(2,6-diisopropylphenyl)-3-nitrophthalimide, melting at 168-169°C.

380. 3-amino-N-(2,6-diethylphenyl)phthalimide, melting at 176-177°C.

381. 3-amino-N-(2-sec-butyl-6-ethylphenyl)phthalimide, melting at 196-197°C.

382. 3-amino-N-(2,6-diisopropylphenyl)phthalimide, melting at 266-267°C.

383. 3-acetamido-N-(2,6-diethylphenyl)phthalimide, melting at 65-66°C.

384. 3-acetamido-N-(2-sec-butyl-6-ethylphenyl)-phthalimide, melting at 83-85°C.

385. 3-acetamido-N-(2,6-diisopropylphenyl)phthalimide, melting at 237-238°C.

386. N-(2,6-diethylphenyl)-3-(2-methoxyacetamido)-phthalimide, melting at 121-122°C.

387. N-(2-sec-butyl-6-ethylphenyl)-3-(2-methoxy-acetamido)phthalimide, $n_D^{23}$ = 1.5637.

388. 3-diacetylamino-N-(2,6-diisopropylphenyl)phthalimide, melting at 234-235°C.

389. N-(2-sec-butyl-6-ethylphenyl)-3-(2-oxazolinyl)-phthalimide, melting at 62-63°C.

390. N-(2,6-diisopropylphenyl)-3-(2-oxazolinyl)-phthalimide, melting at 190-191°C.

391. N-(2,6-diisopropylphenyl)-3-(4,5-dihydro-1,3-oxazin-2-yl)phthalimide, melting at 158-161°C.

392. N-(2-sec-butyl-6-ethylphenyl)-3-(1,3,4-oxadiazol-2-yl)phthalimide.

393. N-(2,6-diisopropylphenyl)-3-(1,3,4-oxadiazol-2-yl)phthalimide, melting at 141-142°C.

394. N-(2,6-diisopropylphenyl)-3-(2-methyl-1,3,4-oxadiazol-5-yl)phthalimide, melting at 213-214°C.

395. N-(2,6-diisopropylphenyl)-3-(5-oxo-1,2,4-oxadiazol-3-yl)phthalimide, melting at 220-221°C.

396. N-(2,6-diisopropylphenyl)-3-(1H-tetrazol-5-yl)phthalimide, melting at 145-146°C.

Of the compounds listed above, the preferred compounds are Nos. 9, 11, 65, 75, 77, 78, 81, 82, 83, 84, 85, 86, 153, 278, 279, 280, 295, 323, 329, 332, 338, 346, 365 and 393.

The compounds of the invention may be prepared by the methods described below.

Method A

Compounds of the invention in which $R^4$ represents a carboxy, thiocarboxy (preferably mercaptocarbonyl) or nitro group, that is to say compounds of formula (Ia) and their nitro analogs, may be prepared by the method illustrated in the following reaction scheme:

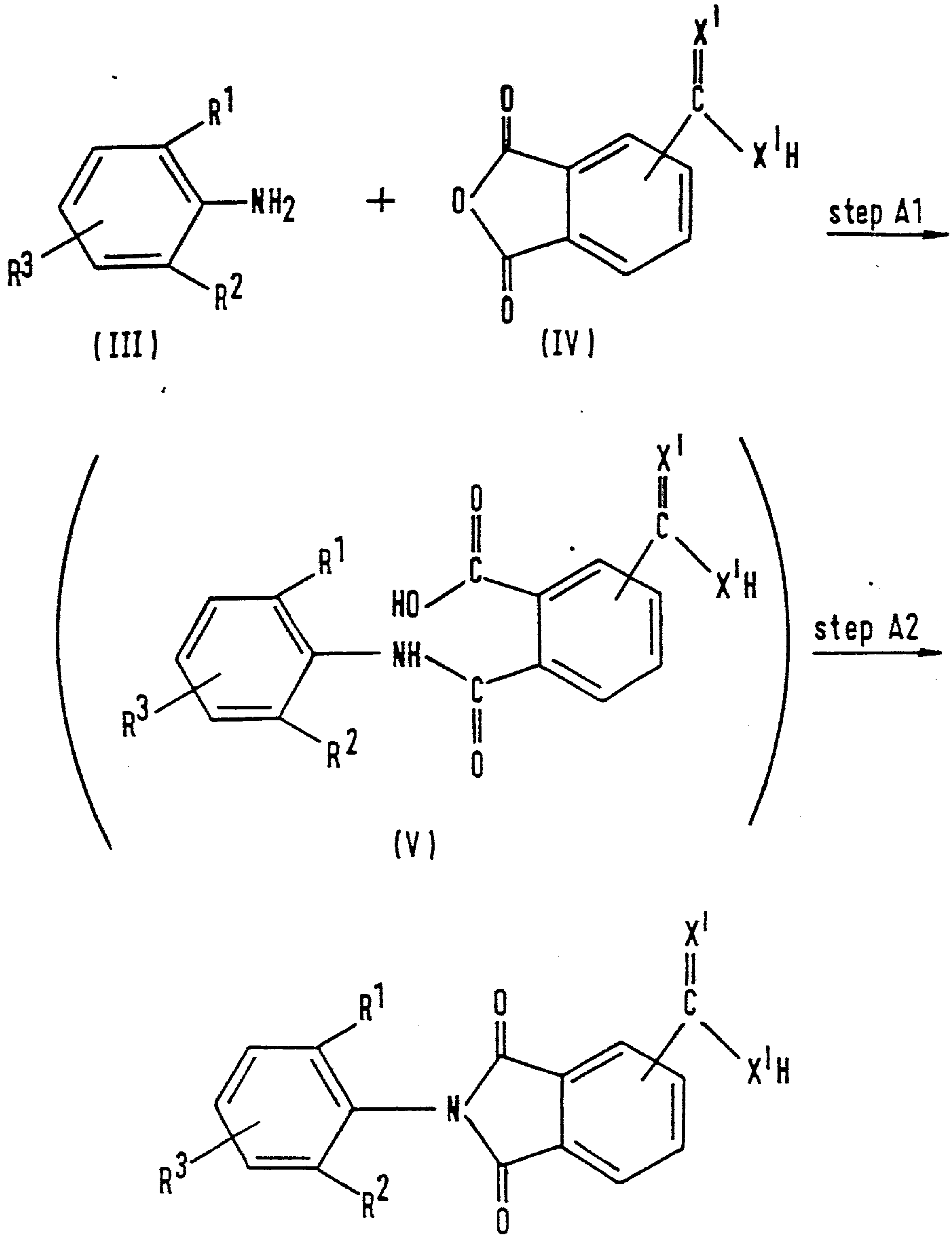


(III) (IV)

(V)

(Ia)

In the formulae, $R^1$, $R^2$, $R^3$ and X' are as defined above.

In step A1 of this reaction scheme, a phthalic anhydride derivative of formula (IV) is reacted with an aniline derivative of formula (III). The reaction may take place in the presence or absence of a solvent. Where a solvent is employed, its nature is not critical, provided that it has no adverse effect upon the reaction. Suitable solvents include: aromatic hydrocarbons, such as toluene or xylene; aprotic polar solvents, such as dimethylformamide or dimethyl sulfoxide; ketones, such as methyl butyl ketone, methyl isobutyl ketone or cyclohexanone; butanol; and acetic acid.

The reaction is preferably effected with heating. Since water is produced by this reaction, we prefer to employ an inert solvent which can produce an azeotropic mixture with water and to carry out the reaction at the reflux temperature of this azeotropic mixture.

Normally and preferably, step A2 of the reaction will take place immediately after step A1 and under the same conditions, without intermediate isolation of the intermediate of formula (V). However, if it is for any reason desired, the intermediate (V) can be isolated and

will normally be obtained in a quantitative yield. For example, in the reaction of the compound of formula (IV) in which both atoms represented by X' are oxygen and the carboxy group is at the 3-position with 2,6-diethylaniline or 2,6-diisopropylaniline, the resulting intermediate of formula (V) has a melting point of 243.5-244°C or 191-193°C, respectively.

Alternatively, instead of using the phthalic acid anhydride derivative of formula (IV), it is possible to use the phthalic acid corresponding to compound (IV) itself or any other active derivative thereof, for example, the acid halides (e.g. chloride or bromide), active amides or active esters (e.g. the *p*-nitrobenzyl ester).

Method B

Compounds of formula (Ib), that is to say compounds of formula (I) in which $R^4$ represents a group of formula $-CX".R^{10}$:

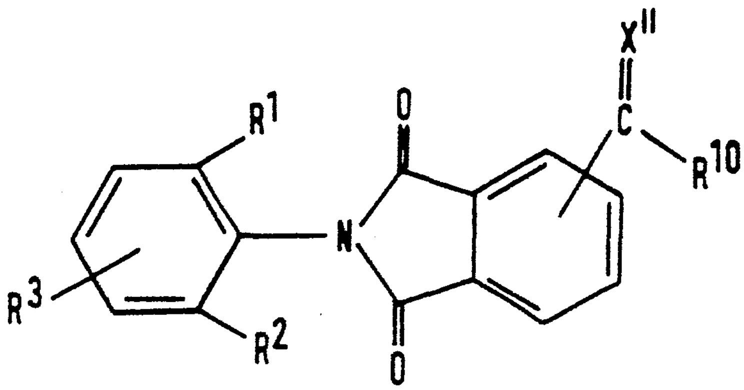

(Ib)

(in which $R^1$, $R^2$, $R^3$, $R^{10}$ and X" are as defined above) may be prepared by reacting the corresponding compound of formula (Ia) with a halogenating agent.

The nature of the halogenating agent is not critical and any halogenating agent commonly used to prepare an acid halide from its carboxylic acid may be used in this process. Suitable halogenating agents include, for example, thionyl chloride and thionyl bromide, but any other conventional halogenating agent may be used.

The conditions under which the reaction takes place are likewise conventional. The reaction is preferably

effected in the presence of a solvent, the nature of which is not critical, provided that it has no adverse effect upon the reaction. Suitable solvents include hydrocarbons, which may be aliphatic, alicyclic or aromatic, for example hexane, heptane, benzene, toluene, xylene or cyclohexane. The reaction temperature is not critical and the reaction will take place over a wide range of temperatures. However, we normally prefer to carry out the reaction at the reflux temperature of the reaction mixture.

Method C

Esters and amides of the carboxylic or thiocarboxylic acids of formula (Ia), that is to say compounds of formula (Ic) and (Ic'), may be prepared from the corresponding acid halide of formulae (Ib) as illustrated by the following reactions:

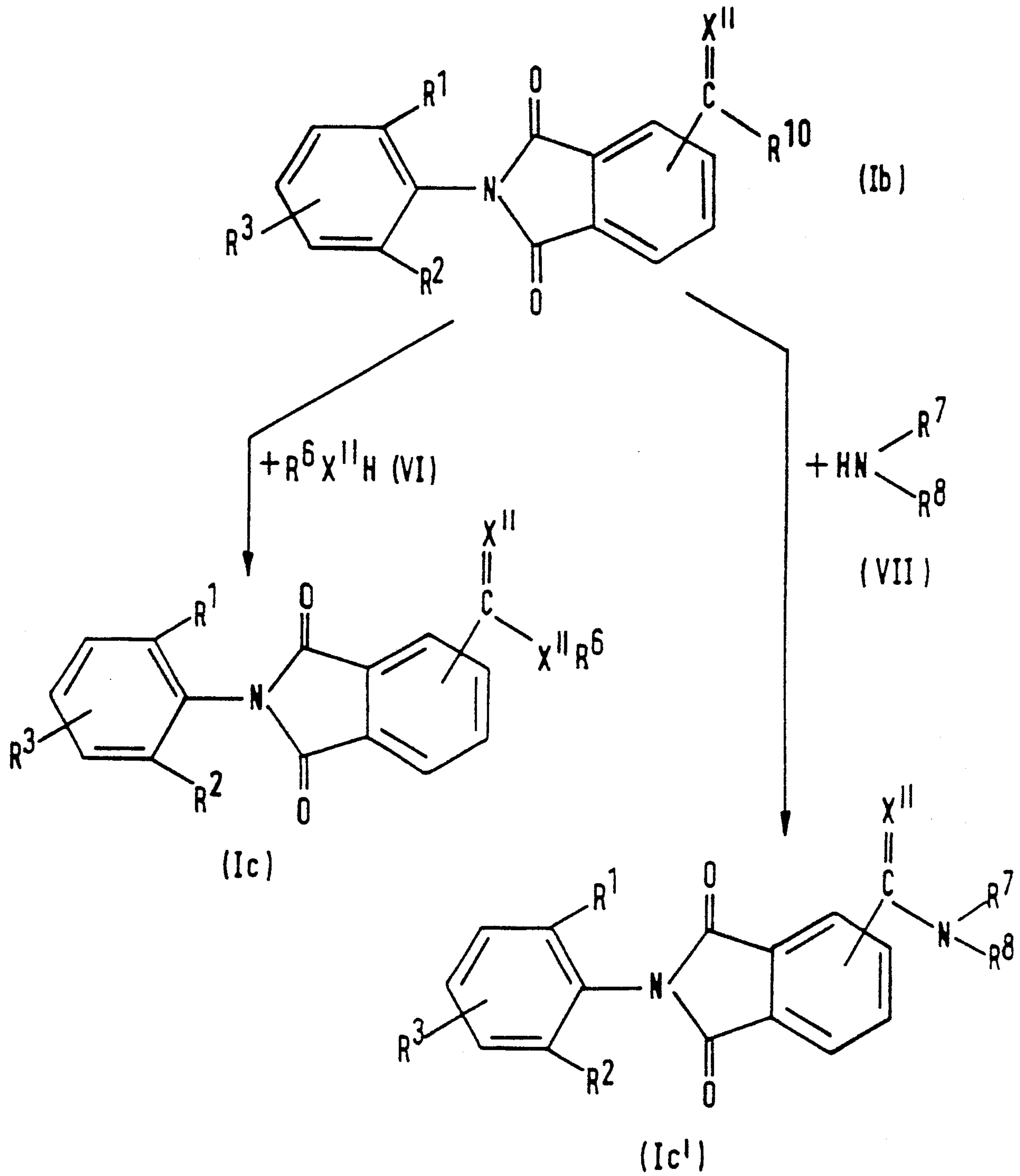


(Ib)

(Ic)

(Ic')

In the above formulae, $R^1$, $R^2$, $R^3$, $R^6$, $R^7$, $R^8$, $R^{10}$ and X" are as defined above.

The compounds of formulae (Ic) and (Ic') are prepared by reacting the compound of formula (Ib) with the compounds of formulae (VI) and (VII), respectively. The two reactions are carried out under essentially the same conditions and may therefore be described together.

Both reactions are preferably carried out in the presence of an acid-binding agent and preferably also in the presence of a solvent. The acid-binding agent serves to remove the acid liberated by the reaction from the reaction mixture and will therefore normally be a base. Examples of suitable acid-binding agents include: tertiary straight chain alkylamines, such as triethylamine or tributylamine; tertiary cycloalkylamines, such as 1,5-diazabicyclo[5.4.0]undecene-5; pyridine; and alkali metal carbonates, such as sodium carbonate or potassium carbonate. The solvent employed is not critical to the invention, provided that it has no adverse effect upon the reaction. Suitable solvents include: aromatic hydrocarbons, such as benzene or toluene; halogenated hydrocarbons, such as chloroform or methylene chloride; ethers, such as diisopropyl ether, diethyl ether, tetrahydrofuran or dioxane; nitriles, such as acetonitrile or propionitrile; ketones, such as acetone; and mixtures of any two or more thereof.

Method D

Compounds of formula (I) in which $R^4$ represents a cyano group, that is to say compounds of formula (Id):

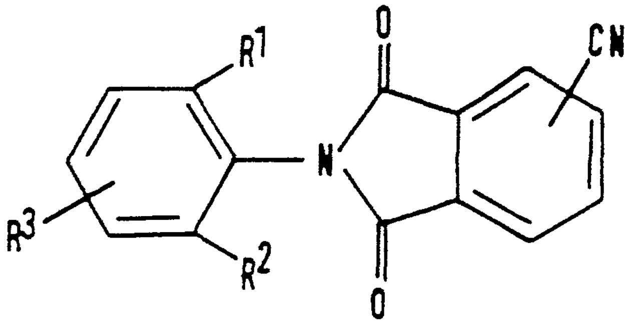

(Id)

may be prepared by reacting a compound of formula (VIII):

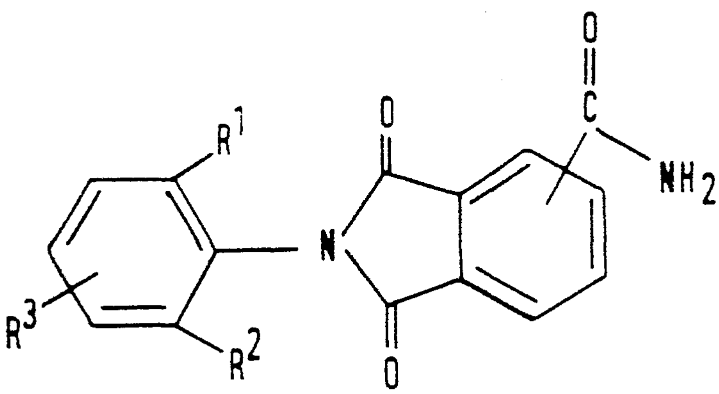

(VIII)

(in both of the above formulae, $R^1$, $R^2$ and $R^3$ are as defined above) with a dehydrating agent by conventional means. The dehydrating agent employed is not critical to the reaction and any dehydrating agent

conventionally employed for converting an acid amide to the corresponding nitrile may be employed in the present process, provided that it does not affect, or does not affect to a substantial extent, any other part of the molecule. Suitable dehydrating agents include phosphorus pentachloride, thionyl chloride and phosphorus oxychloride, of which phosphorus oxychloride is preferred. The reaction is preferably effected in the presence of a solvent, the nature of which is not critical, provided that it has no adverse effect upon the reaction. Suitable solvents include aliphatic, alicyclic and aromatic hydrocarbons, such as hexane, heptane, benzene, toluene, xylene and cyclohexane, and halogenated hydrocarbons, such as chloroform, methylene chloride and ethylene dichloride. The reaction temperature may vary over a wide range but, for convenience, we normally prefer to carry the reaction under reflux.

The starting material employed for Method D, the compound of formula (VIII), is a compound of formula (Ic') in which $R^7$ and $R^8$ both represent hydrogen atoms and X" represents an oxygen atom, and this compound may have been prepared as described in Method C.

METHOD E

Compounds of formula (Ie), (Ie') and (Ie"), that is to say compounds of formula (I) in which $R^4$ represents a hydroxymethyl, halomethyl, alkoxymethyl, cycloalkoxymethyl, aryloxymethyl, alkenyloxymethyl or alkynyloxymethyl group can be prepared as illustrated in the following reaction scheme:

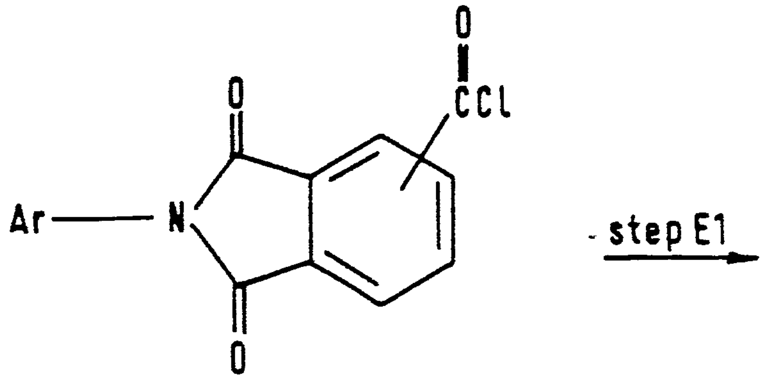


(IX)

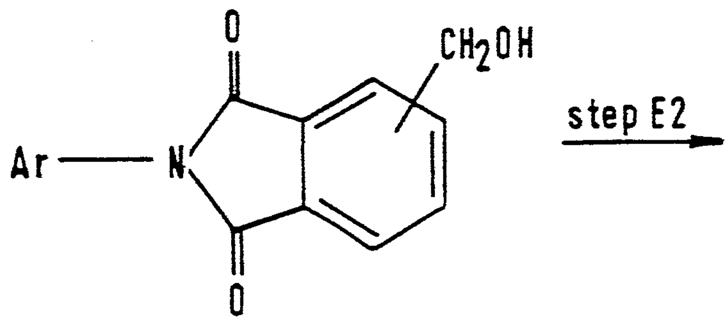


(Ie)

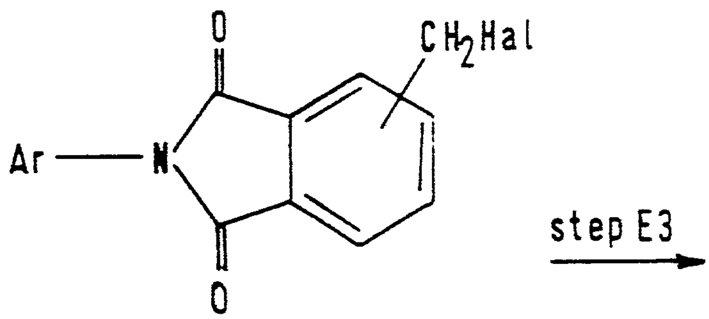


(Ie')

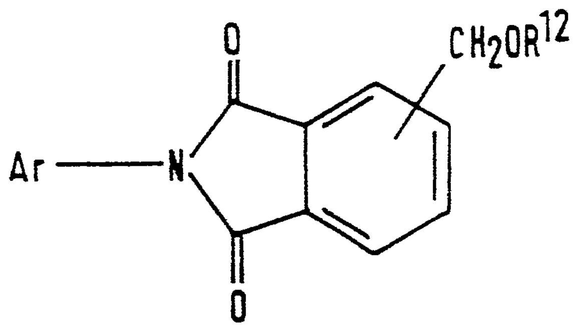

(Ie'')

In the above formulae:

Ar represents a group of formula

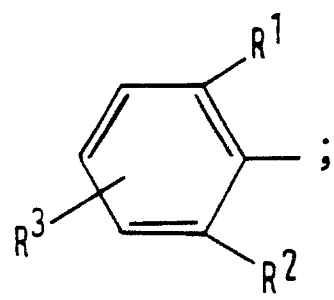

Hal represents a halogen atom, for example a fluorine, chlorine, bromine or iodine atom, preferably a chlorine or bromine atom; and

$R^{12}$ represents any one of the cycloalkyl, aryl, alkenyl, alkynyl, alkyl or substituted alkyl groups represented by $R^5$.

The starting material for this reaction scheme, the compound of formula (IX) is a compound of formula (Ib) in which X" represents an oxygen atom and $R^{10}$ represents a chlorine atom, and may, if desired, be prepared by the process described in Method B.

In step E1 of this reaction scheme, the chloroformyl group of the compound of formula (IX) is reduced to a hydroxymethyl group; any reducing agent known in the art for this reaction may be employed, but we prefer to

employ a complex metal hydride. Examples of such complex metal hydrides include lithium aluminum hydride, lithium borohydride, bis(2-methoxyethoxy)aluminum sodium hydride and sodium borohydride. The reaction is preferably effected in the presence of a solvent, the nature of which is not critical, provided that it has no adverse effect upon the reaction. Examples of suitable solvents include: ethers, such as tetrahydrofuran, diethyl ether or diglyme; and aromatic hydrocarbons, such as benzene or toluene. The temperature at which the reaction is effected is likewise not particularly critical, although high temperatures are normally best avoided in order to reduce the possibility of side reactions. Accordingly, we generally prefer to carry out the reaction at the temperature achieved by cooling with ice or at room temperature.

If desired, the resulting compound of formula (Ie) may be converted, in step E2, to the corresponding halomethyl derivative of formula (Ie'). This is achieved by reacting the compound of formula (Ie) with a halogenating agent, for example thionyl chloride, thionyl bromide or phosphorus tribromide. The reaction is preferably effected in the presence of a solvent, the nature of which is not critical, provided that it has no adverse effect upon the reaction. Suitable solvents include:hydrocarbons, which may be aliphatic, alicyclic

or aromatic, for example hexane, heptane, benzene, toluene, xylene or cyclohexane; and ethers, such as diethyl ether, tetrahydrofuran or dioxane. The reaction temperature is not critical and the reaction will take place over a wide range of temperatures. However, we normally prefer to carry out the reaction at room temperature or at the reflux temperature of the reaction mixture.

If desired, the resulting compound of formula (Ie') may be converted to the corresponding cycloalkoxymethyl, aryloxymethyl, alkenyloxymethyl, alkynyloxymethyl, alkoxymethyl or substituted alkoxymethyl derivative of formula (Ie"). This may be achieved by reacting the compound of formula (Ie') with an alcohol of formula $R^{12}OH$ ($R^{12}$ being as defined above), which is preferably employed in the form of the corresponding metal alkoxide (more preferably alkali metal alkoxide, e.g. sodium alkoxide), normally with heating. The reaction is preferably effected in a solvent, but the solvent is most preferably an excess of the alcohol $R^{12}OH$, particularly where that is a lower alcohol.

<u>Method F</u>

Compounds of formula (Ie) may also be converted to the corresponding esters by reacting the compound of

formula (Ie) with a suitable reactive derivative of the desired acid, for example an anhydride, mixed anhydride, active ester or acid halide, preferably the acid halide and most preferably the chloride or bromide. Where the acid halide is employed, the reaction is preferably effected in the presence of an acid-binding agent, whose function is to remove from effective participation in the reaction mixture the hydrogen halide produced by the reaction. Any acid-binding agent capable of doing this may be employed, preferred examples being: tertiary amines, such as triethylamine, tributylamine, 1,5-diazabicyclo[5.4.0]undec-5-ene or pyridine; or an alkali metal carbonate or bicarbonate, such as sodium carbonate, potassium carbonate, sodium bicarbonate or potassium bicarbonate. The reaction is preferably effected in the presence of a solvent, the nature of which is not critical, provided that it has no adverse effect upon the reaction. Suitable solvents include: aromatic hydrocarbons, such as benzene or toluene; halogenated hydrocarbons, such as chloroform or methylene chloride; ethers, such as diisopropyl ether, diethyl ether, tetrahydrofuran or dioxane; nitriles, such as acetonitrile or propionitrile; or ketones, such as acetone. The reaction may be effected over a very wide temperature range, for example from the temperature achieved by cooling with ice to the reflux temperature of the reaction mixture.

Method G

Compounds of formula (I) in which $R^4$ represents a methyl group having a di($C_1$-$C_3$ alkoxy)phosphorylthio group may be prepared by reacting a compound of formula (Ie') with a metal compound of formula:

$$M - S - R^{9a}$$

in which:

M represents an alkali metal, especially sodium or potassium; and

$R^{9a}$ represents any of the dialkoxyphosphoryl groups heretofore defined for $R^9$.

The reaction is preferably effected in a solvent, the nature of which is not critical, provided that it has no adverse effect on the reaction. Suitable solvents include: ethers such as tetrahydrofuran or dioxane; nitriles, such as acetonitrile; and ketones, such as acetone.

The reaction temperature is likewise not critical and, for convenience, about ambient temperature is preferred.

Method H

Compounds of formula (I) in which $R^4$ represents the aminomethyl or various substituted aminomethyl groups, that is to say compounds of formula (Ih), (Ih'), (Ih"), (Ih''') or (Ih''''), can be prepared as illustrated in the following reaction scheme:

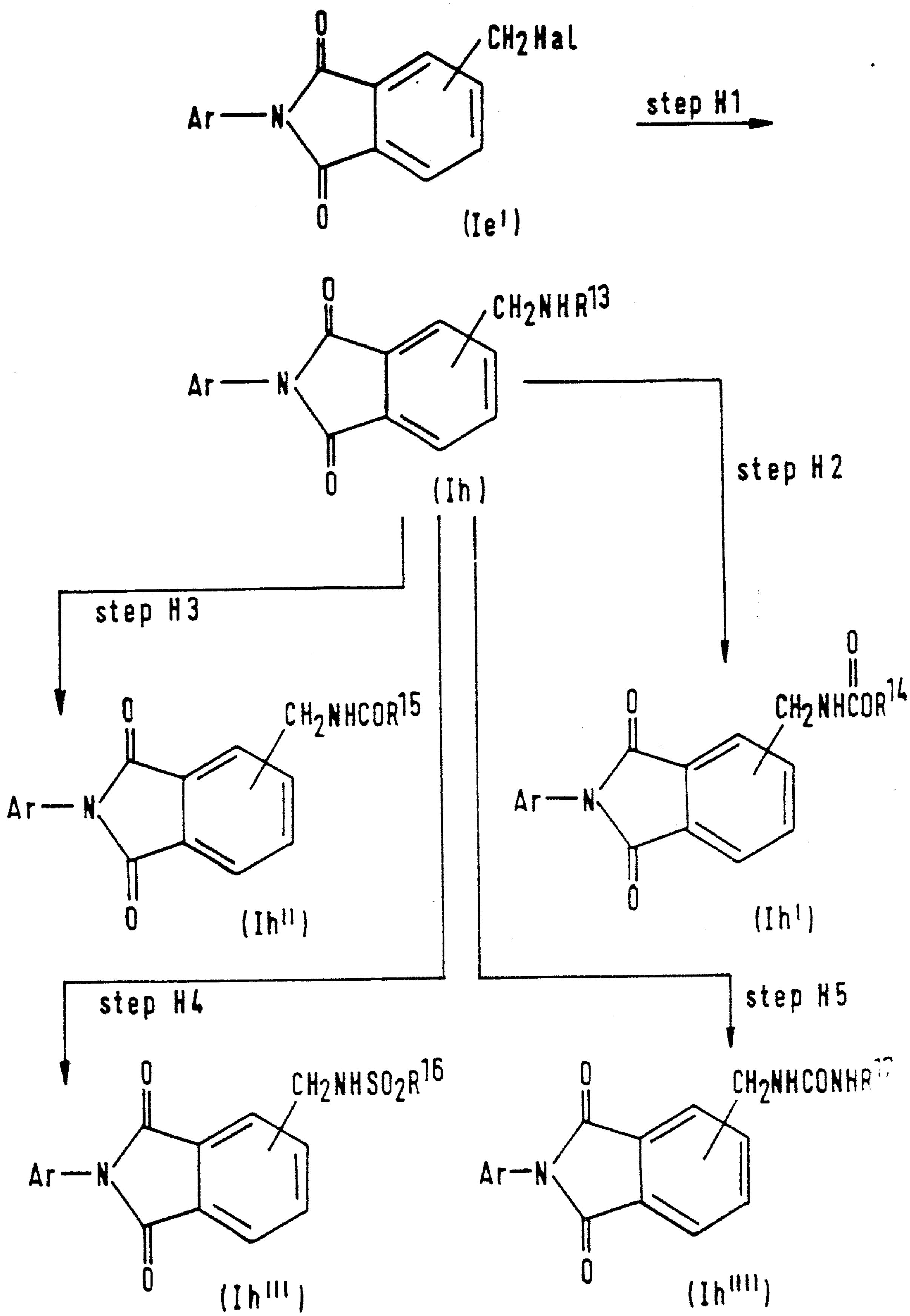


(Ie')

(Ih)

(Ih'')

(Ih')

(Ih''')

(Ih'''')

In the above formulae, Ar is as defined above; $R^{13}$ represents hydrogen or the various alkyl, substituted alkyl, cycloalkyl or aryl groups defined for $R^5$; $R^{14}$ represents hydrogen or the alcoholic moiety of an ester; $R^{15}$ represents the remainder of a carboxylic acyl group, i.e. $-CO.R^{15}$ represents any one of the carboxylic acyl groups heretofore defined for $R^9$; $R^{16}$ represents the remainder of a sulfonic acyl group, i.e. $-SO_2.R^{16}$ represents any one of the sulfonic acyl groups heretofore defined for $R^9$; and $R^{17}$ represents any one of the groups or atoms heretofore defined for $R^7$ or $R^8$.

In step H1 of this reaction scheme, the halomethyl compound of formula (Ie') is reacted with a primary amine of formula $R^{13}NH_2$, in the presence of an acid-binding agent, for example any one of those acid-binding agents listed in Method F. There is no particular limitation on the proportions of the compound of formula (Ie') to the primary amine, but, in general, we prefer to employ them in a molar ratio of primary amine to compound (Ie') of from 1:1 to 3:1.

The reaction is preferably effected in the presence of a solvent, the nature of which is not critical, provided that it has no adverse effect upon the reaction. Suitable solvents include, for example:

aromatic hydrocarbons, such as benzene or toluene; halohydrocarbons, preferably aliphatic halohydrocarbons, such as chloroform or methylene chloride; and ethers, such as tetrahydrofuran or dioxane. The reaction will take place over a wide temperature range, but we generally prefer to effect the reaction at a temperature within the range from room temperature to 80°C.

Steps H2, H3 and H4 may be effected by reacting the compound of formula (Ih) obtained in step H1 with, respectively, a compound of formula Hal.COOR$^{14}$, $R^{15}$COHal or $R^{16}SO_2$Hal. The reaction is preferably effected in the presence of an acid-binding agent and in a suitable solvent, at room temperature or with heating. Examples of solvents and acid-binding agents are as given in Method F. The reaction temperature is preferably from room temperature to 50°C.

In step H5, a compound of formula (Ih'''') may be prepared by reacting the compound of formula (Ih) with an appropriate isocyanate of formula $R^{17}NCO$. The reaction is effected in the presence of a suitable solvent and of a catalytic amount of a base, preferably pyridine, and is normally effected at room temperature. Solvents which may be employed are as described in Method F above.

Method J

Compounds of formula (X):

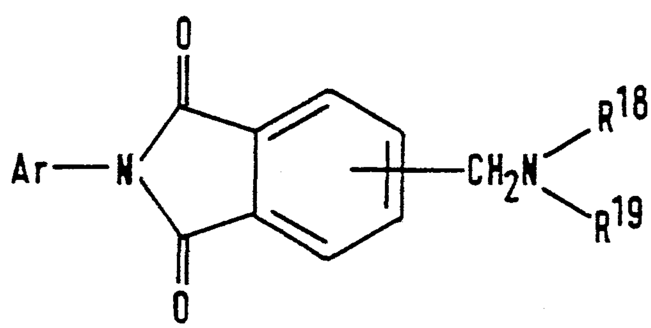

(X)

(in which: Ar is as defined above; and $R^{18}$ and $R^{19}$, together with the nitrogen atom to which they are attached, represent a nitrogen-containing heterocyclic

group, for example any one of those groups heretofore defined for the heterocyclic substituent on the substituted alkyl group represented by $R^4$) may be prepared by reacting a compound of formula (Ie') as defined in Methods E and H, with a heterocyclic compound of formula $HNR^{18}R^{19}$ (in which $R^{18}$ and $R^{19}$ are as defined above). The reaction is preferably effected in the presence of an acid-binding agent, which may be any one of those described in Method F above (preferably an alkali metal carbonate), and in the presence of a suitable solvent. The nature of the solvent is not critical, provided that it has no adverse effect upon the reaction. Suitable solvents include: ethers, such as dioxane; and nitriles, such as acetonitrile. The reaction is preferably effected at room temperature or with heating.

Method K

Compounds of formula (I) in which $R^4$ represents a formyl, hydroxyiminomethyl or cyclic acetal group or a methyl group having two alkoxy, alkenyloxy or alkynyloxy substituents, that is to say compounds of formulae (Ik), (Ik'), (Ik") and (Ik'''), may be prepared as illustrated in the following reaction scheme:

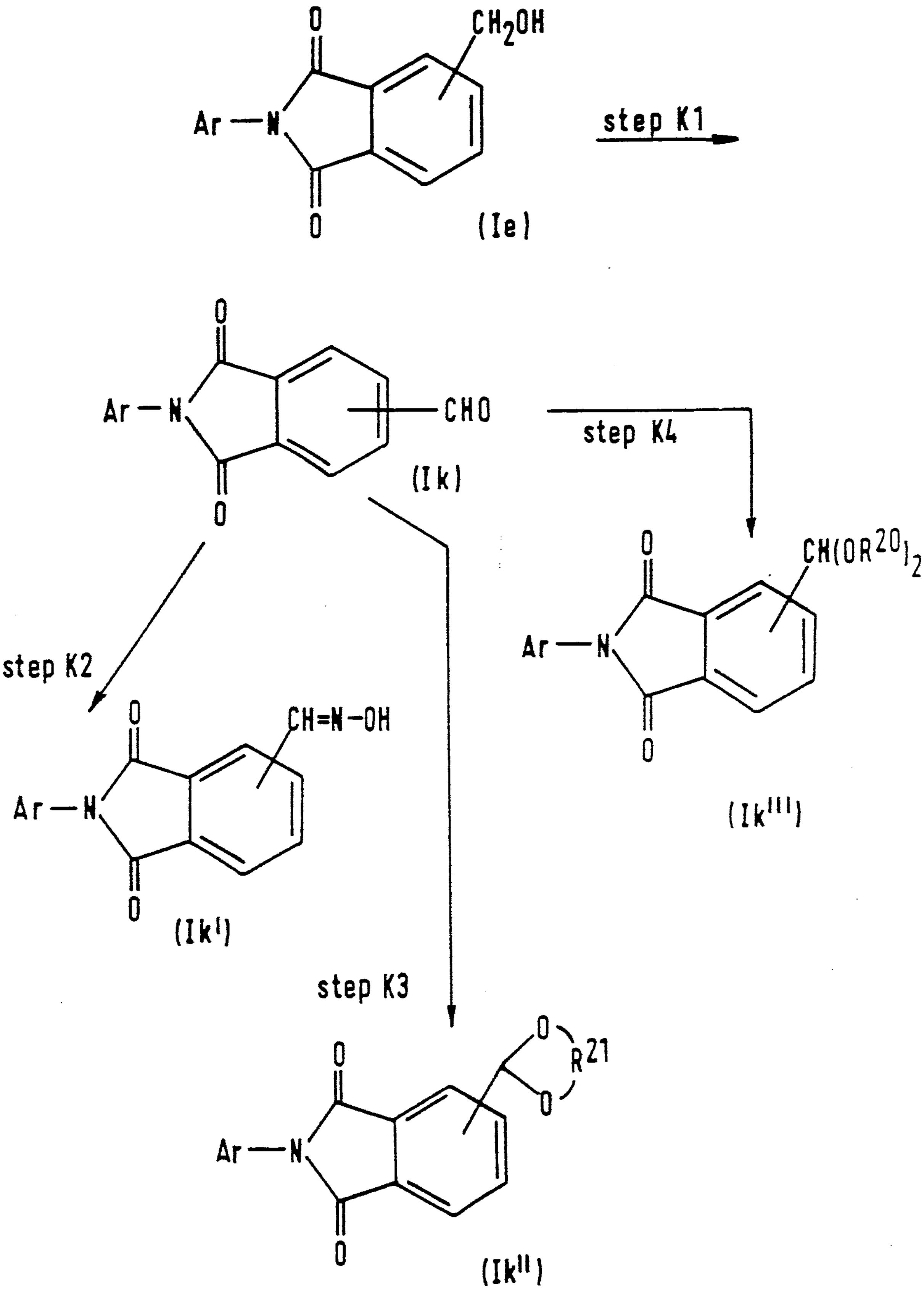


(Ie)

(Ik)

(Ik')

(Ik''')

(Ik'')

In the above formulae: Ar is as defined above; $R^{20}$ represents any one of the alkyl, alkenyl or alkynyl groups defined for $R^5$; and $R^{21}$ represents the residue of a heterocyclic group, as defined for $R^4$. Preferably, $R^{21}$ is an alkylene group, which may be branched.

In step K1, the compound of formula (Ie), which may have been prepared as described in Method E above, is reacted with an oxidising agent to give the corresponding formyl derivative of formula (Ik). Any oxidising agent capable of converting a hydroxymethyl group to a formyl group may be employed and examples include activated manganese dioxide and chromic anhydride, which are preferably used in amounts of from 1 to 6 moles per mole of said compound of formula (Ie). The reaction is preferably effected in the presence of a solvent, the nature of which is not critical, provided that it has no adverse effect upon the reaction. Suitable solvents include, for example: lower aliphatic acids, such as acetic acid; and halogenated hydrocarbons, such as chloroform, carbon tetrachloride or methylene chloride. The reaction is preferably effected at room temperature or with heating.

In step K2, the resulting compound of formula (Ik) is reacted with hydroxylamine in a suitable solvent.

The nature of the solvent is not critical, provided that it has no adverse effect upon the reaction and suitable solvents include alcohols, such as methanol or ethanol. The reaction is preferably effected at room temperature or with heating.

In step K3, the compound of formula (Ik) is reacted with an alkylene glycol (which may be branched), preferably in the presence of a catalytic amount of p-toluenesulfonic acid. The reaction is preferably effected in the presence of a solvent, the nature of which is not critical, provided that it has no adverse effect upon the reaction. Preferred solvents include aromatic hydrocarbons, such as benzene, toluene or the xylenes. The reaction is preferably performed with heating.

In step K4, the compound of formula (Ik) is reacted with a compound of formula $CH(OR^{20})_3$, preferably employing an excess of said compound of formula $CH(OR^{20})_3$. The reaction is preferably effected in the presence of a catalytic amount of p-toluenesulfonic acid and is also preferably effected at about room temperature. The reaction may be effected in the presence of a solvent, preferably an excess of the compound of formula $CH(OR^{20})_3$, which itself serves as reaction solvent.

<u>Method L</u>

Compounds in which $R^4$ represents an ethyl group having two carboxylic acyl substituents, that is to say compounds of formula (XI):

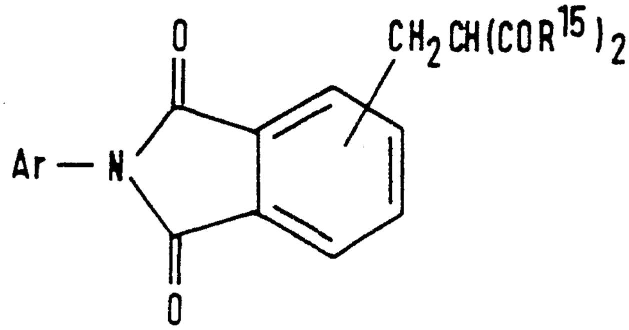

(XI)

(in which Ar is as defined above and $R^{15}$ is as defined above in relation to Method H) may be prepared by reacting a compound of formula (Ie'), as defined in Method E, with a compound of formula (XII):

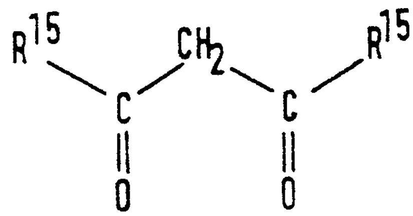

(XII)

(in which $R^{15}$ is as defined above). This reaction is preferably effected by heating the two reagents together in the presence of potassium carbonate and in acetone as the solvent, as described in Organic Synthesis, 42, 75 (1962).

Method M

Compounds of formula (Im), (Im') and (Im"), that is to say compounds of formula (I) in which $R^4$ represents various substituted alkyl groups, may be prepared as illustrated in the following reaction scheme:

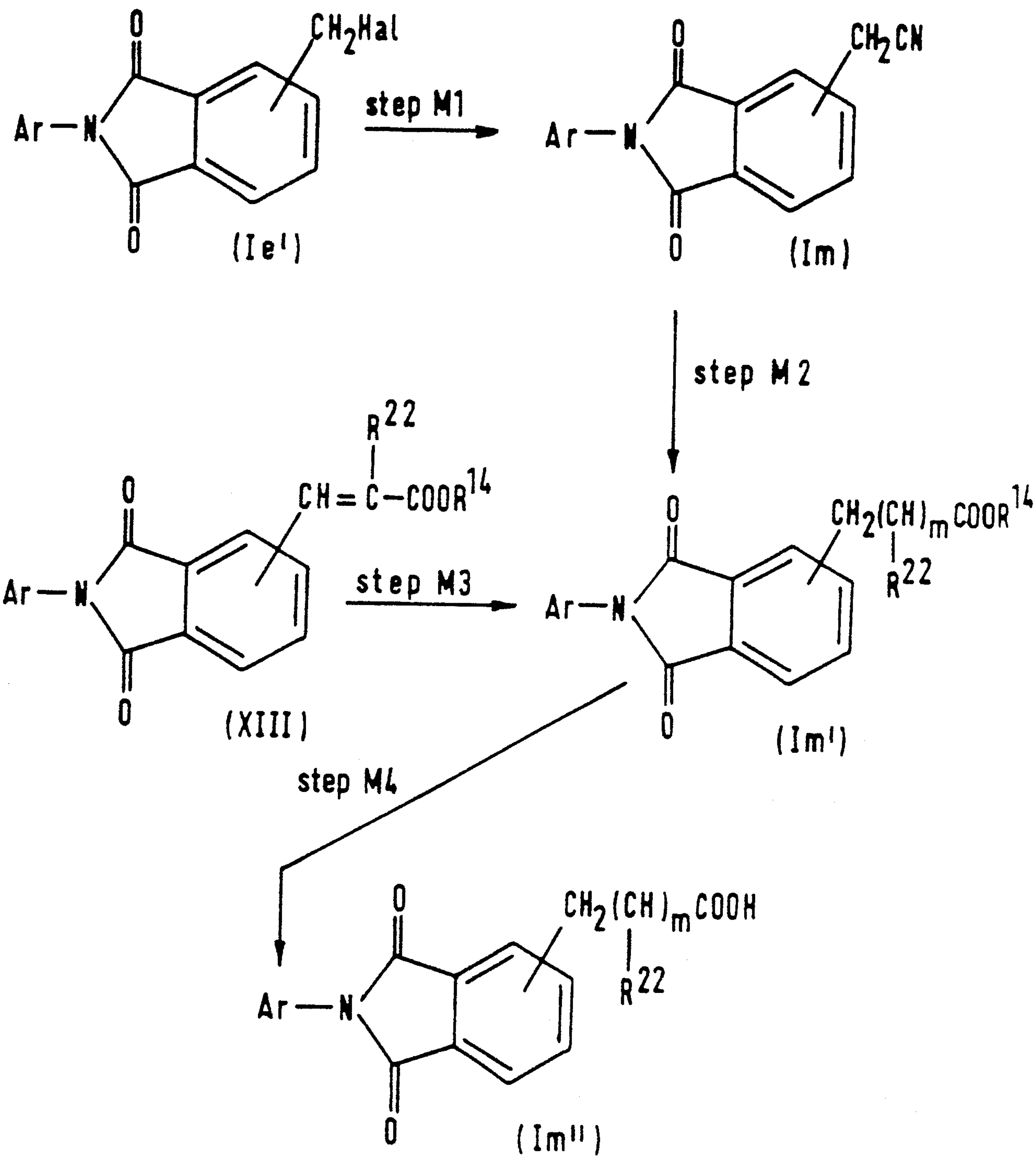


(Ie')
(Im)
(XIII)
(Im')
(Im'')

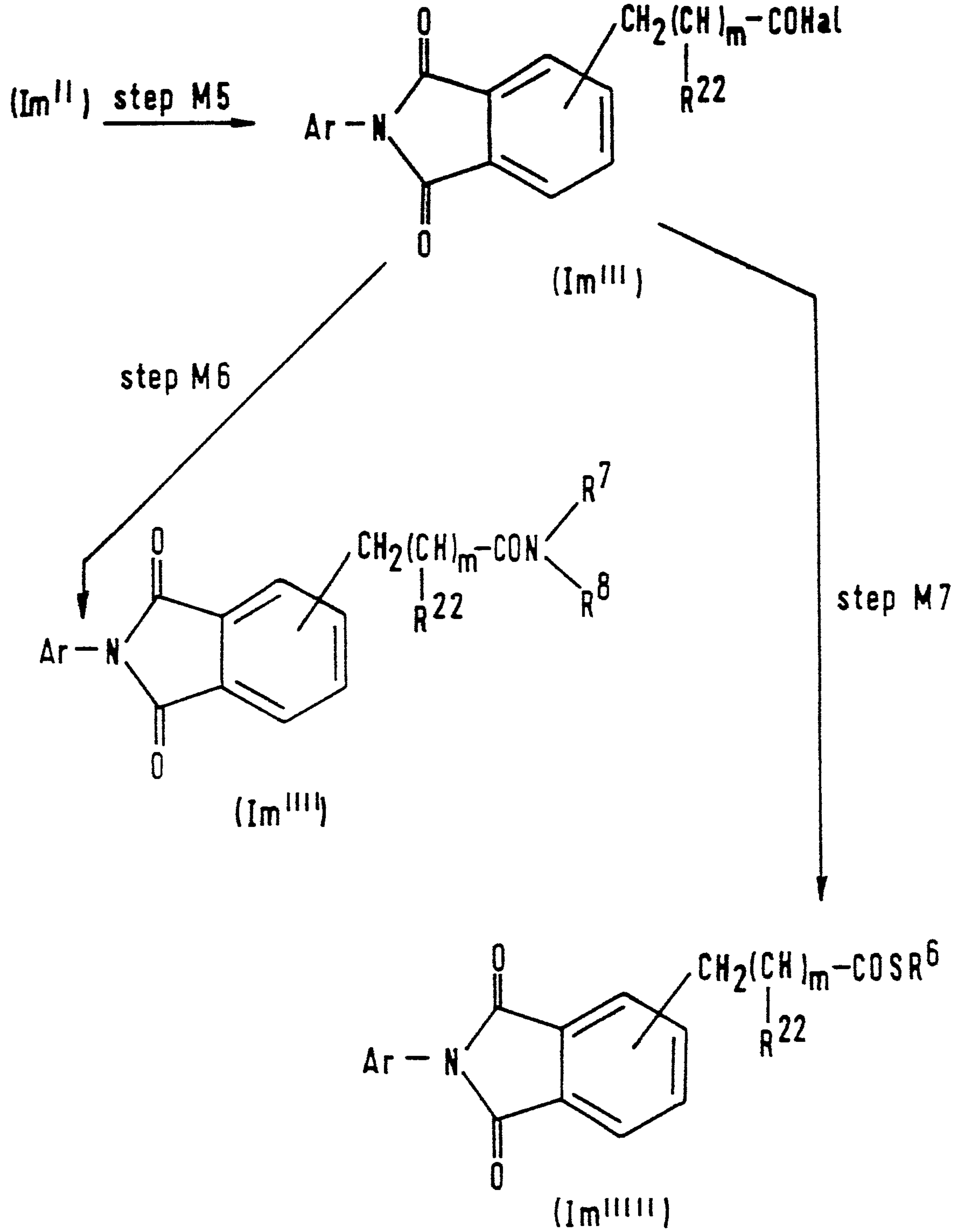


(Im''')

(Im'''')

(Im''''')

In the above formulae, $R^6$, $R^7$, $R^8$, Ar and Hal are as defined above; $R^{14}$ is also as defined above - see Method H; $R^{22}$ represents a hydrogen atom or a $C_1$-$C_4$ alkyl group; and m is 0 or 1.

In step M1 of this reaction, the compound of formula (Ie'), which may have been prepared as described in Method E above, is reacted with a cyanide, preferably an alkali metal cyanide (e.g. sodium cyanide or potassium cyanide) to give the desired compound of formula (Im). The reaction is preferably effected in the presence of a solvent, the nature of which is not critical, provided that it has no adverse effect upon the reaction. Suitable solvents include: alcohols, such as ethanol or tetrahydrofurfuryl alcohol; glycols, such as ethylene glycol; glycol ethers, such as ethylene glycol monomethyl ether; amides, such as dimethylformamide; or dimethyl sulfoxide. The reaction is preferably effected at room temperature or with heating.

If desired, this compound of formula (Im) may be converted to the corresponding acid or ester of formula (Im') in which m is 0 by heating in the presence of a strong acid, such as sulfuric acid. The reaction is preferably effected in the presence of a solvent, the nature of which is not critical, provided that it has no adverse effect upon the reaction. Suitable solvents

include alcohols, such as methanol or ethanol. Where an alcohol is used as the solvent, then the corresponding ester may be obtained. The reaction is preferably effected with heating.

Alternatively, compounds of formula (Im') in which m is 1 may be obtained by the hydrogenation, preferably catalytic hydrogenation, of the corresponding compound of formula (XIII); the compound of formula (XIII) may be prepared as described hereafter in Method P. The hydrogenation is preferably effected in the presence of a solvent, the nature of which is not critical, provided that it has no adverse effect upon the reaction. Suitable solvents include: alcohols, such as methanol or propanol; tetrahydrofuran; and lower aliphatic acids, such as acetic acid. The hydrogenation is preferably effected by bubbling hydrogen through the resulting solution. The catalyst is preferably a metal catalyst, for example palladium-on-charcoal or palladium-on-barium sulfate.

Where required, the compound of formula (Im') in which $R^{14}$ represents the alcoholic moiety of an ester may be converted to the corresponding free carboxylic acid, as in step M4. This is preferably achieved by reacting the compound of formula (Im') with an acid in the presence of a solvent. Suitable solvents include ethers such as dioxane and suitable acids include strong

mineral acids, such as sulfuric acid or hydrochloric acid. The reaction is preferably effected at room temperature or with heating.

In step M5, the compound of formula (Im") is converted to its corresponding acid halide. This is the same reaction as was employed in Method B and may be carried out under the same conditions.

In steps M6 and M7, this acid halide (Im"') is converted to the corresponding amide (Im"") by reaction with a compound of formula $HNR^7R^8$ or the thio compound (Im""') by reaction with a compound of formula $R^6SH$. These reactions may be carried out under the same conditions as described in Method C.

Method N

Compounds of formula (I) in which $R^4$ represents various substituted alkyl groups, alkenyl groups or carboxylic acyl groups may be prepared as illustrated in the following reaction scheme:

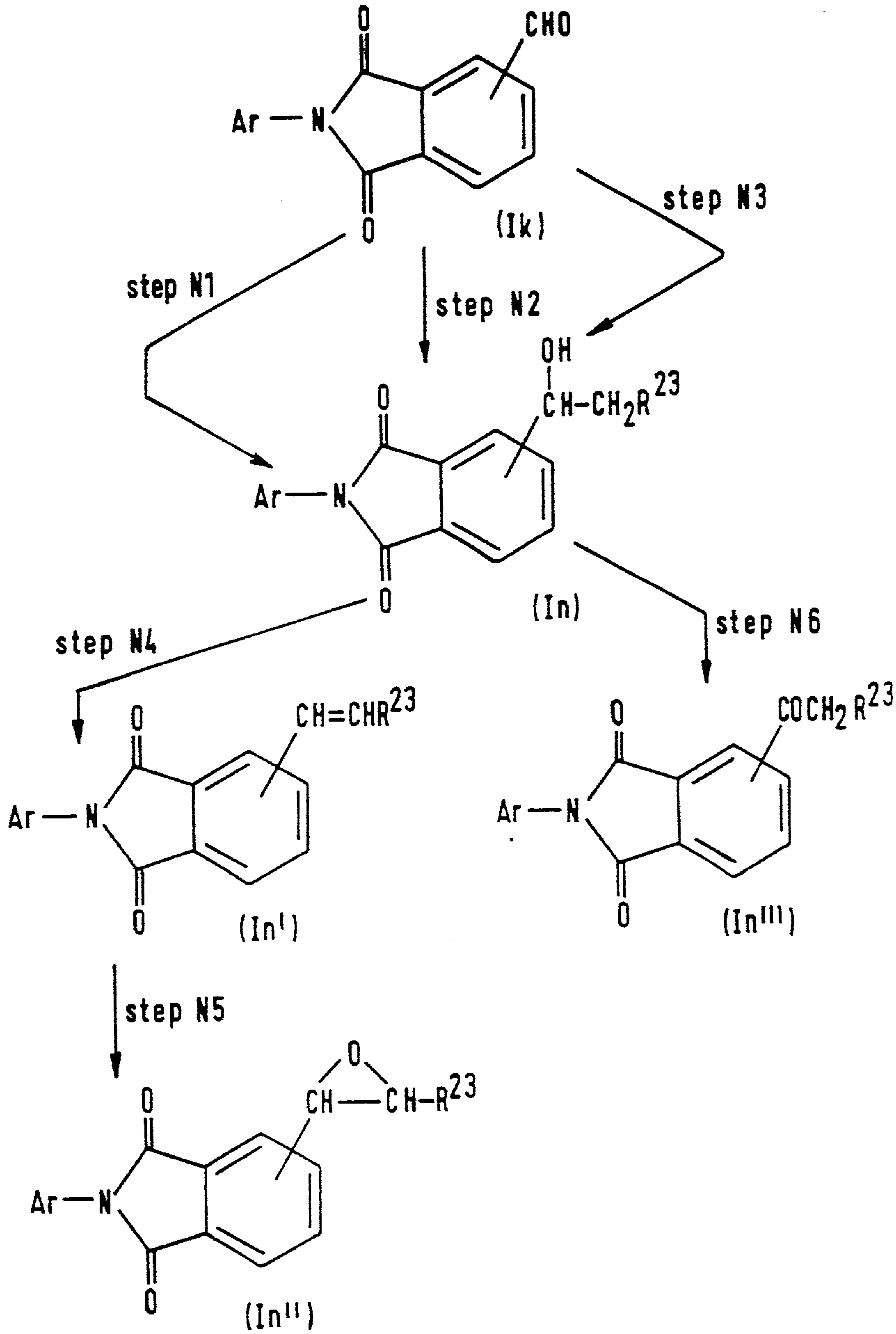


(Ik)

(In)

(In')

(In''')

(In'')

In the above formulae: Ar is as defined above; and $R^{23}$ represents a hydrogen atom, a $C_1$-$C_4$ alkyl group, a nitro group or an aliphatic acyl group.

In step N1 of the above reaction scheme, the compound of formula (Ik), which may have been prepared as described in Method K above, is reacted with a Grignard reagent of formula $R^{23}CH_2MgHal$ (in which $R^{23}$ represents only a hydrogen atom or a $C_1$-$C_4$ alkyl group and Hal is as defined above, preferably a bromine atom). The reaction is preferably effected at room temperature or with cooling. We prefer to carry out the reaction in the presence of a solvent, the nature of which is not particularly critical, provided that it does not adversely affect the reaction. Suitable solvents are ethers, such as diethyl ether or tetrahydrofuran.

Alternatively, the compounds of formula (In) in which $R^{23}$ represents a nitro group may be prepared in step N2 by reacting the formyl compound of formula (Ik) with nitromethane by the method described in Bull. Chem. Soc. Japan, 41, 1444 (1968).

Compounds of formula (In) in which $R^{23}$ represents only an aliphatic acyl group may alternatively be prepared by step N3, in which the

formyl compound of formula (Ik) is reacted with an aldehyde or ketone of formula $CH_3COR^{23}$. The reaction is preferably effected at room temperature in the presence of a base, such as sodium hydroxide, and in the presence of a solvent. The nature of the solvent is not particularly critical, provided that it has no adverse effect upon the reaction. Suitable solvents include: alcohols, such as methanol or ethanol; ethers, such as diethyl ether or tetrahydrofuran; hydrocarbons, such as benzene, toluene, hexane or heptane; and ketones, such as acetone, preferably a mixture of acetone and water.

If desired, the resulting hydroxy compound of formula (In) may be converted to the corresponding alkenyl compound of formula (In'), as in step N4. In this step, the compound of formula (In) is reacted with a suitable dehydrating agent, such as p-toluenesulfonic acid. The reaction is preferably effected in the presence of a solvent, the nature of which is not critical, provided that it has no adverse effect upon the reaction. Suitable solvents include such aromatic hydrocarbons as benzene and toluene. The reaction is preferably effected with heating. If desired, the resulting compound of formula (In') may be converted, in step N5, to the corresponding epoxy compound of formula (In"). This is effected by reacting the compound with an oxidising agent, which may be any such agent known

for converting an ethylenic double bond to an epoxy group. Suitable oxidising agents include m-chloroperbenzoic acid and perbenzoic acid. The reaction is preferably effected in the presence of a solvent, the nature of which is not critical, provided that it has no adverse effect upon the reaction. Suitable solvents include such halogenated hydrocarbons as chloroform and methylene chloride. The reaction temperature and reaction time will vary depending upon various factors; however, where the preferred oxidising agent, m-chloroperbenzoic acid, is used, the reaction will normally be carried out at room temperature or with heating up to 50°C.

An acyl compound of formula (In''') may, if desired, be prepared from the resulting hydroxy compound of formula (In) by oxidisation, preferably under the conditions and employing the same reagents and solvents as described in step K1 of Method K.

Method P

Compounds of formula (I) in which $R^4$ represents certain substituted alkenyl groups, that is to say compounds of formulae (Ip) and (Ip'), may be prepared as shown in the following reaction scheme:

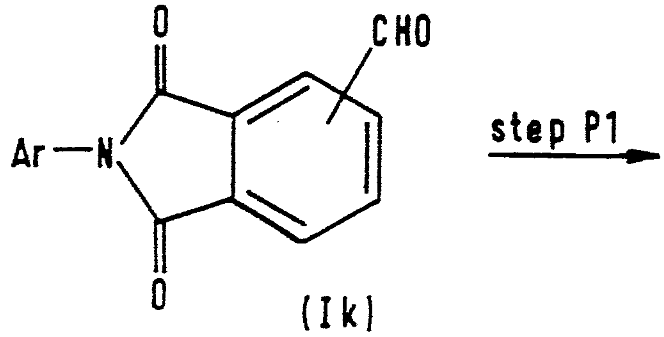


(Ik)

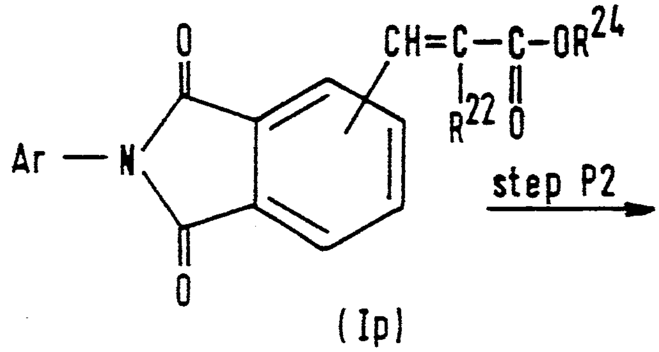


(Ip)

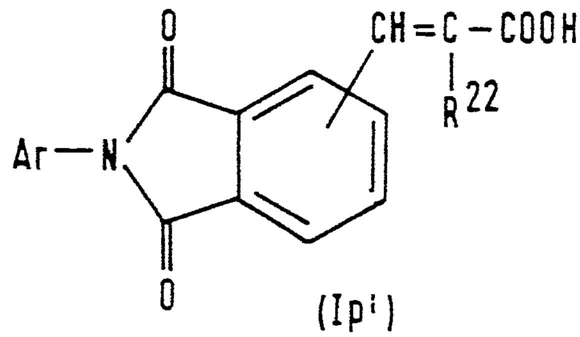

(Ip')

In the above formulae, Ar is as defined above, $R^{22}$ is also as defined above (see Method M) and $R^{24}$ represents a $C_1$-$C_4$ alkyl group, for example a methyl, ethyl, propyl, isopropyl, butyl, isobutyl, sec-butyl or t-butyl group.

In step P1 of this method, a compound of formula (XIV):

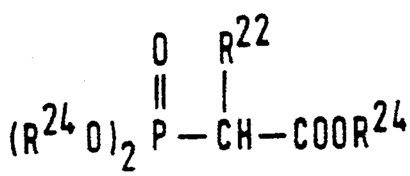 (XIV)

(in which $R^{22}$ and $R^{24}$ are as defined above and the three groups represented by $R^{24}$ may be the same or different) is first converted to its corresponding carbanion by treatment with a strong base, such as sodium hydride. The resulting carbanion is then reacted with the compound of formula (Ik) (which may have been prepared as described in Method K above), at room temperature or with heating. These reactions may be carried out simultaneously and are preferably effected in the presence of a solvent. The nature of the solvent

is not critical, provided that it has no adverse effect upon the reaction. Suitable solvents include: ethers, such as diethyl ether or tetrahydrofuran; and dimethylformamide.

If desired, the resulting compound of formula (Ip) may be hydrolysed to a corresponding acid of formula (Ip') by reaction with an acid at room temperature or with heating in a suitable solvent.

Method Q

Compounds of formula (I) in which $R^4$ represents certain bis(alkoxycarbonyl)-substituted acyl groups, that is to say compounds of formula (Iq) and (Iq'), may be prepared as illustrated in the following reaction scheme:

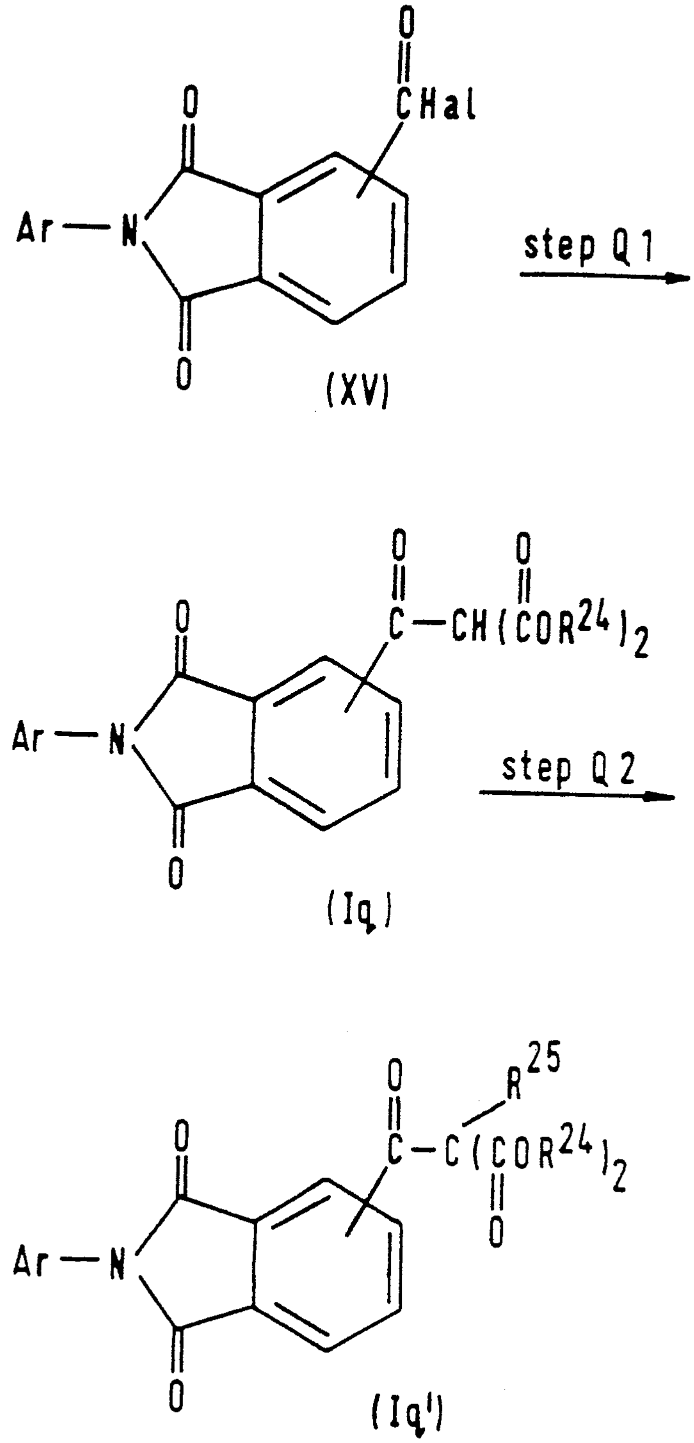


(XV)

(Iq)

(Iq')

In the above formulae, Ar, Hal and $R^{24}$ are as defined above; and $R^{25}$ represents an organic group, particularly an alkyl, alkenyl or alkynyl group, which may or may not be substituted, e.g. as previously described in relation to such groups.

Step Q1 of this method comprises reacting the compound of formula (XV), (which may have been prepared as described in Method B above) with a Grignard reagent of formula $R^{24}OMgCH(COOR^{24})_2$, in which $R^{24}$ is as defined above and the three groups represented by $R^{24}$ may the same or different. This Grignard reagent may be prepared by the method described in Org. Syn. Col., Vol. IV, 708 (1963). The reaction is preferably effected with heating in a suitable solvent.

If desired, the resulting compound of formula (Iq) may then be reacted with a compound of formula $R^{25}Hal$, for example 2-propenyl bromide, to give the compound of formula (Iq'). The reaction is preferably effected in the presence of a base, such as an alkali metal carbonate (e.g. sodium carbonate or potassium carbonate) and preferably with heating. The reaction is preferably effected in the presence of a solvent, the nature of which is not critical, provided that it has no adverse effect upon the reaction. Suitable solvents include ethers, such as dioxane; and nitriles, particularly acetonitrile.

**Method R**

Compounds of formula (I) in which $R^4$ represents a methyl group having a di($C_1$-$C_3$ alkoxy)phosphoryl substituent, that is to say compounds of formula (Ir):

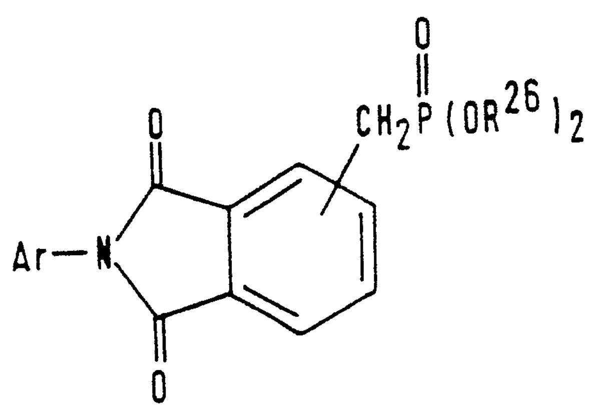

(Ir)

(in which Ar is as defined above and $R^{26}$ represents a $C_1$-$C_3$ alkyl group) may be prepared by reacting a compound of formula (Ie') (which may have been prepared as described in Method E) with a trialkyl phosphite, normally with heating at 90-100°C. The trialkyl phosphite is preferably employed in a molar excess with respect to the compound of formula (Ie').

Corresponding compounds in which $R^4$ represents a higher alkyl group ($C_2$ and above) having a dialkoxyphosphoryl substituent may be similarly prepared from the corresponding compounds in which $R^4$ represents a

higher haloalkyl group.

Method S

Compounds of formula (I) in which $R^4$ represents a mercaptomethyl group, that is to say compounds of formula (Is):

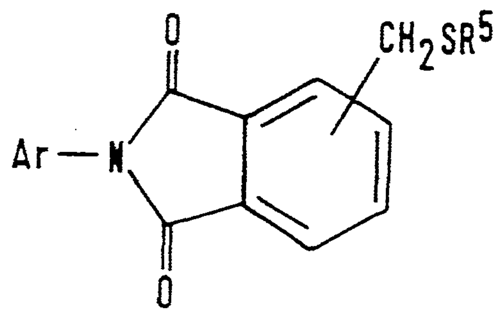

(Is)

(in which Ar and $R^5$ are as defined above) may be prepared by reacting a compound of formula (Ie') (which may have been prepared as described in Method E) with the corresponding thiol of formula $R^5SH$ (preferably in the form of a metal, more preferably alkali metal, thiolate, such as the sodium thiolate) in a suitable solvent. The nature of the solvent is not critical, provided that it has no adverse effect upon the reaction and suitable solvents include: alcohols, such as

ethanol; ketones, such as acetone; and ethers, such as tetrahydrofuran. The reaction temperature is not particularly critical and we therefore normally prefer to carry out the reaction at about room temperature.

Method T

Compounds of formula (I) in which $R^4$ represents an $\underline{N}^2$-hydroxyamidino group or an acylated hydroxyamidino group, that is to say compounds of formulae (It) and (It'), respectively:

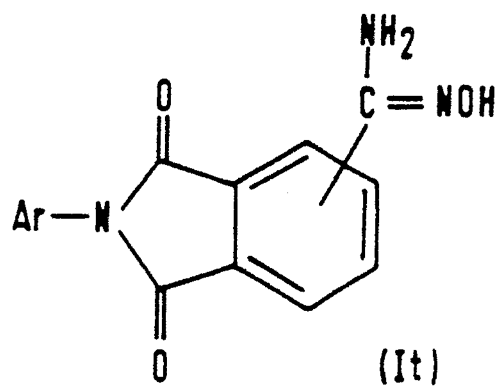

(It)

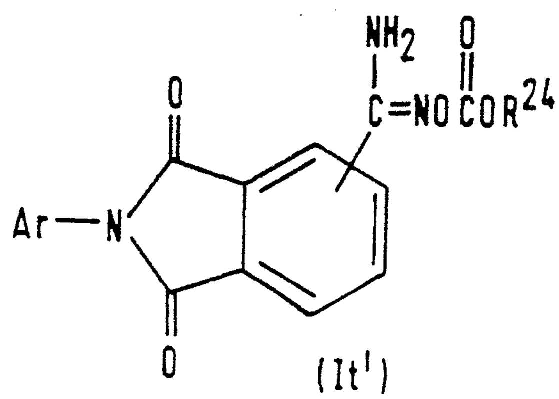

(It')

(in which Ar and $R^{24}$ are as defined above) may be prepared by reacting a cyano compound of formula (Id) (which may have been prepared as described in Method D) with hydroxylamine, to give the above compound of formula (It). This reaction is preferably effected in a suitable solvent (particularly a lower alcohol, such as methanol) and preferably at about room temperature or with heating, to give the compound of formula (It), which is then, if necessary, reacted with an acylating agent, e.g. a compound of formula $HalCOOR^{24}$ in a suitable solvent (particularly a ketone, such as acetone) and in the presence of an acid-binding agent. Suitable acid-binding agents are listed above in Method F; in the present case, the preferred acid-binding agents are the alkali metal carbonates and organic amines, such as triethylamine.

Method U

Compounds of formula (I) in which $R^4$ represents an amino group or a mono- or di- acylamino group may be prepared by converting the nitro group of a compound of formula (XVI):

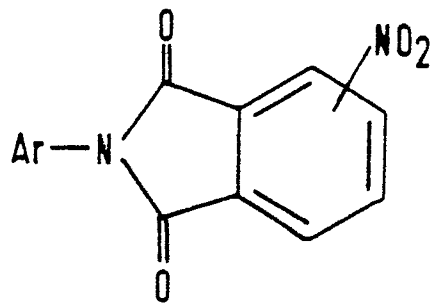

(XVI)

to an amino group and then, if desired, acylating the amino group.

The first step in this reaction, that is to say the conversion of the nitro group to an amino group, may be effected by treating the compound of formula (XVI) with a metal or a with a metal chloride in a mixture of an acid and a solvent. Examples of suitable metals include zinc, iron and tin, and examples of suitable metal chlorides include stannous chloride. Suitable acids include acetic acid and hydrochloric acid. Suitable solvents include such alcohols as ethanol and such ethers as dioxane and tetrahydrofuran.

Alternatively, the preparation of the amino compound may be effected by the catalytic hydrogenation of the compound of formula (XVI). The preferred catalyst is palladium-on-carbon and the reaction is preferably effected in the presence of a solvent, the nature of

which is not critical, provided that it has no adverse effect upon the reaction. Suitable solvents include such alcohols as methanol and ethanol.

The acylated derivatives of the resulting amino compound may be prepared by reacting the amino compound with a conventional acylating agent, for example a compound of formula $R^{24}COHal$, in the presence of an acid-binding agent and preferably in a suitable solvent, examples of both of which have been given above in relation to Method F. The reaction is preferably effected at room temperature, or with heating.

The starting material of formula (XVI) may have been prepared as described in Method A.

Method V

Compounds of formula (I) in which $R^4$ represents certain specific heterocyclic groups, that is to say compounds of formula (Iv)

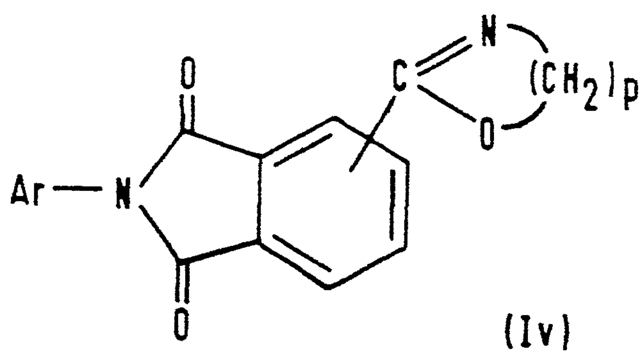

(Iv)

(in which Ar is as defined above and p is an integer, preferably from 2 to 4 and more preferably 2 or 3) may be prepared by reacting a compound of formula (XVII):

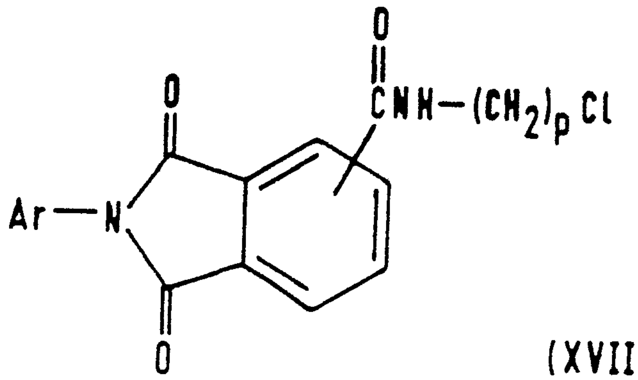

(XVII)

(in which Ar and p are as defined above) with an acid-binding agent, by the method described in J. Org. Chem., 39, 2787 (1974). Suitable acid-binding agents include alkali metal carbonates, such as potassium carbonate. The reaction is preferably effected in the presence of a solvent, the nature of which is not critical, provided that it has no adverse effect upon the reaction. Suitable solvents include: aromatic hydrocarbons, such as benzene or toluene; and ketones, such as acetones. The starting material for this Method, the compound of formula (XVII) may be prepared essentially as described in Method C.

**Method W**

Compounds of formula (I) in which $R^4$ represents an oxadiazolyl group, that is to say compounds of formula (Iw):

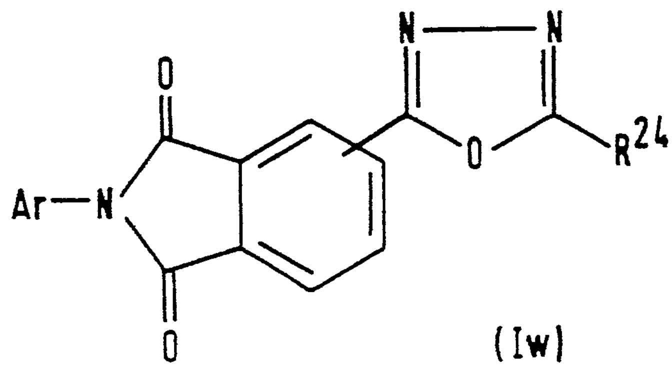

(Iw)

(in which Ar and $R^{24}$ are as defined above) may be prepared by reacting a compound of formula (XVIII):

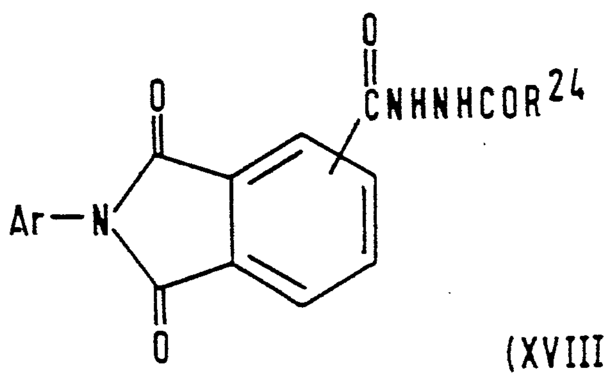

(XVIII)

(in which Ar and $R^{24}$ are as defined above) with a dehydrating agent, normally whilst heating. Suitable dehydrating agents include, for example, phosphorus oxychloride, thionyl chloride and phosphorus pentachloride, preferably phosphorus oxychloride. We prefer to employ a molar excess of the dehydrating agent.

Method X

Compounds of formula (Ix):

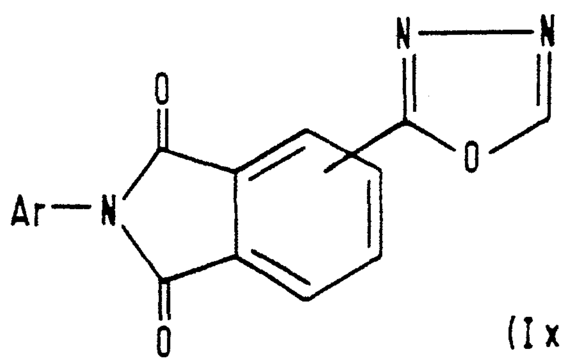

(Ix)

(in which Ar is as defined above), i.e. compounds of formula (I) in which $R^4$ represents an unsubstituted oxadiazolyl group, may be prepared by reacting a compound of formula (XIX):

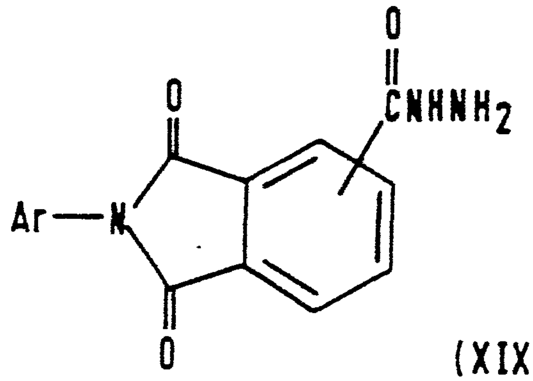

(XIX)

(in which Ar is as defined above) with an appropriate cyclising agent, which is capable of inserting a carbon atom. Suitable cyclising agents include the orthochloroformates (such as ethyl orthochloroformate) or formaldehyde (e.g. in the form of formalin or paraformaldehyde), preferably an orthochloroformate. The cyclising agent is preferably employed in excess and the reaction is preferably effected with heating.

<u>Method Y</u>

Compounds of formula (I) in which $R^4$ represents an oxo-substituted oxadiazolyl group, that is to say compounds of formula (Iy):

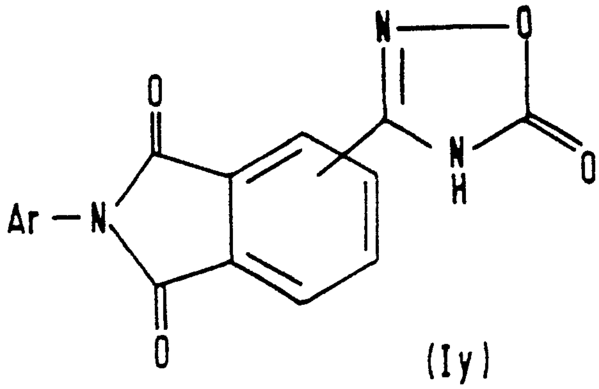

(Iy)

(in which Ar is as defined above) may be prepared by reacting a compound of formula (XX):

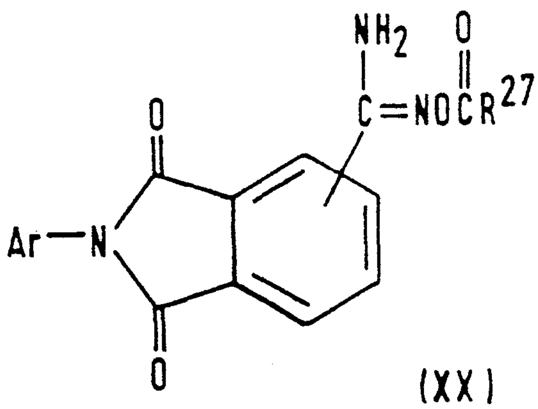

(XX)

(in which Ar is as defined above and $R^{27}$ represents a leaving group) with a base, preferably at room temperature, and preferably in a suitable solvent. Suitable leaving groups which may be represented by $R^{27}$ include lower, e.g. $C_1$-$C_4$, alkoxy groups (such

as methoxy, ethoxy or butoxy groups), aryloxy or aralkoxy groups (such as phenoxy or benzyloxy groups) or halogen atoms (such as chlorine). Suitable bases include: alkali metal alkoxides, particularly sodium methoxide; alkali metal hydrides, particularly sodium hydride; and metal hydroxides, especially alkali metal hydroxides, such as sodium hydroxide. Where a sodium alkoxide is used as the base, the corresponding alcohol is preferably used as the reaction solvent.

Method Z

Compounds of formula (I) in which $R^4$ represents a tetrazolyl or substituted tetrazolyl group, that is to say compounds of formula (Iz) and (Iz'), may be prepared as illustrated in the following reaction scheme:

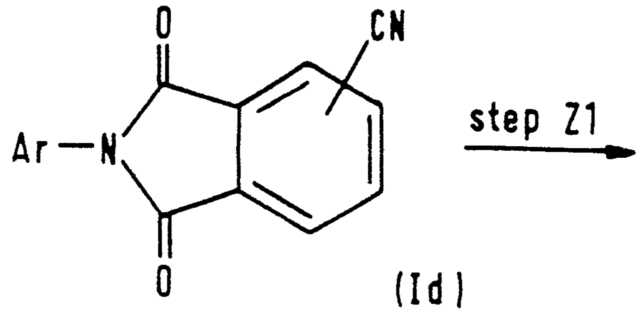


(Id)

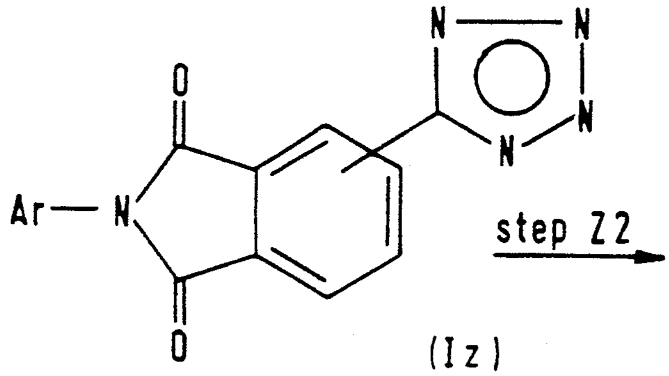


(Iz)

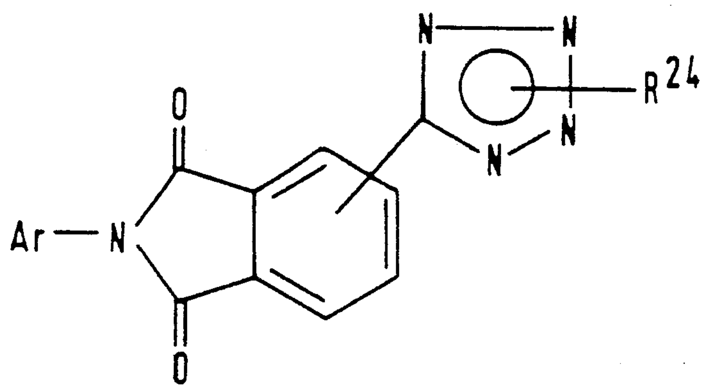

(Iz')

In the above formulae, Ar and $R^{24}$ are as defined above. The starting material, the compound of formula (Id), may have been prepared as described in Method D above.

Step Z1 of this reaction scheme may be effected by reacting the compound of formula (Id) with an azide (e.g. sodium azide) in a suitable solvent at room temperature or with heating. The nature of the solvent is not critical, provided that it has no adverse effect upon the reaction. Suitable solvents include: aromatic hydrocarbons, such as benzene or toluene; petroleum ether; ethers, such as tetrahydrofuran, dioxane or diethyl ether; and other polar solvents, such as dimethyl sulfoxide or dimethylformamide. The resulting compound of formula (Iz) is normally obtained in the form of its salt, corresponding to the azide salt employed in the reaction. The salt may be converted to the free compound of formula (Iz) by treatment with an acid, such as hydrochloric acid or sulfuric acid.

The alkylated compound of formula (Iz') may be obtained by reacting the compound of formula (Iz) with an alkylating agent, in a suitable solvent and in the presence of an acid-binding agent (such as those listed in Method F, particularly sodium carbonate). Suitable alkylating agents include: alkyl iodides, such as methyl iodide; dialkyl sulfates, such as dimethyl sulfate;

trialkyl phosphates; alkyl p-toluenesulfonates; and alkyl trifluoroacetates. The reaction is preferably effected with heating.

Following any of the above reactions, the desired compounds may be separated from the reaction mixture by conventional means. Where two or more reactions are to be carried out in sequence, these may, however, be carried out without isolation of the intermediate product.

The compounds of the invention may be employed as agricultural fungicides and show a preventive and curative effect against plant diseases, without damaging the host plants.

Specifically, they are particularly effective in the control of sheath blight, which is a very serious disease attacking rice plants; for this purpose, they are preferably employed in the form of a spray, particularly a foliar spray, or are applied, dissolved or dispersed in water, to the soil surface.

They also effectively control damping-off of various crops, such as beet, cotton plants and cucurbitaceous plants (i.e. plants of the gourd family), which disease is caused by pathogenic fungi of the class Rhizoctonia. They are also effective against early blight of tomato plants.

However, the compounds of the invention are most notably effective against the pathogenic fungi causing the diseases known as "club root" in various kinds of cruciferous plants. Club root is a disease which has been very difficult to control with known fungicides. For this purpose, the compounds of the invention are preferably applied by soil or foliage application.

The compounds of the invention may be formulated as preparations of the type commonly employed as agricultural fungicides, for example powdery dusts, coarse dusts, fine granules, coarse granules, wettable powders, emulsifiable concentrates, aqueous liquids, water-soluble powders and oil suspensions, by mixing them with a carrier and, if required, with other auxiliary agents. The carrier employed may be natural or synthetic and organic or inorganic; it is mixed with the active compound to assist that compound to reach the material to be treated and to make it easier to store, transport or handle the active compound.

Suitable solid carriers are: inorganic substances, such as clays (examples of which are kaolinite, montmorillonite or attapulgite), talc, mica, pyrophyllite, pumice, vermiculite, gypsum, calcium carbonate, dolomite, diatomaceous earth, magnesium carbonate, apatite, zeolite, silicic anhydride and synthetic calcium silicate; vegetable organic substances, such as soybean meal, tobacco powder, walnut powder, wheat flour, wood meal, starch and crystalline cellulose; synthetic or natural high molecular weight polymers, such as cumarone resins, petroleum resins, alkyd resins, polyvinyl chloride, polyalkylene glycols, ketone resins, ester gums, copal gum and dammar gum; waxes, such as carnauba wax and beeswax; and urea.

Examples of suitable liquid carriers are: paraffinic or naphthenic hydrocarbons, such as kerosine, mineral oil, spindle oil and white oil; aromatic hydrocarbons, such as benzene, toluene, xylene, ethylbenzene, cumene and methylnaphthalene; chlorinated hydrocarbons, such as carbon tetrachloride, chloroform, trichloroethylene, monochlorobenzene and o-chlorotoluene; ethers, such as dioxane and tetrahydrofuran; ketones such as acetone, methyl ethyl ketone, diisobutyl ketone, cyclohexanone, acetophenone and isophorone; esters, such as ethyl acetate, amyl acetate, ethylene glycol acetate, diethylene glycol acetate, dibutyl maleate and diethyl succinate; alcohols, such as methanol, hexanol, ethylene glycol, diethylene glycol, cyclohexanol and benzyl alcohol; ether alcohols, such as ethylene glycol monoethyl ether, ethylene glycol monophenyl ether, diethylene glycol monoethyl ether and diethylene glycol monobutyl ether; polar solvents, such as dimethylformamide and dimethyl sulfoxide; and water.

The fungicidal compositions of the present invention may contain surface active agents to emulsify, disperse, wet, spread, bind, control disintegration of, improve fluidity of or rust-proof the fungicidal composition or to stabilize the active compound; although any of the conventional classes of surface active agent, be they

non-ionic, anionic, cationic or amphoteric, may be employed, we prefer to employ non-ionic and/or anionic surface active agents. Examples of suitable non-ionic surface active agents are: the polymerization adducts of ethylene oxide with higher alcohols, such as lauryl alcohol, stearyl alcohol or oleyl alcohol; the polymerization adducts of ethylene oxide with alkylphenols, such as isooctylphenol or nonylphenol; the polymerization adducts of ethylene oxide with alkylnaphthols, such as butylnaphthol or octylnaphthol; the polymerization adducts of ethylene oxide with higher fatty acids, such as palmitic acid, stearic acid or oleic acid; the polymerization adducts of ethylene oxide with mono- or di-alkylphosphoric acids, such as stearylphosphoric acid or dilaurylphosphoric acid; the polymerization adducts of ethylene oxide with amines, such as dodecylamine; the polymerization adducts of ethylene oxide with higher fatty acid amides, such as stearamide; the polymerization adducts of ethylene oxide with higher fatty acid esters of polyhydric alcohols, such as sorbitan, and said fatty acid esters themselves; and the polymerization adducts of ethylene oxide with propylene oxide.

Examples of suitable anionic surface active agents are: alkyl sulfate salts, such as sodium lauryl sulfate

or oleyl sulfate amine salt; alkyl sulfonate salts, such as sodium dioctyl sulfosuccinate or sodium 2-ethylhexene sulfonate; and aryl sulfonate salts, such as sodium isopropylnaphthalene sulfonate, sodium methylene-bisnaphthalene sulfonate, sodium ligninsulfonate or sodium dodecylbenzene sulfonate.

Moreover, the agricultural fungicidal compositions of the present invention may be used in combination with high molecular weight compounds or other auxiliary agents, such as casein, gelatin, albumin, glue, sodium alginate, carboxymethylcellulose, methylcellulose, hydroxyethylcellulose or polyvinyl alcohol, in order to improve the properties and/or to increase the biological effect of the composition of the invention.

The above-mentioned carriers and various auxiliary agents may be used alone or in any desired combination, depending upon the type of preparation, the application and other factors.

In general, the fungicidal composition of the present invention may contain the compound of the invention in an amount of from 0.1 to 99% by weight, based upon the weight of the composition, although the precise amount of active ingredient in the composition

will, naturally, depend upon the form of the composition, the manner in which it is to be applied and on whether or not the composition contains any other active ingredient.

For example, dusts may conveniently contain from 1 to 25% by weight of the compound of formula (I), the remainder being a solid carrier.

Wettable powders may conveniently contain, for example, from 25 to 90% by weight of the compound (I), the remainder being a solid carrier and a dispersing and wetting agent, if required, together with a protective colloidal agent, a thixotropic agent and an anti-foaming agent.

Granules may conveniently contain from 0.1 to 35% by weight of the compound of formula (I), a major portion of the remainder being a solid carrier. The active compound is homogeneously admixed with the solid carrier or is adhered to or adsorbed onto the carrier surface; the diameter of each granule is preferably from 0.05 to 1.7 mm.

Emulsifiable concentrates may conveniently contain, for example, from 5 to 50% by weight of the compound of

formula (I) and from 5 to 20% by weight of an emulsifying agent, the remainder being a liquid carrier, together with, if required, a corrosion inhibitor.

The fungicidal compositions of the present invention, which are formulated into the various types of preparation described above, may be applied to a paddy or upland (dry) field in an amount of from 1 to 5000 g, more preferably from 10 to 1000 g, of the compound of formula (I) per 10 ares for pre- or post-emergence fungicidal activity; they may be applied by foliage spraying, soil drenching, spraying onto irrigation water or any other known method.

The fungicidal composition of the present invention, when employed for seed disinfection or coating, may effectively control soil-borne or seed infectious diseases by coating seeds in an amount of from 0.01 to 2%, preferably from 0.1 to 0.5%, by weight of the compound of formula (I), based on the weight of the seed.

The fungicidal composition of the present invention may additionally contain other fungicides in order to broaden the fungicidal spectrum and, in some cases, a synergistic effect may be observed. Examples of some known fungicides which may be employed in admixture with the compounds of the invention include:

thiophanate, thiophanatemethyl, benomyl, carbendazole, hymexazol, tecloftalm, chlorothalonil, fentinhydroxide, captan, captafol, thiram, edifenphos, tricyclazole, IBP, quintozene, futhalide, kasugamycin, vinclozolin, procymidone, isoprothiolane, probenazole, pyroquilon, chlorobenthiazone (S-1901), metalaxyl, echlomezol, Iprodione, propiconazole, etaconazole, triadimefon, prochloraz, 4-methylsulphonyloxyphenyl N-methylthiolcarbamate (NK-191) and N-(1-butoxy-2,2,2-trichloroethyl)salicylamide (NK-483).

Further, if desired, the compounds of formula (I) may be employed in any desired combination with: insecticides, such as isoxathion, fenitrothion, diazinon, trichlorphon, disulfoton, acephate, carbaryl, propoxur, methomyl, thiocyclam, cartap, pyrethrin, allethrin and fenvalerate; acaricides; herbicides; plant growth regulators; nematocides; bacteriocides; soil conditioners; inorganic soil additives; paper pots; artificial soil; compost; and chemical fertilizers, depending upon the type of preparation, application and other factors.

The fungicidal compositions of the present invention may also be used together with control agents effective against rice blast, helminthosporium leaf spot, bacterial leaf blight, rice stem borers, planthoppers and/or leafhoppers, to save the labor involved in separate applications. A combination of one or more of these additional control agents with the composition of the invention may be employed, depending upon the disease and/or the insect to be controlled and the form of the composition to be employed.

The invention is further illustrated by the following Examples, of which Examples 1 to 47 illustrate the preparation of compounds of the invention, Examples 48 to 54 illustrate the preparation of compositions of the invention and Examples 55 to 59 illustrate the biological activity of the compounds.

## EXAMPLE 1

3-Carboxy-N-(2,6-diisopropylphenyl)phthalimide (Compound No. 11)

1.92 g of trimellitic anhydride were dissolved, whilst heating and stirring, in 15 ml of methyl isobutyl

ketone. 1.8 g of 2,6-diisopropylaniline were added to the solution, and the resulting mixture was heated under reflux for 3 hours, using a reflux condenser equipped with a water separator. At the end of this time, the title compound, in crude form, was obtained by distilling off the solvent under reduced pressure, and this crude product was purified by recrystallization from 30% v/v aqueous ethanol, to give 3 g of the title compound, melting at 274-275°C.

## EXAMPLE 2

### 3-Chloroformyl-N-(2,6-diisopropylphenyl)phthalimide (Compound No. 44)

2 g of 3-carboxy-*N*-(2,6-diisopropylphenyl)-phthalimide (prepared as described in Example 1) were suspended in 30 ml of benzene; 1.4 g of thionyl chloride were added to the suspension, and the resulting mixture was heated under reflux for 2 hours. At the end of this time, the solvent and excess thionyl chloride were distilled off under reduced pressure, leaving 2.1 g of the title compound, melting at 148-150°C.

## EXAMPLE 3

N-(2,6-Diisopropylphenyl)-3-methoxycarbonylphthalimide (Compound No. 65)

2 g of 3-chloroformyl-N-(2,6-diisopropylphenyl)-phthalimide (prepared as described in Example 2) were dissolved in 15 ml of benzene; 5 ml of methanol were added to the solution, and the resulting mixture was refluxed for 2 hours.. The solvent was distilled off, leaving crude crystals of the title compound; these were recrystallized from methanol, to give 1.9 g of the title compound, melting at 173-173.5°C.

## EXAMPLE 4

3-Carbamoyl-N-(2,6-diisopropylphenyl)phthalimide (Compound No. 153)

2 g of 3-chloroformyl-N-(2,6-diisopropylphenyl)-phthalimide (prepared as described in Example 2) were dissolved in 10 ml of tetrahydrofuran; ammonia was introduced into the resulting solution, whilst stirring and ice-cooling, until the pH value of the reaction mixture reached 7. At this time, addition of ammonia ceased and the solution was stirred at room temperature for a further 1 hour. The reaction mixture was then

poured into water and the desired compound was extracted with ethyl acetate. The ethyl acetate extract was washed with water and dried over anhydrous sodium sulfate. The solvent was distilled from the extract under reduced pressure, giving the title compound in a crude form. This crude compound was recrystallized from methanol, to give 1.8 g of the title compound, melting at 251-252°C.

## EXAMPLE 5

3-Cyano-N-(2,6-diisopropylphenyl)phthalimide (Compound No. 258)

2.3 g of 3-carbamoyl-*N*-(2,6-diisopropylphenyl)-phthalimide (prepared as described in Example 4) were dissolved in 5 ml of ethylene dichloride; 1 ml of phosphorus oxychloride was added to the solution and the resulting mixture was heated under reflux for 2 hours, whilst stirring. At the end of this time, the reaction mixture was cooled and then poured into iced water. The desired compound was extracted with ethyl acetate. The ethyl acetate extract was washed with water and then dried over anhydrous sodium sulfate, after which the solvent was distilled off under reduced pressure. The resulting crude compound was recrystallized from hexane, to give 1.8 g of the title compound, melting at 162-163°C.

## EXAMPLE 6

Sodium 3-carboxylato-N-(2,6-diisopropylphenyl)phthalimide (Compound No. 32)

2 g of 3-carboxy-N-(2,6-diisopropylphenyl)-phthalimide (prepared as described in Example 1 above) were added to a solution of 0.39 g of sodium ethoxide in 15 ml of tetrahydrofuran; the mixture was then stirred at room temperature for 2 hours. At the end of this time, the solvent and the ethanol produced in the reaction were removed by evaporation under reduced pressure, giving 2.1 g of the title compound, melting at a temperature above 345°C.

## EXAMPLE 7

Aluminum tris[3-carboxylato-N-(2,6-diisopropylphenyl)-phthalimide] (Compound No. 266)

2 g of sodium 3-carboxylato-N-(2,6-diisopropylphenyl)phthalimide (prepared as described in Example 6 above) were dissolved in 15 ml of tetrahydrofuran. A solution of 0.24 g of aluminum chloride in 5 ml of tetrahydrofuran was added to the resulting solution, and the mixture was stirred at room temperature for 2 hours. At the end of this time, the sodium chloride which had formed was removed by filtration, and the

solvent was distilled off under reduced pressure, giving 1.9 g of the title compound melting at a temperature above 300°C.

## EXAMPLE 8

N-(2,6-Diisopropylphenyl)-3-hydroxymethylphthalimide (Compound No. 280)

65 mg of lithium borohydride were added to a solution of 1.05 g of 3-chloroformyl-N-(2,6-diisopropylphenyl)phthalimide (prepared as described in Example 2 above) in 8 ml of tetrahydrofuran, and the mixture was stirred at room temperature for 20 minutes. At the end of this time, the reaction mixture was poured into 10 ml of 2N hydrochloric acid and extracted with ethyl acetate. The extract was washed with a saturated aqueous solution of sodium chloride and dried over anhydrous sodium sulfate; the solvent was then distilled off under reduced pressure. The crude crystals obtained were recrystallized from benzene to give 0.85 g of the title compound, melting at 187-189°C.

## EXAMPLE 9

N-(2,6-Diisopropylphenyl)-3-isobutyryloxymethylphthalimide (Compound No. 286)

0.5 g of isobutyryl chloride and 0.45 g of triethylamine were added at room temperature to a solution of 1 g of N-(2,6-diisopropylphenyl)-3-hydroxymethylphthalimide (prepared as described in Example 8 above) in 30 ml of acetonitrile, and the mixture was stirred for 6 hours. At the end of this time, the reaction mixture was poured into water and extracted with ethyl acetate. The extract was washed with a saturated aqueous solution of sodium chloride and then dried over anhydrous sodium sulfate. The solvent was distilled off under reduced pressure to give crude crystals, which were then purified by recrystallization from a 1:1 by volume mixture of benzene and hexane, to give 0.82 g of the title compound melting at 158-159°C.

## EXAMPLE 10

3-Bromomethyl-N-(2,6-diisopropylphenyl)phthalimide (Compound No. 307)

0.54 g of phosphorus tribromide was added to a solution of 2 g of N-(2,6-diisopropylphenyl)-3-hydroxy-

methylphthalimide (prepared as described in Example 8 above) in 50 ml of diethyl ether, and the mixture was stirred at room temperature for 5 hours. At the end of this time, the reaction mixture was poured into water and extracted with diethyl ether. The extract was washed with a saturated aqueous solution of sodium chloride and dried over anhydrous sodium sulfate; the solvent was then distilled off to give crude crystals. The crystals were washed with methanol and dried to give 2.3 g of the title compound melting at 201-202°C.

## EXAMPLE 11

### N-(2,6-Diisopropylphenyl)-3-methoxymethylphthalimide (Compound No. 291)

162 mg of a 28% w/w methanolic solution of sodium methoxide were added to a solution of 1.2 g of 3-bromomethyl-N-(2,6-diisopropylphenyl)phthalimide (prepared as described in Example 10 above) in 10 ml of methanol, and the mixture was refluxed for 4 hours. The reaction mixture was cooled, poured into water and extracted with ethyl acetate. The extract was washed with water and the solvent was distilled off under reduced pressure. The residue was purified by column chromatography through silica gel, eluted with a 5:1 by volume mixture of hexane and ethyl acetate, to give 0.7 g of the title compound melting at 128-129°C.

## EXAMPLE 12

### 3-Aminomethyl-N-(2,6-diisopropylphenyl)phthalimide (Compound No. 308)

1 g of ammonia was added, whilst cooling and stirring, to a solution of 2 g of 3-bromomethyl-N-(2,6-diisopropylphenyl)phthalimide (prepared as described in Example 10 above) in 100 ml of methylene chloride, and the mixture was stirred at room temperature for 2 days. The reaction mixture was thoroughly washed with a saturated aqueous solution of sodium chloride until it became neutral. The solvent was then distilled off under reduced pressure to give crude crystals, which were washed with hexane and dried to afford 1.75 g of the title compound melting at 135-137.5°C.

## EXAMPLE 13

### 3-Cyclohexylaminomethyl-N-(2,6-diisopropylphenyl)-phthalimide (Compound No. 312)

0.28 g of cyclohexylamine and 0.28 g of triethylamine were added to a solution of 1 g of 3-bromomethyl-N-(2,6-diisopropylphenyl)phthalimide (prepared as described in Example 10 above) in 20 ml of methylene chloride, and the mixture was stirred for 6 hours. At

the end of this time, the reaction mixture was treated in the same manner as in Example 12, to give 0.72 g of the title compound melting at 148-149°C.

## EXAMPLE 14

N-(2,6-Diisopropylphenyl)-3-methoxyacetamidomethylphthalimide (Compound No. 316)

0.24 g of methoxyacetyl chloride and 0.28 g of triethylamine were added to a solution of 0.7 g of 3-aminomethyl-*N*-(2,6-diisopropylphenyl)phthalimide (prepared as described in Example 12 above) in 5 ml of methylene chloride, and the mixture was stirred at room temperature for 3 hours. At the end of this time, the reaction mixture was poured into water and extracted with methylene chloride. The extract was washed with water and the solvent was distilled off under reduced pressure. The residue was purified by column chromatography through silica gel, eluted with a 2:1 by volume mixture of hexane and ethyl acetate, to give 0.75 g of the title compound melting at 167-168°C.

EXAMPLE 15

N-(2,6-Diisopropylphenyl)-3-(1-imidazolylmethyl)-phthalimide (Compound No. 319)

204 mg of imidazole and 1.7 g of potassium carbonate were added to a solution of 1 g of 3-bromomethyl-*N*-(2,6-diisopropylphenyl)phthalimide (prepared as described in Example 10 above) in 50 ml of acetonitrile, and the mixture was refluxed for 1 hour. The reaction mixture was cooled, poured into water and extracted with ethyl acetate. The extract was washed with water and the solvent was distilled off. The residue was purified by column chromatography through silica gel, eluted with ethyl acetate, to give 0.62 g of the title compound melting at 216-218°C.

EXAMPLE 16

N-(2,6-Diisopropylphenyl)-3-formylphthalimide (Compound No. 323)

7 g of activated manganese dioxide were added to a solution of 1 g of N-(2,6-diisopropylphenyl)-3-hydroxymethylphthalimide (prepared as described in Example 8 above) in 25 ml of chloroform and the mixture was stirred at 70°C for 3 hours. The reaction mixture was

cooled, the excess manganese dioxide was removed by filtration, and the filtrate was concentrated by evaporation under reduced pressure. The residue was purified by column chromatography through silica gel, eluted with a 7:3 by volume mixture of hexane and ethyl acetate, to give 0.8 g of the title compound melting at 158.5-159.5°C.

## EXAMPLE 17

N-(2,6-Diisopropylphenyl)-3-hydroxyiminomethylphthalimide (Compound No. 326)

0.5 g of hydroxylamine hydrochloride was added at room temperature to a solution of 1 g of N-(2,6-diisopropylphenyl)-3-formylphthalimide (prepared as described in Example 16 above) in 20 ml of methanol, and the mixture was stirred for 2 hours. At the end of this time, the reaction mixture was concentrated by evaporation under reduced pressure and the residue was purified by column chromatography through silica gel, eluted with a 4:1 by volume mixture of hexane and ethyl acetate, to give 0.67 g of the title compound, melting at 232-234°C.

## EXAMPLE 18

N-(2,6-Diisopropylphenyl)-3-dimethoxymethylphthalimide (Compound No. 327)

A mixture of 1 g of N-(2,6-diisopropylphenyl)-3-formylphthalimide (prepared as described in Example 16 above), 5 ml of methyl orthoformate and 0.1 g of p-toluenesulfonic acid was stirred at room temperature for 2 hours. At the end of this time, the reaction mixture was poured into water and extracted with ethyl acetate. The extract was washed with water and the solvent was distilled off. The crude crystals obtained were recrystallized from benzene, to give 1 g of the title compound melting at 154-157°C.

## EXAMPLE 19

N-(2,6-Diisopropylphenyl)-3-(1,3-dioxolan-2-yl)-phthalimide (Compound No. 328)

0.1 g of p-toluenesulfonic acid was added to a mixture of 1 g of N-(2,6-diisopropylphenyl)-3-formylphthalimide (prepared as described in Example 16 above), 3 ml of ethylene glycol and 50 ml of toluene, and the whole mixture was refluxed for 3 hours. The reaction mixture was cooled, poured into water and then extracted

with ethyl acetate. The extract was washed with water and the solvent was distilled off under reduced pressure. The crude crystals produced were recrystallized from benzene to give 0.81 g of the title compound melting at 189.5-190.5°C.

## EXAMPLE 20

### 3-(2,2-Diacetylethyl)-N-(2,6-diisopropylphenyl)-phthalimide (Compound No. 350)

2 g of potassium carbonate were added to a mixture of 2 g of 3-bromomethyl-N-(2,6-diisopropylphenyl)phthalimide (prepared as described in Example 10 above), 2 g of acetylacetone and 50 ml of acetone, and the whole mixture was refluxed for 3 hours. At the end of this time, the reaction mixture was allowed to cool; it was then poured into water and extracted with ethyl acetate. The extract was washed with water and the solvent was distilled off to give crude crystals. The crystals were recrystallized from a 9:1 by volume mixture of hexane and ethyl acetate, to give 1.43 g of the title compound melting at 146-149°C.

## EXAMPLE 21

### 3-Cyanomethyl-N-(2,6-diisopropylphenyl)phthalimide (Compound No. 346)

1 g of 3-bromomethyl-N-(2,6-diisopropylphenyl)-phthalimide (prepared as described in Example 10 above) was added to a solution of 0.15 g of sodium cyanide in 3 ml of dimethyl sulfoxide, and the mixture was stirred at 50°C for 10 minutes. At the end of this time, the reaction mixture was poured into water and extracted with ethyl acetate. The extract was washed with water and the solvent was distilled off. The residue was purified by column chromatography through silica gel, eluted with a 8:1 by volume mixture of hexane and ethyl acetate, to give 0.6 g of the title compound melting at 206-209°C.

## EXAMPLE 22

### N-(2,6-Diisopropylphenyl)-3-ethoxycarbonylmethyl-phthalimide (Compound No. 332)

A mixture of 0.8 g of 3-cyanomethyl-N-(2,6-diisopropylphenyl)phthalimide (prepared as described in Example 21 above), 1 ml of concentrated sulfuric acid and 5 ml of ethanol was refluxed for 5 hours. The

reaction mixture was cooled, poured into water and extracted with ethyl acetate. The extract was washed with water and the solvent was distilled off under reduced pressure. The residue was purified by column chromatography through silica gel, eluted with a 7:1 by volume mixture of hexane and ethyl acetate, to give 0.66 g of the title compound melting at 160-162°C.

EXAMPLE 23

N-(2,6-Diisopropylphenyl)-3-(2-ethoxycarbonylethyl)-phthalimide (Compound No. 338)

0.4 g of 5% w/w palladium-on-carbon was added to a solution of 2.18 g of N-(2,6-diisopropylphenyl)-3-(2-ethoxycarbonylvinyl)phthalimide (prepared as described hereafter in Example 31) in 25 ml of ethyl acetate, and the mixture was subjected to catalytic reduction whilst bubbling hydrogen through the solution at room temperature. The palladium-on-carbon was then filtered off and the filtrate was concentrated by evaporation under reduced pressure to give crude crystals. These crystals were recrystallized from acetone to give 1.7 g of the title compound melting at 133-134.5°C.

EXAMPLE 24

3-(2-Carboxyethyl)-N-(2,6-diisopropylphenyl)phthalimide (Compound No. 330)

A mixture of 0.65 g of N-(2,6-diisopropylphenyl)-3-(2-ethoxycarbonylethyl)phthalimide (prepared as described in Example 23 above), 20 ml of dioxane and 15 ml of 1N sulfuric acid was refluxed for 4 hours. The reaction mixture was cooled, poured into water and extracted with ethyl acetate. The extract was washed with water and the solvent was distilled off under reduced pressure to give crude crystals. These crystals were washed with hexane and dried to give 0.51 g of the title compound melting at 183-185°C.

EXAMPLE 25

N-(2,6-Diisopropylphenyl)-3-(1-hydroxyethyl)phthalimide (Compound No. 351)

4 ml of a 1M solution of methylmagnesium bromide in tetrahydrofuran was added, whilst ice-cooling and stirring, to a solution of 1 g of N-(2,6-diisopropylphenyl)-3-formylphthalimide (prepared as described in Example 16 above) in 60 ml of diethyl ether, and the mixture was stirred at room temperature for 2 hours. At

the end of this time, 6N hydrochloric acid was added to the reaction mixture, followed by ethyl acetate. The organic phase was separated and washed with water, and then the solvent was distilled off. The residue was purified by column chromatography through silica gel, eluted with a 3:1 by volume mixture of hexane and ethyl acetate, to give 0.7 g of the title compound melting at 166-167°C.

## EXAMPLE 26

### N-(2,6-Diisopropylphenyl)-3-(1-hydroxy-2-nitroethyl)phthalimide (Compound No. 354)

1 g of *N*-(2,6-diisopropylphenyl)-3-formylphthalimide (prepared as described in Example 16 above) was dissolved in 20 ml of isopropanol containing 0.87 g of potassium fluoride; 1.83 g of nitromethane were then added, and the mixture was stirred at room temperature for 5 hours. At the end of this period, insoluble matter was filtered off and the filtrate was concentrated by evaporation under reduced pressure. The residue was purified by column chromatography through silica gel, eluted with a 4:1 by volume mixture of hexane and ethyl acetate, to give 0.6 g of the title compound melting at 172-176°C.

EXAMPLE 27

3-(2-Acetyl-1-hydroxyethyl)-N-(2,6-diisopropylphenyl)phthalimide (Compound No. 353)

0.6 ml of a 0.5N aqueous solution of sodium hydroxide was added at room temperature to a mixture of 1.34 g of N-(2,6-diisopropylphenyl)-3-formylphthalimide (prepared as described in Example 16 above), 4 ml of acetone and 10 ml of dioxane, and the mixture was stirred for 8 hours. At the end of this time, the reaction mixture was poured into water, and then the whole mixture was acidified with hydrochloric acid. The mixture was extracted with ethyl acetate and the extract was washed with water. The solvent was distilled off under reduced pressure and the residue was purified by column chromatography through silica gel, eluted with a 4:1 by volume mixture of hexane and ethyl acetate, to give 1.2 g of the title compound melting at 170-176°C.

EXAMPLE 28

3-Acetyl-N-(2,6-diisopropylphenyl)phthalamide (Compound No. 355)

1 g of manganese dioxide was added to a solution of 0.5 g of N-(2,6-diisopropylphenyl)-3-(1-hydroxyethyl)-

phthalimide (prepared as described in Example 25 above) in 10 ml of chloroform, and the mixture was refluxed for 4 hours. The reaction mixture was cooled and filtered to remove manganese dioxide, and the filtrate was concentrated by evaporation under reduced pressure. The residue was purified by column chromatography through silica gel, eluted with a 4:1 by volume mixture of hexane and ethyl acetate, to give 0.42 g of the title compound melting at 150-151°C.

## EXAMPLE 29

### N-(2,6-Diisopropylphenyl)-3-vinylphthalimide (Compound No. 360)

A mixture of 1 g of *N*-(2,6-diisopropylphenyl)-3-(1-hydroxyethyl)phthalimide (prepared as described in Example 25 above) 0.3 g of *p*-toluenesulfonic acid and 30 ml of toluene was refluxed for 5 hours. The reaction mixture was cooled, poured into water and extracted with ethyl acetate. The extract was washed with water and the solvent was distilled off under reduced pressure. The residue was purified by column chromatography through silica gel, eluted with an 8:1 by volume mixture of hexane and ethyl acetate, to give 0.45 g of the title compound melting at 60-61°C.

EXAMPLE 30

N-(2,6-Diisopropylphenyl)-3-oxiranylphthalimide (Compound No. 368)

A mixture of 0.4 g of N-(2,6-diisopropylphenyl)-3-vinylphthalimide (prepared as described in Example 29 above), 1 g of m-chloroperbenzoic acid and 6 ml of methylene chloride was stirred at 50°C for 16 hours. The reaction mixture was cooled, poured into water and extracted with methylene chloride. The extract was washed with water and the solvent was distilled off under reduced pressure. The residue was purified by column chromatography through silica gel, eluted with a 10:1 by volume mixture of hexane and ethyl acetate, to give 0.18 g of the title compound melting at 84-86°C.

EXAMPLE 31

N-(2,6-Diisopropylphenyl)-3-(2-ethoxycarbonylvinyl)-phthalimide (Compound No. 366)

0.144 g of sodium hydride was added to a solution of 0.74 g of triethyl phosphonoacetate in 10 ml of a 1:1 by volume mixture of tetrahydrofuran and dimethyl sulfoxide, and the whole mixture was stirred at room temperature for 15 minutes. 1 g of N-(2,6-diisopropylphenyl)-3-formylphthalimide (prepared as described in Example 25 above) was added and the mixture was stirred at 55-60°C for 20 minutes. At the end of this time, the reaction mixture was poured into water and extracted with ethyl acetate. The extract was washed with water and the solvent was distilled off under reduced pressure to give crude crystals. These crystals were recrystallized from ethanol, affording 0.84 g of the title compound melting at 131.5-132.5°C.

## EXAMPLE 32

### 3-Di(ethoxycarbonyl)acetyl-N-(2,6-diisopropylphenyl)-phthalimide (Compound No. 358)

0.27 g of magnesium was added to anhydrous ethanol, and then 2 ml of anhydrous diethyl ether and 1.76 g of diethyl malonate were added, and the mixture was refluxed for 3 hours. At the end of this time, a solution of 3.69 g of 3-chloroformyl-*N*-(2,6-diisopropylphenyl)phthalimide (prepared as described in Example 2 above) in 5 ml of tetrahydrofuran was added whilst heating, and the mixture was refluxed for 1 hour. The reaction mixture was cooled, poured into water and extracted with ethyl acetate. The extract was washed with water and the solvent was distilled off under reduced pressure to give crude crystals. These

crystals were recrystallized from ethanol, affording 4.5 g of the title compound melting at 114-122.5°C

EXAMPLE 33

3-[2,2-Di(ethoxycarbonyl)-4-pentenoyl]-N-(2,6-diisopropylphenyl)phthalimide (Compound No. 359)

0.5 g of allyl bromide and 1 g of potassium carbonate were added to a solution of 1 g of 3-di(ethoxycarbonyl)acetyl-N-(2,6-diisopropylphenyl)-phthalimide (prepared as described in Example 32 above) in 30 ml of acetonitrile, and the mixture was refluxed for 1 hour. The reaction mixture was cooled, poured into water and extracted with ethyl acetate. The extract was washed with water and the solvent was distilled off under reduced pressure. The residue was purified by column chromatography through silica gel, eluted with a 9:1 by volume mixture of hexane and ethyl acetate, to give 0.25 g of the title compound melting at 106.5-107.5°C.

EXAMPLE 34

N-(2,6-Diisopropylphenyl)-3-dimethoxyphosphorylmethylphthalimide (Compound No. 369)

A mixture of 0.6 g of 3-bromomethyl-N-(2,6-diisopropylphenyl)phthalimide (prepared as described in Example 10 above) and 2 ml of trimethyl phosphite was heated at 90-100°C, with stirring, for 1 hour. At the end of this time, the excess trimethyl phosphite was distilled off under reduced pressure to give crude crystals. These crystals were recrystallized from diisopropyl ether to give 0.46 g of the title compound melting at 133-134°C.

EXAMPLE 35

3-Diethoxyphosphorylthiomethyl-N-(2,6-diisopropylphenyl)phthalimide (Compound No. 370)

0.36 g of S-potassium O,O-diethyl thiophosphate was added to a solution of 0.7 g of 3-bromomethyl-N-(2,6-diisopropylphenyl)phthalimide (prepared as described in Example 10 above) in 20 ml of acetone, and the mixture was stirred at room temperature for 3 hours. At the end of this period, the reaction mixture was poured into water and extracted with ethyl acetate.

The extract was washed with water and the solvent was distilled off under reduced pressure. The residue was purified by column chromatography through silica gel, eluted with a 2:1 by volume mixture of hexane and ethyl acetate, to give 0.6 g of the title compound melting at 80-82°C.

## EXAMPLE 36

N-(2,6-Diisopropylphenyl)-3-(N-hydroxyamidino)-phthalimide (Compound No. 375)

0.42 g of hydroxylamine hydrochloride, 20 ml of methanol and 0.38 g of a 28% w/v methanolic solution of sodium methoxide were mixed for 10 minutes. A solution of 2 g of 3-cyano-N-(2,6-diisopropylphenyl)phthalimide (prepared as described in Example 5 above) in 30 ml of methanol was added, and the whole mixture was stirred at room temperature for 2 hours. At the end of this time, the reaction mixture was concentrated by evaporation under reduced pressure, and the residue was purified by column chromatography through silica gel, eluted with a 2:1 by volume mixture of hexane and ethyl acetate, to give 1.6 g of the title compound melting at 233-234°C.

## EXAMPLE 37

3-Amino-N-(2,6-diisopropylphenyl)phthalimide
(Compound No. 382)

3.5 g of N-(2,6-diisopropylphenyl)-3-nitrophthalimide (prepared as described in Example 1 above, but using 4-nitrophthalic anhydride instead of trimellitic anhydride) were dissolved in 30 ml of tetrahydrofuran and the solution was ice-cooled. A solution of 9 g of stannous chloride in 8 ml of concentrated hydrochloric acid was added to the ice-cooled solution, with stirring, and then stirring was continued for 3 hours. At the end of this period, the reaction mixture was poured into water and the mixture was adjusted to a neutral pH value by the addition of sodium hydrogen carbonate. The solid matter produced was removed by filtration and the filtrate was extracted with chloroform. The extract was washed with water and the solvent was distilled off to give crude crystals. These crystals were recrystallized from benzene containing a small volume of hexane to give 2.8 g of the title compound melting at 266-267°C.

## EXAMPLE 38

3-Acetamido-N-(2,6-diisopropylphenyl)phthalimide (Compound No. 385)

65 mg of acetyl bromide and 53 mg of triethylamine were added to a solution of 0.17 g of 3-amino-N-(2,6-diisopropylphenyl)phthalimide (prepared as described in Example 37 above) in 10 ml of benzene, and the mixture was stirred at room temperature for 1 hour. At the end of this time, the benzene was distilled off and the residue was purified by column chromatography through silica gel, eluted with a 1:1 by volume mixture of hexane and ethyl acetate, to give 0.16 g of the title compound melting at 237-238°C.

## EXAMPLE 39

N-(2,6-Diisopropylphenyl)-3-(2-oxazolinyl)phthalimide (Compound No. 390)

0.3 g of potassium carbonate was added to a solution of 0.5 g of 3-(2-chloroethylcarbamoyl)-N-(2,6-diisopropylphenyl)phthalimide (prepared as described in Example 4 above, but using 2-chloroethylamine instead of ammonia) in 30 ml of acetone, and the mixture was stirred at room temperature for 4 hours. At the end of this time, the reaction mixture was poured into water

and extracted with ethyl acetate. The extract was washed with water and the solvent was removed by evaporation under reduced pressure to leave crystals. These crystals were recrystallized from ethanol to give 0.35 g of the title compound melting at 190-191°C.

## EXAMPLE 40

### N-(2,6-Diisopropylphenyl)-3-(1,3,4-oxadiazol-2-yl)phthalimide (Compound No. 393)

A mixture of 1.5 g of N-(2,6-diisopropylphenyl)-3-hydrazinocarbonylphthalimide (prepared as described in Example 4 above, but using hydrazine instead of ammonia) and 10 ml of ethyl orthochloroformate was refluxed for 10 hours. The reaction mixture was cooled, poured into water and extracted with ethyl acetate. The extract was washed with water and the solvent was distilled off under reduced pressure. The residue was purified by column chromatography through silica gel, eluted with a 5:1 by volume mixture of hexane and ethyl acetate, to give 0.8 g of the title compound melting at 141-142°C.

EXAMPLE 41

N-(2,6-Diisopropylphenyl)-3-(2-methyl-1,3,4-oxadiazol-5-yl)phthalimide (Compound No. 394)

A mixture of 1 g of 3-(2-acetylhydrazinocarbonyl)-N-(2,6-diisopropylphenyl)phthalimide (prepared as described in Example 4 above, but using acetylhydrazine instead of ammonia) and 7 ml of phosphorus oxychloride was heated at 100-110°C for 3 hours, with stirring. The reaction mixture was cooled and then treated in the same manner as in Example 40, affording 0.35 g of the title compound melting at 213-214°C.

EXAMPLE 42

N-(2,6-Diisopropylphenyl)-3-(5-oxo-1,2,4-oxadiazol-3-yl)phthalimide (Compound No.395)

A mixture of 0.6 g of N-(2,6-diisopropylphenyl)-3-(N-ethoxycarbonyloxyamidino)phthalimide (prepared as described hereafter in Example 47), 10 ml of methanol and 0.1 g of sodium methoxide was stirred at room temperature for 20 minutes. At the end of this time, the reaction mixture was poured into water and the whole mixture was adjusted to neutrality by the addition of hydrochloric acid. The mixture was extracted with ethyl acetate, and the extract was washed with water. The

solvent was distilled off and the residue was treated in the same manner as in Example 40, to give 0.4 g of the title compound melting at 220-221°C.

EXAMPLE 43

N-(2,6-Diisopropylphenyl)-3-(1H-tetrazol-5-yl)-phthalimide (Compound No. 396)

1.8 g of aluminum chloride, 2.64 g of sodium azide and 1.5 g of 3-cyano-N-(2,6-diisopropylphenyl)-phthalimide (prepared as described in Example 5 above) were added in that order to 30 ml of ice-cooled anhydrous tetrahydrofuran. The mixture was refluxed for 5 hours. It was then cooled and poured into ice-cooled water, and the pH of the mixture was adjusted to neutrality by the addition of hydrochloric acid. The mixture was extracted with ethyl acetate, and the extract was washed with water. The solvent was distilled off to leave crystals, which were recrystallized from benzene to give 1.55 g of the title compound melting at 145-146°C.

## EXAMPLE 44

N-(2,6-Diisopropylphenyl)-3-(methyltetrazol-5-yl)phthalimide (Compound No. 269)

0.57 g of methyl iodide and 0.37 g of potassium carbonate were added to a solution of 1 g of N-(2,6-diisopropylphenyl)-3-(1H-tetrazol-5-yl)phthalimide (prepared as described in Example 43 above) in 20 ml of dioxane, and the mixture was refluxed for 1 hour. The reaction mixture was cooled and then treated in the same manner as in Example 40, to give 0.42 g of the title compound melting at 196-197°C. (The position on the tetrazole ring at which the methyl group is attached has not been confirmed but is believed to be the 1- or 2-position).

## EXAMPLE 45

N-(2-sec-Butyl-6-ethylphenyl)-3-carboxyphthalimide (Compound No.9)

1.92 g of trimellitic anhydride were dissolved in 15 ml of methyl isobutyl ketone, whilst heating and stirring. 1.8 g of 2-sec-butyl-6-ethylaniline were added, and the mixture was refluxed for 3 hours, while removing the water produced *in situ*. The solvent was

then removed by evaporation under reduced pressure to give crude crystals, which were recrystallised from a 4:1 by volume mixture of benzene and hexane, to give 2.95 g of the title compound melting at 210-211°C.

## EXAMPLE 46

3-Carboxy-N-(4-chloro-2,6-diisopropylphenyl)-phthalimide (Compound No.25)

11.8 g of trimellitic anhydride were dissolved in 60 ml of methyl isobutyl ketone, whilst heating and stirring. 13 g of 4-chloro-2,6-diisopropylaniline were added and the mixture was refluxed for 3 hours, while removing the water produced *in situ*. The solvent was removed by evaporation under reduced pressure to give crystals, which were recrystallised from a 5:3 by volume mixture of hexane and benzene, to give 12 g of the title compound melting at 252-253°C

## EXAMPLE 47

N-(2,6-diisopropylphenyl)-3-(N-ethoxycarbonyloxyamidino)-phthalimide (Compound No.376)

0.15 g of ethyl chloroformate and 0.14 g of triethylamine were added to a solution of 0.5 g of *N*-(2,6-diisopropylphenyl)-3-(*N*-hydroxyamidino)phthalimide

(prepared as described in Example 36) in 20 ml of acetone, and the mixture was stirred at room temperature for 3 hours. At the end of this time, the insoluble matter was filtered off and the filtrate was concentrated by evaporation under reduced pressure. The residue was purified by column chromatography through silica gel, eluted with methylene chloride, to give 0.55 g of the title compound melting at 111-112°C.

## EXAMPLE 48

Dust

2.5 parts of Compound No. 11, 47.5 parts of talc, 47 parts of clay and 3 parts of white carbon were uniformly mixed in a blender and then pulverized in a hammer mill to form the dust formulation.

## EXAMPLE 49

Dust

5 parts of Compound No. 153, 25 parts of activated carbon, 30 parts of talc and 40 parts of clay were uniformly mixed in a blender and then pulverized in a hammer mill to form a dust formulation with long-lasting efficacy.

## EXAMPLE 50

Coated Granules

77.5 parts of Kagalite No. 2 (crushed granular particles of a type of pumice, a trade name of Silver Sangyo K.K., Japan) were loaded into a jacketed ribbon

blender. 20.0 parts of an acetone solution containing 2.5 parts of Compound No. 11 were added in the blender and mixed thoroughly to secure its uniform adsorption on the granular particles. Acetone was then volatilized off while mixing by circulating steam through the jacket of the blender to heat the contents, thus preparing the base granules. These base granules were kept at 50-60°C and 20.0 parts of melted paraffin (melting range 68-70°C) were added while mixing to coat the granules with the paraffin. The coated granules were then cooled by circulating water through the jacket of the blender to give a less phytotoxic coated granular formulation containing 2.5 % of the active ingredient.

EXAMPLE 51

Coated Granules

A less phytotoxic coated granular formulation containing 2.5% of Compound No. 11 was obtained by following the same procedure as described in Example 49, except that stearic acid (melting range 64-70°C) was used as a coating agent instead of paraffin.

EXAMPLE 52

Granules

5 parts of Compound No. 258, 20 parts of bentonite, 1 part of calcium ligninsulfonate and 74 parts of clay were mixed. The mixture was kneaded with a suitable

amount of water and then granulated using a screw extruding granulator. The resulting product was dried and then sifted to form a granular formulation.

EXAMPLE 53

Wettable powder

50 parts of Compound No. 166, 5 parts of white carbon, 5 parts of Sorpol AC-2495 G (a trade name of Toho Kagaku K.K., Japan) and 40 parts of clay were uniformly mixed in a blender and pulverized in a hammer mill to form the wettable powder formulation.

EXAMPLE 54

Emulsifiable concentrate

10 parts of Compound No. 81, 10 parts of xylene, 12 parts of Paracol PS-2 (a trade name of Nihon Nyukazai K.K., Japan) and 68 parts of cyclohexanone were mixed to form an emulsifiable concentrate formulation.

The following Examples illustrate the use and activities of the agricultural fungicides of the present invention. In these Examples, the test compounds were employed in the form of a wettable powder, prepared as described in Example 53 above and containing 50% by weight of active compound, and then diluted with water to the appropriate concentration.

## EXAMPLE 55

Control of club root on chinese cabbage by soil incorporation.

Artificially infested soil containing the pathogenic fungus of club root (Plasmodiophora brassicae) was prepared by mixing the homogenized roots of diseased chinese cabbage into soil. Each test compound was incorporated into infested soil packed in a 300 ml pot at a concentration of 12.5 ppm. Seeds of chinese cabbage (variety "Muso") were sown at the rate of 5 seeds per pot. After emergence, the plants were thinned to one plant in each pot. The plants were grown for 45 days in a greenhouse and then removed for observation. The intensity of the disease was assessed according to the following standards.

| Disease intensity | Symptoms |
|---|---|
| 0 | No galls. |
| 1 | Few and small galls on lateral roots only. |
| 2 | Many small galls or a few big galls on lateral roots only. |
| 3 | Small galls on main roots but only a few galls on lateral roots. |
| 4 | Big galls on main roots but the size of galls on lateral roots was medium. |
| 5 | Many big galls on main roots as well as on lateral roots. |

Each experiment was carried out with 5 pots treated identically, and the results were averaged over the 5 pots. The results are reported in Table 1.

In addition to the compounds of the invention, the following prior art compounds were also tested:

Compound A = N-(2,6-diethylphenyl)-2-methylphthalimide.

Compound B = N-(2,6-diethylphenyl)-3-methylphthalimide.

Compound C = N-(2,6-diisopropylphenyl)-2-methylphthalimide.

Compound D = N-(2,6-diisopropylphenyl)-3-methylphthalimide.

Compound E = 2-chloro-N-(2,6-diethylphenyl)phthalimide.

Compound F = 3-chloro-N-(2,6-diethylphenyl)phthalimide.

Compound G = 2-chloro-N-(2,6-diisopropylphenyl)phthalimide.

Compound H = 3-chloro-N-(2,6-diisopropylphenyl)phthalimide.

Compound J = N-(2,6-diethylphenyl)-2-methoxyphthalimide.

Compound K = N-(2,6-diethylphenyl)-3-methoxyphthalimide.

Compound L = N-(2,6-diisopropylphenyl)-2-methoxyphthalimide.

Compound M = N-(2,6-diisopropylphenyl)-3-methoxyphthalimide.

As a control, a group of infested, untreated plants was also observed.

Table 1

| Compound No. | Disease Intensity | Compound No. | Disease Intensity |
|---|---|---|---|
| 2 | 0.8 | 83 | 0 |
| 4 | 0.6 | 84 | 0 |
| 5 | 0 | 85 | 0 |
| 6 | 1.2 | 86 | 0 |
| 8 | 0 | 87 | 0 |
| 9 | 0 | 88 | 0 |
| 11 | 0 | 91 | 0 |
| 21 | 1.4 | 93 | 0 |
| 25 | 0 | 95 | 0 |
| 26 | 1.0 | 96 | 0 |
| 32 | 0 | 97 | 0 |
| 34 | 0 | 98 | 0 |
| 35 | 1.2 | 99 | 0 |
| 38 | 0 | 100 | 0 |
| 41 | 0 | 101 | 0 |
| 44 | 0 | 102 | 0.8 |
| 52 | 1.8 | 103 | 0 |
| 58 | 1.6 | 104 | 0 |
| 59 | 0.4 | 105 | 1.0 |
| 60 | 1.2 | 106 | 0 |
| 61 | 0.4 | 108 | 0 |
| 62 | 0 | 109 | 0 |
| 63 | 0 | 110 | 0 |
| 65 | 0 | 111 | 1.2 |
| 70 | 0.8 | 112 | 0 |
| 75 | 0 | 113 | 0 |
| 77 | 0 | 114 | 0 |
| 78 | 0 | 115 | 1.0 |
| 79 | 0 | 121 | 0 |
| 81 | 0 | 123 | 0 |
| 82 | 0 | 124 | 1.4 |

| Compound No. | Disease Intensity | Compound No. | Disease Intensity |
|---|---|---|---|
| 126 | 0 | 191 | 0 |
| 127 | 0 | 193 | 1.4 |
| 128 | 0 | 194 | 0 |
| 129 | 0 | 195 | 0 |
| 130 | 2.4 | 196 | 0.2 |
| 134 | 0.4 | 197 | 0.6 |
| 135 | 0 | 199 | 1.0 |
| 136 | 0 | 200 | 1.0 |
| 137 | 0 | 203 | 0 |
| 138 | 0.6 | 210 | 1.2 |
| 139 | 1.4 | 216 | 0 |
| 141 | 0.4 | 229 | 0 |
| 142 | 0 | 232 | 0 |
| 143 | 0 | 236 | 0.4 |
| 144 | 0 | 238 | 0 |
| 145 | 0 | 239 | 0 |
| 149 | 0 | 240 | 0 |
| 150 | 0.8 | 242 | 0 |
| 151 | 0 | 243 | 0 |
| 152 | 0 | 244 | 0 |
| 153 | 0 | 245 | 0.4 |
| 160 | 0 | 246 | 0 |
| 161 | 1.4 | 248 | 0 |
| 162 | 0 | 256 | 0 |
| 163 | 0 | 257 | 0 |
| 164 | 0 | 258 | 0.2 |
| 166 | 0 | 264 | 0 |
| 174 | 0.8 | 265 | 0 |
| 176 | 0 | 266 | 0 |
| 177 | 1.0 | 267 | 0 |
| 179 | 1.0 | 271 | 0 |
| 182 | 0 | 275 | 0 |
| 188 | 0 | 276 | 0 |
| 189 | 0 | 277 | 0.5 |
| 190 | 0 | 278 | 0 |

| Compound No. | Disease Intensity | Compound No. | Disease Intensity |
|---|---|---|---|
| 279 | 0 | 327 | 1.2 |
| 280 | 0 | 328 | 0 |
| 283 | 0 | 329 | 0 |
| 284 | 0 | 330 | 0 |
| 285 | 0 | 332 | 0 |
| 286 | 0 | 338 | 0 |
| 287 | 0 | 346 | 0 |
| 288 | 1.0 | 350 | 0 |
| 289 | 0 | 353 | 0 |
| 290 | 0 | 354 | 0 |
| 292 | 1.0 | 356 | 1.4 |
| 293 | 0.5 | 359 | 0 |
| 294 | 0.8 | 361 | 0 |
| 295 | 0 | 362 | 0.8 |
| 297 | 0 | 363 | 0 |
| 299 | 0.4 | 365 | 0 |
| 300 | 0.4 | 366 | 0 |
| 301 | 0.8 | 367 | 0 |
| 304 | 0 | 368 | 0 |
| 305 | 0 | 370 | 0.8 |
| 306 | 1.3 | 375 | 0 |
| 307 | 0 | 387 | 0 |
| 308 | 0 | 389 | 0 |
| 310 | 1.4 | 390 | 0 |
| 311 | 1.0 | 391 | 0.4 |
| 313 | 0 | 393 | 0 |
| 314 | 0 | 394 | 0.8 |
| 316 | 0.8 | 395 | 0 |
| 317 | 0.5 | Control | 5 |
| 318 | 0 | A | 5 |
| 321 | 1.0 | B | 5 |
| 322 | 0 | C | 5 |
| 323 | 0 | D | 5 |
| 325 | 1.0 | E | 5 |
| 326 | 0 | F | 5 |

| Compound No. | Disease Intensity | Compound No. | Disease Intensity |
|---|---|---|---|
| G | 5 | K | 5 |
| H | 5 | L | 5 |
| J | 5 | M | 5 |

A result of "5", as achieved with the untreated control and the prior art compounds, indicates that the respective test compounds are totally ineffective.

EXAMPLE 56

Control of club root on chinese cabbage by foliar spray

Soil inoculated with the pathogenic fungus of club root (*Plasmodiophora brassicae*) was packed in a pot (9 cm in diameter). Seeds of chinese cabbage (variety: "Muso") were sown, and the plants were thinned to a plant density of 5 plants per pot after emergence. A 100 ppm solution of the test compound was sprayed onto the plants twice, first at the true leaf emergence stage and then at the 2-3 leaf stage, at the rate of 10 ml per pot. The plants were grown for 45 days in a greenhouse, after which they were removed and the intensity of disease was assessed according to the same standard as in Example 55. The results were averaged over 2 pots (10 plants) and the results are shown in Table 2.

Table 2

| Compound No. | Disease Intensity | Compound No. | Disease Intensity |
|---|---|---|---|
| 6 | 1.4 | 78 | 1.0 |
| 11 | 0.6 | 153 | 1.1 |
| 65 | 1.0 | 258 | 0.9 |
| 75 | 0.8 | Untreated Control | 5.0 |

EXAMPLE 57

Control of rice sheath blight by preventive spraying

Rice seedlings (variety : "Nihonbare") at the 4-5 leaf stage were sprayed with a 300 ppm solution of the compound under test at the rate of 50 ml per 3 pots. The sprayed plants were kept for 24 hours at room temperature and then inoculated with the pathogenic fungus of rice sheath blight (Rhizoctonia solani) by placing 4-5 oat grains on which the pathogenic fungus had previously been cultured around the foot of each rice seedling. Thereafter the plants were transfered to and kept in a moist chamber at 25 - 27°C. The intensity of the disease was assessed by measuring the height (cm) of the uppermost lesions 7 days after inoculation. The results obtained are shown in Table 3.

Table 3

| Compound No. | Height of lesion (cm) | Compound No. | Height of lesion (cm) |
|---|---|---|---|
| 11 | 0.9 | 191 | 0 |
| 41 | 0 | 192 | 0 |
| 52 | 4.4 | 193 | 0 |
| 59 | 5.6 | 194 | 0 |
| 61 | 0.9 | 195 | 0 |
| 62 | 0 | 196 | 0.6 |
| 74 | 1.4 | 200 | 1.2 |
| 98 | 1.5 | 203 | 0 |
| 99 | 0 | 204 | 0.8 |
| 100 | 0 | 205 | 0.8 |
| 101 | 0 | 206 | 1.0 |
| 103 | 6.1 | 207 | 1.0 |
| 104 | 0 | 229 | 0 |
| 110 | 0 | 232 | 0 |
| 112 | 0 | 236 | 0 |
| 143 | 7.5 | 237 | 3.0 |
| 145 | 1.6 | 238 | 2.8 |
| 150 | 0 | 240 | 1.3 |
| 151 | 0 | 242 | 0.4 |
| 152 | 0 | 243 | 0 |
| 153 | 7.4 | 246 | 0 |
| 160 | 0 | 247 | 0 |
| 162 | 0 | 256 | 0 |
| 163 | 0 | 257 | 0 |
| 166 | 0 | 258 | 0 |
| 174 | 6.8 | 264 | 3.0 |
| 175 | 0 | 273 | 1.4 |
| 176 | 5.2 | 275 | 0 |
| 178 | 7.7 | 276 | 1.6 |
| 180 | 0 | 277 | 2.3 |
| 183 | 0 | 278 | 0 |
| 186 | 3.5 | 279 | 0 |
| 188 | 3.5 | 280 | 0 |

| Compound No. | Height of lesion (cm) | Compound No. | Height of lesion (cm) |
|---|---|---|---|
| 283 | 2.8 | 350 | 3.4 |
| 284 | 0 | 351 | 0 |
| 285 | 0 | 352 | 0 |
| 286 | 1.4 | 354 | 1.8 |
| 290 | 3.1 | 355 | 4.8 |
| 292 | 0 | 361 | 0 |
| 293 | 1.8 | 365 | 0 |
| 297 | 3.0 | 367 | 0 |
| 298 | 4.3 | 368 | 0 |
| 304 | 0.9 | 369 | 1.5 |
| 307 | 0 | 373 | 1.4 |
| 308 | 2.0 | 374 | 1.8 |
| 309 | 0 | 375 | 2.0 |
| 311 | 0.9 | 378 | 2.0 |
| 312 | 2.4 | 381 | 3.3 |
| 315 | 2.1 | 382 | 2.1 |
| 316 | 1.4 | 383 | 0.6 |
| 317 | 3.3 | 385 | 1.8 |
| 319 | 0.9 | 386 | 0.8 |
| 320 | 1.1 | 387 | 2.3 |
| 323 | 0 | 388 | 0.8 |
| 325 | 0 | 389 | 3.7 |
| 326 | 0 | 390 | 0.9 |
| 329 | 7.1 | 395 | 0 |
| 330 | 0 | Untreated control | 23.0 |
| 332 | 6.5 | | |
| 346 | 0 | | |

EXAMPLE 58

Control of damping off on cucumbers by soil drenching

The pathogenic fungus of damping off disease (Rizoctonia solani) which had been cultured on wheat bran for 2 weeks at 28°C was mixed uniformly into soil. The infested soil was packed in a pot (12 cm in diameter) and then 20 seeds of cucumber (variety : "Sagamihanpaku") were sown in each pot. Thereafter the soil was drenched with a 250 ppm solution of the compound under test at the rate of 3 litres per square meter. The treated plants were kept in a greenhouse for 2 weeks at 25°C and then the number of diseased plants was counted. The cumulative number of diseased plants in each group of 3 identically treated pots is shown in Table 4.

| Compound No. | No. of Diseased Plants | Compound No. | No. of Diseased Plants |
|---|---|---|---|
| 8 | 3 | 161 | 3 |
| 9 | 2 | 163 | 3 |
| 11 | 5 | 164 | 5 |
| 62 | 10 | 166 | 0 |
| 79 | 10 | 174 | 10 |
| 98 | 14 | 176 | 1 |
| 99 | 2 | 190 | 2 |
| 100 | 4 | 195 | 4 |
| 101 | 5 | 196 | 5 |
| 149 | 12 | 199 | 18 |
| 151 | 2 | 200 | 3 |
| 152 | 2 | 207 | 5 |
| 153 | 10 | 223 | 11 |
| 160 | 2 | 232 | 3 |

| Compound No. | No. of Diseased Plants | Compound No. | No. of Diseased Plants |
|---|---|---|---|
| 236 | 5 | 277 | 2 |
| 243 | 9 | 280 | 0 |
| 244 | 3 | 283 | 5 |
| 246 | 4 | 291 | 4 |
| 248 | 2 | 302 | 4 |
| 255 | 8 | 383 | 9 |
| 257 | 6 | Untreated | |
| 258 | 13 | Control | 54 |

EXAMPLE 59

Control of early blight on tomato plants by preventive spraying

Tomato seedlings (variety : "Fukuju" No. 2) at the 3-4 leaf stage were sprayed with a 300 ppm solution of the compound under test at the rate of 50 ml per 3 pots. One day after this treatment, a conidial suspension of the pathogenic fungus of early blight of tomatoes (*Alternaria solani*) was sprayed onto the plants. The inoculated plants were kept in a moist chamber maintained at 22°C to promote the development of lesions. The intensity of disease was assessed 7 days after the inoculation according to the following standards.

| Disease intensity | Symptoms |
|---|---|
| 0 | No lesions |
| 1 | Diseased area of each leaf is less than 10% |
| 2 | Diseased area of each leaf is 10 - 50% |
| 3 | Diseased area of each leaf is more than 50%. |

The results were averaged over 3 pots and are shown in Table 5.

Table 5

| Compound No. | Disease Intensity. |
|---|---|
| 162 | 0 |
| 173 | 0 |
| 237 | 0 |
| Untreated control | 3.0 |

CLAIMS

1. Compounds of formula (I):

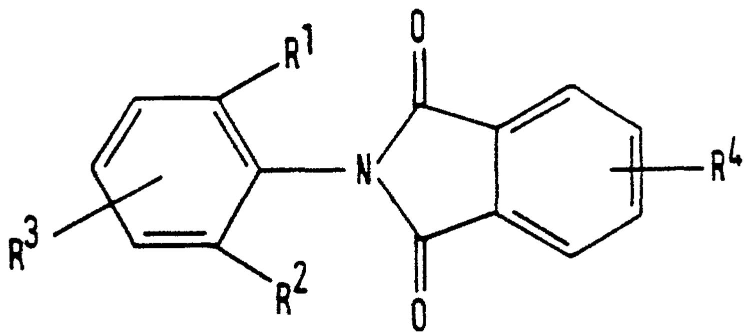

(I)

in which:

$R^1$ and $R^2$ are the same or different and each represents a $C_1$-$C_6$ alkyl group or a $C_2$-$C_6$ alkenyl group;

$R^3$ represents a hydrogen atom, a halogen atom or a $C_1$-$C_6$ alkyl group; and

$R^4$ represents a carboxy group; a thiocarboxy group; a cyano group; a nitro group; an amino group; an amino group having 1 or 2 aliphatic carboxylic acyl substituents; a heterocyclic group; an aliphatic carboxylic acyl group; a $C_2$-$C_4$ alkenyl group; or a $C_1$-$C_4$ alkyl or $C_2$-$C_4$ alkenyl group having 1 or 2 substituents selected from groups of formula $-XR^5$

(wherein X represents an oxygen or sulfur atom or an -NH- group, and $R^5$ represents a hydrogen atom, a $C_3$-$C_8$ cycloalkyl group, a $C_2$-$C_4$ alkenyl group, a $C_2$-$C_4$ alkynyl group, an aryl group, a carboxy group, an acyl group, a $C_1$-$C_6$ alkyl group, or a $C_1$-$C_6$ alkyl group having at least one substituent selected from cyano groups, nitro groups, $C_1$-$C_4$ alkoxy groups, aryl groups, aryloxy groups and $C_1$-$C_4$ alkylamino groups), halogen atoms, heterocyclic groups, carboxy groups, thiocarboxy groups, cyano groups, aliphatic carboxylic acyl groups, aromatic carboxylic acyl groups, nitro groups, di($C_1$-$C_3$ alkoxy)phosphoryl groups and hydroxyimino groups;
and, where $R^4$ represents a carboxy or thiocarboxy group or a group including said carboxy or thiocarboxy group, the corresponding acid halides, esters, amides and salts thereof;
and, where $R^4$ represents a group including a hydroxy or mercapto group, esters thereof;
and, where $R^4$ represents or includes a substituted or unsubstituted amino group, acid addition salts thereof.

2. Compounds as claimed in Claim 1, wherein $R^4$ represents a group of formula $-CO.R^{30}$, and $R^{30}$ represents a hydrogen or halogen atom.

3. Compounds as claimed in Claim 1, wherein $R^4$

represents a group of formula $-CO.X''R^{11}$, and: X" represents an oxygen or sulfur atom; and $R^{11}$ represents a hydrogen atom, an alkali metal atom, an alkaline earth metal atom, a trivalent metal atom, an ammonium group or an organic amine residue.

4. Compounds as claimed in Claim 1, wherein $R^4$ represents a group of formula $-CO.X''R^{11}$, and: X" represents an oxygen or sulfur atom; and $R^{11}$ represents an alkyl group, a substituted alkyl group [wherein the substituents are selected from halogen atoms, cyano groups, hydroxy groups, $C_1$-$C_6$ alkoxy groups, $C_1$-$C_6$ alkylthio groups, phenoxy groups, carboxylic acyloxy groups, epoxy groups, carboxy groups, ($C_1$-$C_6$ alkoxy)carbonyl groups, di($C_1$-$C_6$ alkyl)amino groups and 5- or 6-membered heterocyclic rings containing at least one nitrogen or oxygen hetero-atom], a $C_2$-$C_6$ alkenyl group, a $C_2$-$C_6$ alkynyl group, a $C_3$-$C_8$ cycloalkyl group, a $C_3$-$C_8$ cycloalkyl group having at least one $C_1$-$C_6$ alkyl substituent, an aralkyl group, a substituted aralkyl group (wherein the substituent is at least one group or atom selected from halogen atoms, $C_1$-$C_6$ alkyl groups and nitro groups), a phenyl group, a substituted phenyl group [having at least one substituent selected from halogen atoms, nitro groups, $C_1$-$C_6$ alkyl groups, $C_2$-$C_6$ alkenyl groups, $C_1$-$C_6$ alkoxy groups,

$C_1-C_6$ alkanoyl groups, $(C_1-C_6$ alkoxy)carbonyl groups, di($C_1-C_6$ alkyl)amino groups, $C_1-C_6$ alkanoylamino groups and cyano groups], a di($C_1-C_6$ alkyl)amino group or a 5- or 6-membered heterocyclic group containing at least one nitrogen or oxygen hetero-atom.

5. Compounds as claimed in Claim 1, wherein $R^4$ represents a group of formula $-A-COX''R^{31}$, and: X" represents an oxygen or sulfur atom; A represents a $C_1-C_5$ alkylene, alkylidene, alkenylene or alkenylidene group or such a group having one of the substituents specified for alkyl or alkenyl groups of $R^4$; and $R^{31}$ represents a hydrogen atom or a $C_1-C_6$ alkyl group.

6. Compounds as claimed in Claim 1, wherein $R^4$ represents a group of formula $-CO.NR^7R^8$ or $-A-CO.NR^7R^8$, and: A represents a $C_1-C_5$ alkylene, alkylidene, alkenylene or alkenylidene group or such a group having one of the substituents specified for alkyl or alkenyl groups of $R^4$; $R^7$ represents a hydrogen atom, a $C_1-C_6$ alkyl group, a $C_1-C_6$ hydroxyalkyl group or a $C_2-C_6$ alkenyl group; and $R^8$ represents a hydrogen atom, a $C_1-C_6$ alkyl group, a substituted $C_1-C_6$ alkyl group [having at least one substituent selected from halogen atoms, cyano groups, hydroxy

groups, $C_1$-$C_6$ alkoxy groups, phenoxy groups, phenoxy groups having at least one halogen substituent, carboxy groups, ($C_1$-$C_6$ alkoxy)carbonyl groups, di($C_1$-$C_6$ alkyl)amino groups and 5- or 6-membered heterocyclic rings containing at least one nitrogen or oxygen hetero-atom], a $C_2$-$C_6$ alkenyl group, a $C_2$-$C_6$ alkynyl group, a $C_3$-$C_8$ cycloalkyl group, a substituted $C_3$-$C_8$ cycloalkyl group having at least one $C_1$-$C_6$ alkyl substituent, an aralkyl group, a substituted aralkyl group (having at least one substituent selected from the group consisting of halogen atoms, $C_1$-$C_6$ alkyl groups and nitro groups), a phenyl group, a substituted phenyl group [having at least one substituent selected from halogen atoms, hydroxy groups, nitro groups, cyano groups, $C_1$-$C_6$ alkyl groups, trifluoromethyl groups, $C_1$-$C_6$ alkoxy groups and ($C_1$-$C_6$ alkoxy)carbonyl groups], a hydroxy group, a $C_1$-$C_6$ alkoxy group, a $C_1$-$C_6$ alkanoyloxy group, a $C_1$-$C_6$ alkanoyl group, a substituted $C_1$-$C_6$ alkanoyl group having at least one $C_1$-$C_6$ alkoxy substituent, a ($C_1$-$C_6$ alkoxy)thiocarbonyl group, a ($C_1$-$C_6$ alkyl)thiocarbamoyl group, a phenylthiocarbamoyl group, a phenylthiocarbamoyl group having at least one halogen substituent on the phenyl moiety, an amino group, a di($C_1$-$C_6$ alkyl)amino group, an anilino group or a 5- or a 6-membered heterocyclic group containing at least one nitrogen,

sulfur or oxygen hetero-atom; or $R^7$ and $R^8$, together with the nitrogen atom to which they are attached, represent a piperidino group or a morpholino group.

7. Compounds as claimed in Claim 1, wherein $R^4$ represents a group of formula $-A-NHR^{32}$, and: A represents a $C_1-C_5$ alkylene, alkylidene, alkenylene or alkenylidene group or such a group having one of the substituents specified for alkyl or alkenyl groups of $R^4$; and $R^{32}$ represents a phenyl group or a phenyl group having a $C_1-C_6$ alkyl or ($C_1$ - $C_6$ alkyl)carbamoyl substituent.

8. Compounds as claimed in Claim 1, wherein $R^4$ represents a group of formula $-A-X''R^{33}$, and: A represents a $C_1-C_5$ alkylene, alkylidene, alkenylene or alkenylidene group or such a group having one of the substituents specified for alkyl or alkenyl groups of $R^4$;and $R^{33}$ represents a hydrogen atom or a $C_1-C_6$ alkanoyl, $C_1-C_6$ haloalkanoyl, benzoyl or $C_1-C_6$ alkyl group.

9. Compounds as claimed in Claim 1, wherein $R^4$ represents a group of formula $-A-R^{34}$, and: A represents a $C_1-C_5$ alkylene, alkylidene, alkenylene or alkenylidene group or such a group having one of the substituents specified for alkyl or alkenyl groups of

$R^4$; and $R^{34}$ represents a halogen atom, a cyano group or a $C_1$-$C_6$ alkanoyl group.

10. Compounds as claimed in Claim 1, wherein $R^4$ represents an oxiranyl, 1,3,4-oxadiazol-2-yl or 5-oxo-1,3,4-triazol-2-yl group.

11. Compounds as claimed in any one of the preceding Claims, wherein $R^4$ is present at the 3-position.

12. Compounds as claimed in Claim 1 or Claim 11, in which $R^4$ represents a group of formula $-COR^{30}$, $-CO.X''R^{11}$, $-A-COX''R^{31}$, $-CO.NHR^8$, $-A-CONHR^8$, $-A-X''R^{33}$ or $-A-R^{34}$, or a cyano or 1,3,4-oxadiazol-2-yl group (in which X" represents an oxygen or sulphur atom; $R^{11}$ represents a hydrogen atom, a $C_1$-$C_{10}$ alkyl group or a phenyl group; $R^8$ represents a hydrogen atom or a $C_1$-$C_6$ alkyl group; A represents a $C_1$-$C_4$ alkylene or alkenylene group; $R^{30}$ represents a hydrogen atom; $R^{33}$ represents a hydrogen atom or a $C_1$-$C_6$ alkanoyl, $C_1$-$C_6$ haloalkanoyl, benzoyl or $C_1$-$C_6$ alkyl group; and $R^{34}$ represents a halogen atom, a cyano group or a $C_1$-$C_6$ alkanoyl group.

13. Compounds as claimed in any one of the preceding Claims, wherein $R^3$ represents a hydrogen atom.

14. Compounds as claimed in any one of the preceding Claims, wherein $R^1$ and $R^2$ both represent ethyl groups.

15. Compounds as claimed in any one of Claims 1 to 13, wherein $R^1$ and $R^2$ both represent isopropyl groups.

16. Compounds as claimed in any one of Claims 1 to 13, wherein $R^1$ represents an ethyl group and $R^2$ represents a sec-butyl group.

17. N-(2-sec-butyl-6-ethylphenyl)-3-carboxyphthalimide.

18. 3-carboxy-N-(2,6-diisopropylphenyl)phthalimide.

19. N-(2,6-diisopropylphenyl)-3-methoxycarbonylphthalimide.

20. N-(2,6-diisopropylphenyl)-3-ethoxycarbonylphthalimide.

21. N-(2,6-diisopropylphenyl)-3-propoxycarbonylphthalimide.

22. 3-butoxycarbonyl-N-(2,6-diisopropylphenyl)-phthalimide.

23. N-(2,6-diisopropylphenyl)-3-pentyloxycarbonylphthalimide.

24. N-(2,6-diisopropylphenyl)-3-hexyloxycarbonylphthalimide.

25. N-(2,6-diisopropylphenyl)-3-heptyloxycarbonylphthalimide.

26. N-(2,6-diisopropylphenyl)-3-octyloxycarbonylphthalimide.

27. N-(2,6-diisopropylphenyl)-3-nonyloxycarbonylphthalimide.

28. 3-decyloxycarbonyl-N-(2,6-diisopropylphenyl)phthalimide.

29. 3-carbamoyl-N-(2,6-diisopropylphenyl)phthalimide.

30. N-(2-ethyl-6-isopropylphenyl)-3-hydroxymethylphthalimide.

31. N-(2-sec-butyl-6-ethylphenyl)-3-hydroxymethylphthalimide.

32. N-(2,6-diisopropylphenyl)-3-hydroxymethyl-

phthalimide.

33. 3-butoxymethyl-N-(2,6-diisopropylphenyl)-phthalimide.

34. N-(2,6-diisopropylphenyl)-3-formylphthalimide.

35. 3-carboxymethyl-N-(2,6-diisopropylphenyl)-phthalimide.

36. N-(2,6-diisopropylphenyl)-3-ethoxycarbonylmethyl-phthalimide.

37. N-(2,6-diisopropylphenyl)-3-(2-ethoxycarbonylethyl)phthalimide.

38. 3-cyanomethyl-N-(2,6-diisopropylphenyl)-phthalimide.

39. 3-(2-carboxyvinyl)-N-(2,6-diisopropylphenyl)-phthalimide.

40. N-(2,6-diisopropylphenyl)-3-(1,3,4-oxadiazol-2-yl)phthalimide.

41. An agricultural fungicidal composition comprising a fungicidal compound in admixture with a carrier or

diluent, wherein the fungicide is a compound as claimed in any one of the preceding Claims.

42. A process for preparing compounds as claimed in any one of Claims 1 to 40, which process comprises the steps:

(a) reacting an aniline derivative of formula (III):

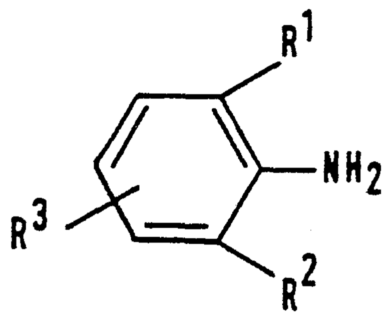

(III)

(wherein $R^1$, $R^2$ and $R^3$ are as defined in Claim 1) with a phthalic acid derivative of formula (IVa)

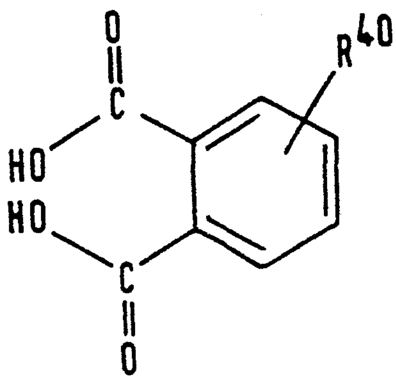

(IVa)

(wherein $R^{40}$ represents any one of the groups defined for $R^4$ or any one of said groups wherein any active group or atom is protected) or with an active derivative of said phthalic acid derivative;

(b) if necessary, removing any protecting group;

(c) if necessary, converting any group represented by $R^4$ to any other group represented by $R^4$; and

(d) if necessary, forming an acid halide, ester, amide or salt of the resulting compound.

43. A process according to Claim 42, in which said active derivative of said phthalic acid derivative (IVa) is an active ester, active amide, acid halide or anhydride.

44. A process according to Claim 43, in which said active derivative is the anhydride of formula (IVb):

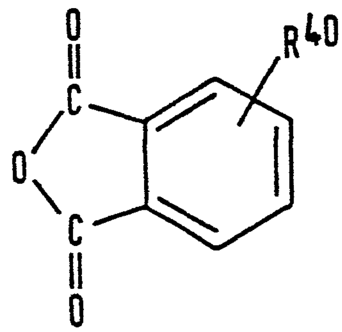

(IVb)

(in which $R^{40}$ is as defined in Claim 42).